# EUROPEAN PATENT SPECIFICATION

(11) **EP 1 399 472 B1**
(45) Date of publication and mention of the grant of the patent: **03.06.2009**
(21) Application number: 02713823.9
(22) Date of filing: 07.03.2002
(51) Int. Cl.: C07K 14/00, C07K 14/715

(54) **SOLUBLE HETERODIMERIC CYTOKINE RECEPTOR**
LÖSLICHER HETERODIMERER CYTOKINREZEPTOR
RECEPTEUR DE CYTOKINE HETERODIMERE SOLUBLE

(30) Priority: 09.03.2001 US 274560 P; 21.06.2001 US 299865 P
(43) Date of publication of application: 24.03.2004
(73) Proprietor: ZymoGenetics, Inc., Seattle, WA 98102 (US)
(72) Inventor: CHANDRASEKHER, Yasmin, A., Saratoga, CA 95070 (US); NOVAK, Julia, E., Suquamish WA 98392 (US); FOSTER, Donald, C., Lake Forest Park, WA 98155 (US); XU, Wenfeng, Seattle , WA 98115-3407 (US); JASPERS, Stephen, R., Edmonds, WA 98020 (US)
(74) Representative: MacLean, Martin Robert
(86) International application number: PCT/US2002/007214
(87) International publication number: WO 2002/072607

(56) References cited:
- WO-A-99/07848
- WO-A1-00/39161
- BLUMBERG H ET AL: "Interleukin 20: Discovery, receptor identification, and role in epidermal function" CELL, CELL PRESS, CAMBRIDGE, NA, US, vol. 104, no. 1, 12 January 2001 (2001-01-12), pages 9-19, XP002210052 ISSN: 0092-8674
- XU W ET AL: "A soluble class II cytokine receptor, IL-22RA2, is a naturally occurring IL-22 antagonist" PROCEEDINGS OF THE NATIONAL ACADEMY OF SCIENCES OF USA, NATIONAL ACADEMY OF SCIENCE. WASHINGTON, US, vol. 98, no. 17, 14 August 2001 (2001-08-14), pages 9511-9516, XP002206183 ISSN: 0027-8424
- XIE ET AL.: 'Interleukin (IL)-22, a novel human cytokine that signals through the interferon receptor-related proteins CRF2-4 and IL-22R' J. BIOL. CHEM. vol. 275, no. 40, 06 October 2000, pages 31335 - 31339, XP002164307

### SEQUENCE LISTING

<110> Chandrasekher, Yasmin A.
   Novak, Julia E.
   Foster, Donald C.
   Wenfeng, Xu
   Jaspers Stephen R.
<120> Soluble Heterodimeric Cytokine Receptor
<130> 01-10PC
<150> 60/274,560
   <151> 2001-03-09
<150> 60/299,865
   <151> 2001-06-21
<160> 40
<170> FastSEQ for Windows Version 3.0
<210> 1
   <211> 176
   <212> PRT
   <213> Homo sapiens
<400> 1
<210> 2
   <211> 152
   <212> PRT
   <213> Homo sapiens
<400> 2
   Leu Lys Thr Leu Asn Leu Gly Ser Cys Val Ile Ala Thr Asn Leu Gln
    1              5                  10                  15
   Glu Ile Arg Asn Gly Phe Ser Asp Ile Arg Gly Ser Val Gln Ala Lys
               20                  25                  30
   Asp Gly Asn Ile Asp Ile Arg Ile Leu Arg Arg Thr Glu Ser Leu Gln
           35                  40                  45
   Asp Thr Lys Pro Ala Asn Arg Cys Cys Leu Leu Arg His Leu Leu Arg
       50                  55                  60
   Leu Tyr Leu Asp Arg Val Phe Lys Asn Tyr Gln Thr Pro Asp His Tyr
   65                  70                  75                  80
   Thr Leu Arg Lys Ile Ser Ser Leu Ala Asn Ser Phe Leu Thr Ile Lys
                   85                  90                  95
   Lys Asp Leu Arg Leu Cys His Ala His Met Thr Cys His Cys Gly Glu
               100                 105                 110
   Glu Ala Met Lys Lys Tyr Ser Gln Ile Leu Ser His Phe Glu Lys Leu
           115                 120                 125
   Glu Pro Gln Ala Ala Val Val Lys Ala Leu Gly Glu Leu Asp Ile Leu
       130                 135                 140
   Leu Gln Trp Met Glu Glu Thr Glu
   145                 150
<210> 3
   <211> 151
   <212> PRT
   <213> Homo sapiens
<400> 3
   Met Lys Ala Ser Ser Leu Ala Phe Ser Leu Leu Ser Ala Ala Phe Tyr
    1               5                  10                  15
   Leu Leu Trp Thr Pro Ser Thr Gly Leu Lys Thr Leu Asn Leu Gly Ser
               20                  25                  30
   Cys Val Ile Ala Thr Asn Leu Gln Glu Ile Arg Asn Gly Phe Ser Asp
           35                  40                  45
   Ile Arg Gly Ser Val Gln Ala Lys Asp Gly Asn Ile Asp Ile Arg Ile
       50                  55                  60
   Leu Arg Arg Thr Glu Ser Leu Gln Asp Thr Lys Pro Ala Asn Arg Cys
   65                  70                  75                  80
   Cys Leu Leu Arg His Leu Leu Arg Leu Tyr Leu Asp Arg Val Phe Lys
                   85                  90                  95
   Asn Tyr Gln Thr Pro Asp His Tyr Thr Leu Arg Lys Ile Ser Ser Leu
               100                 105                 110
   Ala Asn Ser Phe Leu Thr Ile Lys Lys Asp Leu Arg Leu Cys Leu Glu
           115                 120                 125
   Pro Gln Ala Ala Val Val Lys Ala Leu Gly Glu Leu Asp Ile Leu Leu
       130                 135                 140
   Gln Trp Met Glu Glu Thr Glu
   145                 150
<210> 4
   <211> 127
   <212> PRT
   <213> Homo sapiens
<400> 4
   Leu Lys Thr Leu Asn Leu Gly Ser Cys Val Ile Ala Thr Asn Leu Gln
    1               5                  10                  15
   Glu Ile Arg Asn Gly Phe Ser Asp Ile Arg Gly Ser Val Gln Ala Lys
               20                  25                  30
   Asp Gly Asn Ile Asp Ile Arg Ile Leu Arg Arg Thr Glu Ser Leu Gln
           35                  40                  45
   Asp Thr Lys Pro Ala Asn Arg Cys Cys Leu Leu Arg His Leu Leu Arg
       50                  55                  60
   Leu Tyr Leu Asp Arg Val Phe Lys Asn Tyr Gln Thr Pro Asp His Tyr
   65                  70                  75                  80
   Thr Leu Arg Lys Ile Ser Ser Leu Ala Asn Ser Phe Leu Thr Ile Lys
                   85                  90                  95
   Lys Asp Leu Arg Leu Cys Leu Glu Pro Gln Ala Ala Val Val Lys Ala
               100                 105                 110
   Leu Gly Glu Leu Asp Ile Leu Leu Gln Trp Met Glu Glu Thr Glu
           115                 120                 125
<210> 5
   <211> 176
   <212> PRT
   <213> mouse
<400> 5
   Met Lys Gly Phe Gly Leu Ala Phe Gly Leu Phe Ser Ala Val Gly Phe
    1               5                  10                  15
   Leu Leu Trp Thr Pro Leu Thr Gly Leu Lys Thr Leu His Leu Gly Ser
               20                  25                  30
   Cys Val Ile Thr Ala Asn Leu Gln Ala Ile Gln Lys Glu Phe Ser Glu
           35                  40                  45
   Ile Arg Asp Ser Val Gln Ala Glu Asp Thr Asn Ile Asp Ile Arg Ile
       50                  55                  60
   Leu Arg Thr Thr Glu Ser Leu Lys Asp Ile Lys Ser Leu Asp Arg Cys
   65                  70                  75                  80
   Cys Phe Leu Arg His Leu Val Arg Phe Tyr Leu Asp Arg Val Phe Lys
                   85                  90                  95
   Val Tyr Gln Thr Pro Asp His His Thr Leu Arg Lys Ile Ser Ser Leu
               100                 105                 110
   Ala Asn Ser Phe Leu Ile Ile Lys Lys Asp Leu Ser Val Cys His Ser
           115                 120                 125
   His Met Ala Cys His Cys Gly Glu Glu Ala Met Glu Lys Tyr Asn Gln
       130                 135                 140
   Ile Leu Ser His Phe Ile Glu Leu Glu Leu Gln Ala Ala Val Val Lys
   145                 150                 155                 160
   Ala Leu Gly Glu Leu Gly Ile Leu Leu Arg Trp Met Glu Glu Met Leu
                   165                 170                 175
<210> 6
   <211> 152
   <212> PRT
   <213> Mouse
<400> 6
   Leu Lys Thr Leu His Leu Gly Ser Cys Val Ile Thr Ala Asn Leu Gln
    1               5                  10                  15
   Ala Ile Gln Lys Glu Phe Ser Glu Ile Arg Asp Ser Val Gln Ala Glu
               20                  25                  30
   Asp Thr Asn Ile Asp Ile Arg Ile Leu Arg Thr Thr Glu Ser Leu Lys
           35                  40                  45
   Asp Ile Lys Ser Leu Asp Arg Cys Cys Phe Leu Arg His Leu Val Arg
       50                  55                  60
   Phe Tyr Leu Asp Arg Val Phe Lys Val Tyr Gln Thr Pro Asp His His
   65                  70                  75                  80
   Thr Leu Arg Lys Ile Ser Ser Leu Ala Asn Ser Phe Leu Ile Ile Lys
                   85                  90                  95
   Lys Asp Leu Ser Val Cys His Ser His Met Ala Cys His Cys Gly Glu
               100                 105                 110
   Glu Ala Met Glu Lys Tyr Asn Gln Ile Leu Ser His Phe Ile Glu Leu
           115                 120                 125
   Glu Leu Gln Ala Ala Val Val Lys Ala Leu Gly Glu Leu Gly Ile Leu
       130                 135                 140
   Leu Arg Trp Met Glu Glu Met Leu
   145                 150
<210> 7
   <211> 144
   <212> PRT
   <213> Mouse
<400> 7
   Cys Val Ile Thr Ala Asn Leu Gln Ala Ile Gln Lys Glu Phe Ser Glu
    1               5                  10                  15
   Ile Arg Asp Ser Val Gln Ala Glu Asp Thr Asn Ile Asp Ile Arg Ile
               20                  25                  30
   Leu Arg Thr Thr Glu Ser Leu Lys Asp Ile Lys Ser Leu Asp Arg Cys
           35                  40                  45
   Cys Phe Leu Arg His Leu Val Arg Phe Tyr Leu Asp Arg Val Phe Lys
       50                  55                  60
   Val Tyr Gln Thr Pro Asp His His Thr Leu Arg Lys Ile Ser Ser Leu
   65                  70                  75                  80
   Ala Asn Ser Phe Leu Ile Ile Lys Lys Asp Leu Ser Val Cys His Ser
                   85                  90                  95
   His Met Ala Cys His Cys Gly Glu Glu Ala Met Glu Lys Tyr Asn Gln
               100                 105                 110
   Ile Leu Ser His Phe Ile Glu Leu Glu Leu Gln Ala Ala Val Val Lys
           115                 120                 125
   Ala Leu Gly Glu Leu Gly Ile Leu Leu Arg Trp Met Glu Glu Met Leu
       130                 135                 140
<210> 8
   <211> 154
   <212> PRT
   <213> Homo sapiens
<400> 8
   Met Lys Gly Phe Gly Leu Ala Phe Gly Leu Phe Ser Ala Val Gly Phe
    1               5                  10                  15
   Leu Leu Trp Thr Pro Leu Thr Gly Leu Lys Thr Leu His Leu Gly Ser
               20                  25                  30
   Cys Val Ile Thr Ala Asn Leu Gln Ala Ile Gln Lys Glu Phe Ser Glu
           35                  40                  45
   Ile Arg Asp Ser Val Ser Leu Asp Arg Cys Cys Phe Leu Arg His Leu
       50                  55                  60
   Val Arg Phe Tyr Leu Asp Arg Val Phe Lys Val Tyr Gln Thr Pro Asp
   65                  70                  75                  80
   His His Thr Leu Arg Lys Ile Ser Ser Leu Ala Asn Ser Phe Leu Ile
                   85                  90                  95
   Ile Lys Lys Asp Leu Ser Val Cys His Ser His Met Ala Cys His Cys
               100                 105                 110
   Gly Glu Glu Ala Met Glu Lys Tyr Asn Gln Ile Leu Ser His Phe Ile
           115                 120                 125
   Glu Leu Glu Leu Gln Ala Ala Val Val Lys Ala Leu Gly Glu Leu Gly
       130                 135                 140
   Ile Leu Leu Arg Trp Met Glu Glu Met Leu
   145                 150
<210> 9
   <211> 130
   <212> PRT
   <213> Human
<400> 9
   Leu Lys Thr Leu His Leu Gly Ser Cys Val Ile Thr Ala Asn Leu Gln
    1               5                  10                  15
   Ala Ile Gln Lys Glu Phe Ser Glu Ile Arg Asp Ser Val Ser Leu Asp
               20                  25                  30
   Arg Cys Cys Phe Leu Arg His Leu Val Arg Phe Tyr Leu Asp Arg Val
           35                  40                  45
   Phe Lys Val Tyr Gln Thr Pro Asp His His Thr Leu Arg Lys Ile Ser
       50                  55                  60
   Ser Leu Ala Asn Ser Phe Leu Ile Ile Lys Lys Asp Leu Ser Val Cys
   65                  70                  75                  80
   His Ser His Met Ala Cys His Cys Gly Glu Glu Ala Met Glu Lys Tyr
                   85                  90                  95
   Asn Gln Ile Leu Ser His Phe Ile Glu Leu Glu Leu Gln Ala Ala Val
               100                 105                 110
   Val Lys Ala Leu Gly Glu Leu Gly Ile Leu Leu Arg Trp Met Glu Glu
           115                 120                 125
   Met Leu
       130
<210> 10
   <211> 2831
   <212> DNA
   <213> human
<220>
   <221> CDS
   <222> (34)...(1755)
<400> 10
   tagaggccaa gggagggctc tgtgccagcc ccg atg agg acg ctg ctg acc atc       54
   ttg act gtg gga tcc ctg gct gct cac gcc cct gag gac ccc tcg gat       102
   ctg ctc cag cac gtg aaa ttc cag tcc agc aac ttt gaa aac atc ctg       150
   acg tgg gac agc ggg cca gag ggc acc cca gac acg gtc tac agc atc       198
   gag tat aag acg tac gga gag agg gac tgg gtg gca aag aag ggc tgt       246
   cag cgg atc acc cgg aag tcc tgc aac ctg acg gtg gag acg ggc aac       294
   ctc acg gag ctc tac tat gcc agg gtc acc gct gtc agt gcg gga ggc       342
   cgg tca gcc acc aag atg act gac agg ttc agc tct ctg cag cac act       390
   acc ctc aag cca cct gat gtg acc tgt atc tcc aaa gtg aga tcg att       438
   cag atg att gtt cat cct acc ccc acg cca atc cgt gca ggc gat ggc       486
   cac cgg cta acc ctg gaa gac atc ttc cat gac ctg ttc tac cac tta       534
   gag ctc cag gtc aac cgc acc tac caa atg cac ctt gga ggg aag cag       582
   aga gaa tat gag ttc ttc ggc ctg acc cct gac aca gag ttc ctt ggc       630
   acc atc atg att tgc gtt ccc acc tgg gcc aag gag agt gcc ccc tac       678
   atg tgc cga gtg aag aca ctg cca gac cgg aca tgg acc tac tcc ttc       726
   tcc gga gcc ttc ctg ttc tcc atg ggc ttc ctc gtc gca gta ctc tgc       774
   tac ctg agc tac aga tat gtc acc aag ccg cct gca cct ccc aac tcc       822
   ctg aac gtc cag cga gtc ctg act ttc cag ccg ctg cgc ttc atc cag       870
   gag cac gtc ctg atc cct gtc ttt gac ctc agc ggc ccc agc agt ctg       918
   gcc cag cct gtc cag tac tcc cag atc agg gtg tct gga ccc agg gag       966
   ccc gca gga gct cca cag cgg cat agc ctg tcc gag atc acc tac tta      1014
   ggg cag cca gac atc tcc atc ctc cag ccc tcc aac gtg cca cct ccc      1062
   cag atc ctc tcc cca ctg tcc tat gcc cca aac gct gcc cct gag gtc      1110
   ggg ccc cca tcc tat gca cct cag gtg acc ccc gaa gct caa ttc cca      1158
   ttc tac gcc cca cag gcc atc tct aag gtc cag cct tcc tcc tat gcc      1206
   cct caa gcc act ccg gac agc tgg cct ccc tcc tat ggg gta tgc atg      1254
   gaa ggt tct ggc aaa gac tcc ccc act ggg aca ctt tct agt cct aaa      1302
   cac ctt agg cct aaa ggt cag ctt cag aaa gag cca cca gct gga agc      1350
   tgc atg tta ggt ggc ctt tct ctg cag gag gtg acc tcc ttg gct atg      1398
   gag gaa tcc caa gaa gca aaa tca ttg cac cag ccc ctg ggg att tgc      1446
   aca gac aga aca tct gac cca aat gtg cta cac agt ggg gag gaa ggg      1494
   aca cca cag tac cta aag ggc cag ctc ccc ctc ctc tcc tca gtc cag      1542
   atc gag ggc cac ccc atg tcc ctc cct ttg caa cct cct tcc ggt cca      1590
   tgt tcc ccc tcg gac caa ggt cca agt ccc tgg ggc ctg ctg gag tcc      1638
   ctt gtg tgt ccc aag gat gaa gcc aag agc cca gcc cct gag acc tca      1686
   gac ctg gag cag ccc aca gaa ctg gat tct ctt ttc aga ggc ctg gcc      1734
   ctg act gtg cag tgg gag tcc tgaggggaat gggaaaggct tggtgcttcc         1785
   tccctgtccc tacccagtgt cacatccttg gctgtcaatc ccatgcctgc ccatgccaca    1845
   cactctgcga tctggcctca gacgggtgcc cttgagagaa gcagagggag tggcatgcag    1905
   ggcccctgcc atgggtgcgc tcctcaccgg aacaaagcag catgataagg actgcagcgg    1965
   gggagctctg gggagcagct tgtgtagaca agcgcgtgct cgctgagccc tgcaaggcag     2025
   aaatgacagt gcaaggagga aatgcaggga aactcccgag gtccagagcc ccacctccta    2085
   acaccatgga ttcaaagtgc tcagggaatt tgcctctcct tgccccattc ctggccagtt    2145
   tcacaatcta gctcgacaga gcatgaggcc cctgcctctt ctgtcattgt tcaaaggtgg    2205
   gaagagagcc tggaaaagaa ccaggcctgg aaaagaacca gaaggaggct gggcagaacc    2265
   agaacaacct gcacttctgc caaggccagg gccagcagga cggcaggact ctagggaggg    2325
   gtgtggcctg cagctcattc ccagccaggg caactgcctg acgttgcacg atttcagctt    2385
   cattcctctg atagaacaaa gcgaaatgca ggtccaccag ggagggagac acacaagcct    2445
   tttctgcagg caggagtttc agaccctatc ctgagaatgg ggtttgaaag gaaggtgagg    2505
   gctgtggccc ctggacgggt acaataacac actgtactga tgtcacaact ttgcaagctc    2565
   tgccttgggt tcagcccatc tgggctcaaa ttccagcctc accactcaca agctgtgtga   2625
   cttcaaacaa atgaaatcag tgcccagaac ctcggtttcc tcatctgtaa tgtggggatc   2685
   ataacaccta cctcatggag ttgtggtgaa gatgaaatga agtcatgtct ttaaagtgct   2745
   taatagtgcc tggtacatgg gcagtgccca ataaacggta gctatttaaa aaaaaaaaaa   2805
   aaaaaaaaaa atagcggccg cctcga                                        2831
<210> 11
   <211> 574
   <212> PRT
   <213> human
<400> 11
<210> 12
   <211> 228
   <212> PRT
   <213> human
<400> 12
   Met Arg Thr Leu Leu Thr Ile Leu Thr Val Gly Ser Leu Ala Ala His
    1               5                  10                  15
   Ala Pro Glu Asp Pro Ser Asp Leu Leu Gln His Val Lys Phe Gln Ser
               20                  25                  30
   Ser Asn Phe Glu Asn Ile Leu Thr Trp Asp Ser Gly Pro Glu Gly Thr
           35                  40                  45
   Pro Asp Thr Val Tyr Ser Ile Glu Tyr Lys Thr Tyr Gly Glu Arg Asp
       50                  55                  60
   Trp Val Ala Lys Lys Gly Cys Gln Arg Ile Thr Arg Lys Ser Cys Asn
   65                  70                  75                  80
   Leu Thr Val Glu Thr Gly Asn Leu Thr Glu Leu Tyr Tyr Ala Arg Val
                   85                  90                  95
   Thr Ala Val Ser Ala Gly Gly Arg Ser Ala Thr Lys Met Thr Asp Arg
               100                 105                 110
   Phe Ser Ser Leu Gln His Thr Thr Leu Lys Pro Pro Asp Val Thr Cys
           115                 120                 125
   Ile Ser Lys Val Arg Ser Ile Gln Met Ile Val His Pro Thr Pro Thr
       130                 135                 140
   Pro Ile Arg Ala Gly Asp Gly His Arg Leu Thr Leu Glu Asp Ile Phe
   145                 150                 155                 160
   His Asp Leu Phe Tyr His Leu Glu Leu Gln Val Asn Arg Thr Tyr Gln
                   165                 170                 175
   Met His Leu Gly Gly Lys Gln Arg Glu Tyr Glu Phe Phe Gly Leu Thr
               180                 185                 190
   Pro Asp Thr Glu Phe Leu Gly Thr Ile Met Ile Cys Val Pro Thr Trp
           195                 200                 205
   Ala Lys Glu Ser Ala Pro Tyr Met Cys Arg Val Lys Thr Leu Pro Asp
       210                 215                 220
   Arg Thr Trp Thr
   225
<210> 13
   <211> 211
   <212> PRT
   <213> human
<400> 13
   Pro Glu Asp Pro Ser Asp Leu Leu Gln His Val Lys Phe Gln Ser Ser
    1               5                  10                  15
   Asn Phe Glu Asn Ile Leu Thr Trp Asp Ser Gly Pro Glu Gly Thr Pro
               20                  25                  30
   Asp Thr Val Tyr Ser Ile Glu Tyr Lys Thr Tyr Gly Glu Arg Asp Trp
           35                  40                  45
   Val Ala Lys Lys Gly Cys Gln Arg Ile Thr Arg Lys Ser Cys Asn Leu
       50                  55                  60
   Thr Val Glu Thr Gly Asn Leu Thr Glu Leu Tyr Tyr Ala Arg Val Thr
   65                  70                  75                  80
   Ala Val Ser Ala Gly Gly Arg Ser Ala Thr Lys Met Thr Asp Arg Phe
                   85                  90                  95
   Ser Ser Leu Gln His Thr Thr Leu Lys Pro Pro Asp Val Thr Cys Ile
               100                 105                 110
   Ser Lys Val Arg Ser Ile Gln Met Ile Val His Pro Thr Pro Thr Pro
           115                 120                 125
   Ile Arg Ala Gly Asp Gly His Arg Leu Thr Leu Glu Asp Ile Phe His
       130                 135                 140
   Asp Leu Phe Tyr His Leu Glu Leu Gln Val Asn Arg Thr Tyr Gln Met
   145                 150                 155                 160
   His Leu Gly Gly Lys Gln Arg Glu Tyr Glu Phe Phe Gly Leu Thr Pro
                   165                 170                 175
   Asp Thr Glu Phe Leu Gly Thr Ile Met Ile Cys Val Pro Thr Trp Ala
               180                 185                 190
   Lys Glu Ser Ala Pro Tyr Met Cys Arg Val Lys Thr Leu Pro Asp Arg
           195                 200                 205
   Thr Trp Thr
       210
<210> 14
   <211> 971
   <212> DNA
   <213> Human
<220>
   <221> CDS
   <222> (18)...(950)
<400> 14
   gaattcgagt ctaccaa atg cag act ttc aca atg gtt cta gaa gaa atc        50
                      Met Gln Thr Phe Thr Met Val Leu Glu Glu Ile
                      1               5                   10
   tgg aca agt ctt ttc atg tgg ttt ttc tac gca ttg att cca tgt ttg       98
   Trp Thr Ser Leu Phe Met Trp Phe Phe Tyr Ala Leu Ile Pro Cys Leu
               15                  20                  25
   ctc aca gat gaa gtg gcc att ctg cct gcc cct cag aac ctc tct gta      146
   Leu Thr Asp Glu Val Ala Ile Leu Pro Ala Pro Gln Asn Leu Ser Val
           30                  35                  40
   ctc tca acc aac atg aag cat ctc ttg atg tgg agc cca gtg atc gcg      194
   Leu Ser Thr Asn Met Lys His Leu Leu Met Trp Ser Pro Val Ile Ala
       45                  50                  55
   cct gga gaa aca gtg tac tat tct gtc gaa tac cag ggg gag tac gag      242
   Pro Gly Glu Thr Val Tyr Tyr Ser Val Glu Tyr Gln Gly Glu Tyr Glu
   60                  65                  70                  75
   agc ctg tac acg agc cac atc tgg atc ccc agc agc tgg tgc tca ctc        290
   Ser Leu Tyr Thr Ser His Ile Trp Ile Pro Ser Ser Trp Cys Ser Leu
                   80                  85                  90
   act gaa ggt cct gag tgt gat gtc act gat gac atc acg gcc act gtg        338
   Thr Glu Gly Pro Glu Cys Asp Val Thr Asp Asp Ile Thr Ala Thr Val
               95                  100                 105
   cca tac aac ctt cgt gtc agg gcc aca ttg ggc tca cag acc tca gcc        386
   Pro Tyr Asn Leu Arg Val Arg Ala Thr Leu Gly Ser Gln Thr Ser Ala
           110                 115                 120
   tgg agc atc ctg aag cat ccc ttt aat aga aac tca acc atc ctt acc        434
   Trp Ser Ile Leu Lys His Pro Phe Asn Arg Asn Ser Thr Ile Leu Thr
       125                 130                 135
   cga cct ggg atg gag atc acc aaa gat ggc ttc cac ctg gtt att gag        482
   Arg Pro Gly Met Glu Ile Thr Lys Asp Gly Phe His Leu Val Ile Glu
   140                 145                 150                 155
   ctg gag gac ctg ggg ccc cag ttt gag ttc ctt gtg gcc tac tgg agg        530
   Leu Glu Asp Leu Gly Pro Gln Phe Glu Phe Leu Val Ala Tyr Trp Arg
                   160                 165                 170
   agg gag cct ggt gcc gag gaa cat gtc aaa atg gtg agg agt ggg ggt        578
   Arg Glu Pro Gly Ala Glu Glu His Val Lys Met Val Arg Ser Gly Gly
               175                 180                 185
   att cca gtg cac cta gaa acc atg gag cca ggg gct gca tac tgt gtg        626
   Ile Pro Val His Leu Glu Thr Met Glu Pro Gly Ala Ala Tyr Cys Val
           190                 195                 200
   aag gcc cag aca ttc gtg aag gcc att ggg agg tac agc gcc ttc agc        674
   Lys Ala Gln Thr Phe Val Lys Ala Ile Gly Arg Tyr Ser Ala Phe Ser
       205                 210                 215
   cag aca gaa tgt gtg gag gtg caa gga gag gcc att ccc ctg gta ctg        722
   Gln Thr Glu Cys Val Glu Val Gln Gly Glu Ala Ile Pro Leu Val Leu
   220                 225                 230                 235
   gcc ctg ttt gcc ttt gtt ggc ttc atg ctg atc ctt gtg gtc gtg cca        770
   Ala Leu Phe Ala Phe Val Gly Phe Met Leu Ile Leu Val Val Val Pro
                   240                 245                 250
   ctg ttc gtc tgg aaa atg ggc cgg ctg ctc cag tac tcc tgt tgc ccc        818
   Leu Phe Val Trp Lys Met Gly Arg Leu Leu Gln Tyr Ser Cys Cys Pro
               255                 260                 265
   gtg gtg gtc ctc cca gac acc ttg aaa ata acc aat tca ccc cag aag        866
   Val Val Val Leu Pro Asp Thr Leu Lys Ile Thr Asn Ser Pro Gln Lys
           270                 275                 280
   tta atc agc tgc aga agg gag gag gtg gat gcc tgt gcc acg gct gtg        914
   Leu Ile Ser Cys Arg Arg Glu Glu Val Asp Ala Cys Ala Thr Ala Val
       285                 290                 295
   atg tct cct gag gaa ctc ctc agg gcc tgg atc tca taggtttgcg             960
   Met Ser Pro Glu Glu Leu Leu Arg Ala Trp Ile Ser
   300                 305                 310
   gaaggctcga g                                                           971
<210> 15
   <211> 311
   <212> PRT
   <213> Human
<400> 15
   Met Gln Thr Phe Thr Met Val Leu Glu Glu Ile Trp Thr Ser Leu Phe
    1               5                  10                  15
   Met Trp Phe Phe Tyr Ala Leu Ile Pro Cys Leu Leu Thr Asp Glu Val
               20                  25                  30
   Ala Ile Leu Pro Ala Pro Gln Asn Leu Ser Val Leu Ser Thr Asn Met
           35                  40                  45
   Lys His Leu Leu Met Trp Ser Pro Val Ile Ala Pro Gly Glu Thr Val
       50                  55                  60
   Tyr Tyr Ser Val Glu Tyr Gln Gly Glu Tyr Glu Ser Leu Tyr Thr Ser
   65                  70                  75                  80
   His Ile Trp Ile Pro Ser Ser Trp Cys Ser Leu Thr Glu Gly Pro Glu
                   85                  90                  95
   Cys Asp Val Thr Asp Asp Ile Thr Ala Thr Val Pro Tyr Asn Leu Arg
               100                 105                 110
   Val Arg Ala Thr Leu Gly Ser Gln Thr Ser Ala Trp Ser Ile Leu Lys
           115                 120                 125
   His Pro Phe Asn Arg Asn Ser Thr Ile Leu Thr Arg Pro Gly Met Glu
       130                 135                 140
   Ile Thr Lys Asp Gly Phe His Leu Val Ile Glu Leu Glu Asp Leu Gly
   145                 150                 155                 160
   Pro Gln Phe Glu Phe Leu Val Ala Tyr Trp Arg Arg Glu Pro Gly Ala
                   165                 170                 175
   Glu Glu His Val Lys Met Val Arg Ser Gly Gly Ile Pro Val His Leu
               180                 185                 190
   Glu Thr Met Glu Pro Gly Ala Ala Tyr Cys Val Lys Ala Gln Thr Phe
           195                 200                 205
   Val Lys Ala Ile Gly Arg Tyr Ser Ala Phe Ser Gln Thr Glu Cys Val
       210                 215                 220
   Glu Val Gln Gly Glu Ala Ile Pro Leu Val Leu Ala Leu Phe Ala Phe
   225                 230                 235                 240
   Val Gly Phe Met Leu Ile Leu Val Val Val Pro Leu Phe Val Trp Lys
                   245                 250                 255
   Met Gly Arg Leu Leu Gln Tyr Ser Cys Cys Pro Val Val Val Leu Pro
               260                 265                 270
   Asp Thr Leu Lys Ile Thr Asn Ser Pro Gln Lys Leu Ile Ser Cys Arg
           275                 280                 285
   Arg Glu Glu Val Asp Ala Cys Ala Thr Ala Val Met Ser Pro Glu Glu
       290                 295                 300
   Leu Leu Arg Ala Trp Ile Ser
   305                 310
<210> 16
   <211> 203
   <212> PRT
   <213> human
<400> 16
   Asp Glu Val Ala Ile Leu Pro Ala Pro Gln Asn Leu Ser Val Leu Ser
   1               5                   10                  15
   Thr Asn Met Lys His Leu Leu Met Trp Ser Pro Val Ile Ala Pro Gly
               20                  25                  30
   Glu Thr Val Tyr Tyr Ser Val Glu Tyr Gln Gly Glu Tyr Glu Ser Leu
           35                  40                  45
   Tyr Thr Ser His Ile Trp Ile Pro Ser Ser Trp Cys Ser Leu Thr Glu
       50                  55                  60
   Gly Pro Glu Cys Asp Val Thr Asp Asp Ile Thr Ala Thr Val Pro Tyr
   65                  70                  75                  80
   Asn Leu Arg Val Arg Ala Thr Leu Gly Ser Gln Thr Ser Ala Trp Ser
                   85                  90                  95
   Ile Leu Lys His Pro Phe Asn Arg Asn Ser Thr Ile Leu Thr Arg Pro
               100                 105                 110
   Gly Met Glu Ile Thr Lys Asp Gly Phe His Leu Val Ile Glu Leu Glu
           115                 120                 125
   Asp Leu Gly Pro Gln Phe Glu Phe Leu Val Ala Tyr Trp Arg Arg Glu
       130                 135                 140
   Pro Gly Ala Glu Glu His Val Lys Met Val Arg Ser Gly Gly Ile Pro
   145                 150                 155                 160
   Val His Leu Glu Thr Met Glu Pro Gly Ala Ala Tyr Cys Val Lys Ala
                   165                 170                 175
   Gln Thr Phe Val Lys Ala Ile Gly Arg Tyr Ser Ala Phe Ser Gln Thr
               180                 185                 190
   Glu Cys Val Glu Val Gln Gly Glu Ala Ile Pro
           195                 200
<210> 17
   <211> 201
   <212> PRT
   <213> Homo sapiens
<400> 17
   Asp Glu Val Ala Ile Leu Pro Ala Pro Gln Asn Leu Ser Val Leu Ser
   1 5 10 15
   Thr Asn Met Lys His Leu Leu Met Trp Ser Pro Val Ile Ala Pro Gly
   20 25 30
   Glu Thr Val Tyr Tyr Ser Val Glu Tyr Gln Gly Glu Tyr Glu Ser Leu
   35 40 45
   Tyr Thr Ser His Ile Trp Ile Pro Ser Ser Trp Cys Ser Leu Thr Glu
   50 55 60
   Gly Pro Glu Cys Asp Val Thr Asp Asp Ile Thr Ala Thr Val Pro Tyr
   65 70 75 80
   Asn Leu Arg Val Arg Ala Thr Leu Gly Ser Gln Thr Ser Ala Trp Ser
   85 90 95
   Ile Leu Lys His Pro Phe Asn Arg Asn Ser Thr Ile Leu Thr Arg Pro
   100 105 110
   Gly Met Glu Ile Thr Lys Asp Gly Phe His Leu Val Ile Glu Leu Glu
   115 120 125
   Asp Leu Gly Pro Gln Phe Glu Phe Leu Val Ala Tyr Trp Arg Arg Glu
   130 135 140
   Pro Gly Ala Glu Glu His Val Lys Met Val Arg Ser Gly Gly Ile Pro
   145 150 155 160
   Val His Leu Glu Thr Met Glu Pro Gly Ala Ala Tyr Cys Val Lys Ala
   165 170 175
   Gln Thr Phe Val Lys Ala Ile Gly Arg Tyr Ser Ala Phe Ser Gln Thr
   180 185 190
   Glu Cys Val Glu Val Gln Gly Glu Ala
   195 200
<210> 18
   <211> 201
   <212> PRT
   <213> Homo sapiens
<400> 18
   Asp Glu Val Ala Ile Leu Pro Ala Pro Gln Asn Leu Ser Val Leu Ser
   1 5 10 15
   Thr Asn Met Lys His Leu Leu Met Trp Ser Pro Val Ile Ala Pro Gly
   20 25 30
   Glu Thr Val Tyr Tyr Ser Val Glu Tyr Gln Gly Glu Tyr Glu Ser Leu
           35                  40                  45
   Tyr Thr Ser His Ile Trp Ile Pro Ser Ser Trp Cys Ser Leu Thr Glu
       50                  55                  60
   Gly Pro Glu Cys Asp Val Thr Asp Asp Ile Thr Ala Thr Val Pro Tyr
   65                  70                  75                  80
   Asn Leu Arg Val Arg Ala Thr Leu Gly Ser Gln Thr Ser Ala Trp Ser
                   85                  90                  95
   Ile Leu Lys His Pro Phe Asn Arg Asn Ser Thr Ile Leu Thr Arg Pro
               100                 105                 110
   Gly Met Glu Ile Pro Lys His Gly Phe His Leu Val Ile Glu Leu Glu
           115                 120                 125
   Asp Leu Gly Pro Gln Phe Glu Phe Leu Val Ala Tyr Trp Thr Arg Glu
       130                 135                 140
   Pro Gly Ala Glu Glu His Val Lys Met Val Arg Ser Gly Gly Ile Pro
   145                 150                 155                 160
   Val His Leu Glu Thr Met Glu Pro Gly Ala Ala Tyr Cys Val Lys Ala
                   165                 170                 175
   Gln Thr Phe Val Lys Ala Ile Gly Arg Tyr Ser Ala Phe Ser Gln Thr
               180                 185                 190
   Glu Cys Val Glu Val Gln Gly Glu Ala
           195                 200
<210> 19
   <211> 196
   <212> PRT
   <213> Homo sapiens
<400> 19
   Leu Pro Ala Pro Gln Asn Leu Ser Val Leu Ser Thr Asn Met Lys His
   1               5                   10                  15
   Leu Leu Met Trp Ser Pro Val Ile Ala Pro Gly Glu Thr Val Tyr Tyr
               20                  25                  30
   Ser Val Glu Tyr Gln Gly Glu Tyr Glu Ser Leu Tyr Thr Ser His Ile
           35                  40                  45
   Trp Ile Pro Ser Ser Trp Cys Ser Leu Thr Glu Gly Pro Glu Cys Asp
       50                  55                  60
   Val Thr Asp Asp Ile Thr Ala Thr Val Pro Tyr Asn Leu Arg Val Arg
   65                  70                  75                  80
   Ala Thr Leu Gly Ser Gln Thr Ser Ala Trp Ser Ile Leu Lys His Pro
                   85                  90                  95
   Phe Asn Arg Asn Ser Thr Ile Leu Thr Arg Pro Gly Met Glu Ile Thr
               100                 105                 110
   Lys Asp Gly Phe His Leu Val Ile Glu Leu Glu Asp Leu Gly Pro Gln
           115                 120                 125
   Phe Glu Phe Leu Val Ala Tyr Trp Arg Arg Glu Pro Gly Ala Glu Glu
       130                 135                 140
   His Val Lys Met Val Arg Ser Gly Gly Ile Pro Val His Leu Glu Thr
   145                 150                 155                 160
   Met Glu Pro Gly Ala Ala Tyr Cys Val Lys Ala Gln Thr Phe Val Lys
                   165                 170                 175
   Ala Ile Gly Arg Tyr Ser Ala Phe Ser Gln Thr Glu Cys Val Glu Val
               180                 185                 190
   Gln Gly Glu Ala
           195
<210> 20
   <211> 203
   <212> PRT
   <213> Homo sapiens
<400> 20
   Asp Glu Val Ala Ile Leu Pro Ala Pro Gln Asn Leu Ser Val Leu Ser
    1               5                  10                  15
   Thr Asn Met Lys His Leu Leu Met Trp Ser Pro Val Ile Ala Pro Gly
               20                  25                  30
   Glu Thr Val Tyr Tyr Ser Val Glu Tyr Gln Gly Glu Tyr Glu Ser Leu
           35                  40                  45
   Tyr Thr Ser His Ile Trp Ile Pro Ser Ser Trp Cys Ser Leu Thr Glu
       50                  55                  60
   Gly Pro Glu Cys Asp Val Thr Asp Asp Ile Thr Ala Thr Val Pro Tyr
   65                  70                  75                  80
   Asn Leu Arg Val Arg Ala Thr Leu Gly Ser Gln Thr Ser Ala Trp Ser
                   85                  90                  95
   Ile Leu Lys His Pro Phe Asn Arg Asn Ser Thr Ile Leu Thr Arg Pro
               100                 105                 110
   Gly Met Glu Ile Pro Lys His Gly Phe His Leu Val Ile Glu Leu Glu
           115                 120                 125
   Asp Leu Gly Pro Gln Phe Glu Phe Leu Val Ala Tyr Trp Thr Arg Glu
       130                 135                 140
   Pro Gly Ala Glu Glu His Val Lys Met Val Arg Ser Gly Gly Ile Pro
   145                 150                 155                 160
   Val His Leu Glu Thr Met Glu Pro Gly Ala Ala Tyr Cys Val Lys Ala
                   165                 170                 175
   Gln Thr Phe Val Lys Ala Ile Gly Arg Tyr Ser Ala Phe Ser Gln Thr
               180                 185                 190
   Glu Cys Val Glu Val Gln Gly Glu Ala Ile Pro
           195                 200
<210> 21
   <211> 196
   <212> PRT
   <213> Homo sapiens
<400> 21
   Leu Pro Ala Pro Gln Asn Leu Ser Val Leu Ser Thr Asn Met Lys His
    1              5                  10                  15
   Leu Leu Met Trp Ser Pro Val Ile Ala Pro Gly Glu Thr Val Tyr Tyr
               20                  25                  30
   Ser Val Glu Tyr Gln Gly Glu Tyr Glu Ser Leu Tyr Thr Ser His Ile
           35                  40                  45
   Trp Ile Pro Ser Ser Trp Cys Ser Leu Thr Glu Gly Pro Glu Cys Asp
       50                  55                  60
   Val Thr Asp Asp Ile Thr Ala Thr Val Pro Tyr Asn Leu Arg Val Arg
   65                  70                  75                  80
   Ala Thr Leu Gly Ser Gln Thr Ser Ala Trp Ser Ile Leu Lys His Pro
                   85                  90                  95
   Phe Asn Arg Asn Ser Thr Ile Leu Thr Arg Pro Gly Met Glu Ile Pro
               100                 105                 110
   Lys His Gly Phe His Leu Val Ile Glu Leu Glu Asp Leu Gly Pro Gln
           115                 120                 125
   Phe Glu Phe Leu Val Ala Tyr Trp Thr Arg Glu Pro Gly Ala Glu Glu
       130                 135                 140
   His Val Lys Met Val Arg Ser Gly Gly Ile Pro Val His Leu Glu Thr
   145                 150                 155                 160
   Met Glu Pro Gly Ala Ala Tyr Cys Val Lys Ala Gln Thr Phe Val Lys
                   165                 170                 175
   Ala Ile Gly Arg Tyr Ser Ala Phe Ser Gln Thr Glu Cys Val Glu Val
               180                 185                 190
   Gln Gly Glu Ala
           195
<210> 22
   <211> 1382
   <212> DNA
   <213> Homo sapiens
<220>
   <221> CDS
   <222> (132)...(1034)
<400> 22
   tcgacccacg cgtccgcgct gcgactcaga cctcagctcc aacatatgca ttctgaagaa      60
   agatggctga gatggacaga atgctttatt ttggaaagaa acaatgttct aggtcaaact     120
   gagtctacca a atg cag act ttc aca atg gtt cta gaa gaa atc tgg aca      170
                Met Gln Thr Phe Thr Met Val Leu Glu Glu Ile Trp Thr
                1               5                   10
   agt ctt ttc atg tgg ttt ttc tac gca ttg att cca tgt ttg ctc aca       218
   Ser Leu Phe Met Trp Phe Phe Tyr Ala Leu Ile Pro Cys Leu Leu Thr
       15                  20                  25
   gat gaa gtg gcc att ctg cct gcc cct cag aac ctc tct gta ctc tca       266
   Asp Glu Val Ala Ile Leu Pro Ala Pro Gln Asn Leu Ser Val Leu Ser
   30                  35                  40                  45
   acc aac atg aag cat ctc ttg atg tgg agc cca gtg atc gcg cct gga       314
   Thr Asn Met Lys His Leu Leu Met Trp Ser Pro Val Ile Ala Pro Gly
                   50                  55                  60
   gaa aca gtg tac tat tct gtc gaa tac cag ggg gag tac gag agc ctg       362
   Glu Thr Val Tyr Tyr Ser Val Glu Tyr Gln Gly Glu Tyr Glu Ser Leu
               65                  70                  75
   tac acg agc cac atc tgg atc ccc agc agc tgg tgc tca ctc act gaa       410
   Tyr Thr Ser His Ile Trp Ile Pro Ser Ser Trp Cys Ser Leu Thr Glu
           80                  85                  90
   ggt cct gag tgt gat gtc act gat gac atc acg gcc act gtg cca tac       458
   Gly Pro Glu Cys Asp Val Thr Asp Asp Ile Thr Ala Thr Val Pro Tyr
       95                  100                 105
   aac ctt cgt gtc agg gcc aca ttg ggc tca cag acc tca gcc tgg agc       506
   Asn Leu Arg Val Arg Ala Thr Leu Gly Ser Gln Thr Ser Ala Trp Ser
   110                 115                 120                 125
   atc ctg aag cat ccc ttt aat aga aac tca acc atc ctt acc cga cct       554
   Ile Leu Lys His Pro Phe Asn Arg Asn Ser Thr Ile Leu Thr Arg Pro
                   130                 135                 140
   ggg atg gag atc ccc aaa cat ggc ttc cac ctg gtt att gag ctg gag       602
   Gly Met Glu Ile Pro Lys His Gly Phe His Leu Val Ile Glu Leu Glu
               145                 150                 155
   gac ctg ggg ccc cag ttt gag ttc ctt gtg gcc tac tgg acg agg gag       650
   Asp Leu Gly Pro Gln Phe Glu Phe Leu Val Ala Tyr Trp Thr Arg Glu
           160                 165                 170
   cct ggt gcc gag gaa cat gtc aaa atg gtg agg agt ggg ggt att cca       698
   Pro Gly Ala Glu Glu His Val Lys Met Val Arg Ser Gly Gly Ile Pro
       175                 180                 185
   gtg cac cta gaa acc atg gag cca ggg gct gca tac tgt gtg aag gcc       746
   Val His Leu Glu Thr Met Glu Pro Gly Ala Ala Tyr Cys Val Lys Ala
   190                 195                 200                 205
   cag aca ttc gtg aag gcc att ggg agg tac agc gcc ttc agc cag aca      794
   Gln Thr Phe Val Lys Ala Ile Gly Arg Tyr Ser Ala Phe Ser Gln Thr
                   210                 215                 220
   gaa tgt gtg gag gtg caa gga gag gcc att ccc ctg gta ctg gcc ctg      842
   Glu Cys Val Glu Val Gln Gly Glu Ala Ile Pro Leu Val Leu Ala Leu
               225                 230                 235
   ttt gcc ttt gtt ggc ttc atg ctg atc ctt gtg gtc gtg cca ctg ttc      890
   Phe Ala Phe Val Gly Phe Met Leu Ile Leu Val Val Val Pro Leu Phe
           240                 245                 250
   gtc tgg aaa atg ggc cgg ctg ctc cag tac tcc tgt tgc ccc gtg gtg      938
   Val Trp Lys Met Gly Arg Leu Leu Gln Tyr Ser Cys Cys Pro Val Val
       255                 260                 265
   gtc ctc cca gac acc ttg aaa ata acc aat tca ccc cag gtt aat cag      986
   Val Leu Pro Asp Thr Leu Lys Ile Thr Asn Ser Pro Gln Val Asn Gln
   270                 275                 280                 285
   ctg cag aag gga gga ggt gga tgc ctg tgc cac ggc tgt gat gtc tcc     1034
   Leu Gln Lys Gly Gly Gly Gly Cys Leu Cys His Gly Cys Asp Val Ser
                   290                 295                 300
   tgaggaactc ctcagggcct ggatctcata tcaggtttgc ggaagggccc aggtgaagcc   1094
   gagaacctgg tctgcatgac atggaaacca tgaggggaca agttgtgttt ctgttttccg   1154
   ccacggacaa gggatgagag aagtaggaag agcctgttgt ctacaagtct agaagcaacc   1214
   atcagaggca gggtggtttg tctaacagaa caactgactg aggctatggg ggttgtgacc   1274
   tctagacttt gggcttccac ttgcttggct gagcaaccct gggaaaagtg acttcatccc   1334
   ttcggtccca agtttctcag tctgtaatgg gggatcccta caaaactg                 1382
<210> 23
   <211> 301
   <212> PRT
   <213> Homo sapiens
<400> 23
   Met Gln Thr Phe Thr Met Val Leu Glu Glu Ile Trp Thr Ser Leu Phe
    1               5                  10                  15
   Met Trp Phe Phe Tyr Ala Leu Ile Pro Cys Leu Leu Thr Asp Glu Val
               20                  25                  30
   Ala Ile Leu Pro Ala Pro Gln Asn Leu Ser Val Leu Ser Thr Asn Met
           35                  40                  45
   Lys His Leu Leu Met Trp Ser Pro Val Ile Ala Pro Gly Glu Thr Val
       50                  55                  60
   Tyr Tyr Ser Val Glu Tyr Gln Gly Glu Tyr Glu Ser Leu Tyr Thr Ser
   65                  70                  75                  80
   His Ile Trp Ile Pro Ser Ser Trp Cys Ser Leu Thr Glu Gly Pro Glu
                   85                  90                  95
   Cys Asp Val Thr Asp Asp Ile Thr Ala Thr Val Pro Tyr Asn Leu Arg
               100                 105                 110
   Val Arg Ala Thr Leu Gly Ser Gln Thr Ser Ala Trp Ser Ile Leu Lys
           115                 120                 125
   His Pro Phe Asn Arg Asn Ser Thr Ile Leu Thr Arg Pro Gly Met Glu
       130                 135                 140
   Ile Pro Lys His Gly Phe His Leu Val Ile Glu Leu Glu Asp Leu Gly
   145                 150                 155                 160
   Pro Gln Phe Glu Phe Leu Val Ala Tyr Trp Thr Arg Glu Pro Gly Ala
                   165                 170                 175
   Glu Glu His Val Lys Met Val Arg Ser Gly Gly Ile Pro Val His Leu
               180                 185                 190
   Glu Thr Met Glu Pro Gly Ala Ala Tyr Cys Val Lys Ala Gln Thr Phe
           195                 200                 205
   Val Lys Ala Ile Gly Arg Tyr Ser Ala Phe Ser Gln Thr Glu Cys Val
       210                 215                 220
   Glu Val Gln Gly Glu Ala Ile Pro Leu Val Leu Ala Leu Phe Ala Phe
   225                 230                 235                 240
   Val Gly Phe Met Leu Ile Leu Val Val Val Pro Leu Phe Val Trp Lys
                   245                 250                 255
   Met Gly Arg Leu Leu Gln Tyr Ser Cys Cys Pro Val Val Val Leu Pro
               260                 265                 270
   Asp Thr Leu Lys Ile Thr Asn Ser Pro Gln Val Asn Gln Leu Gln Lys
           275                 280                 285
   Gly Gly Gly Gly Cys Leu Cys His Gly Cys Asp Val Ser
       290                 295                 300
<210> 24
   <211> 1764
   <212> DNA
   <213> Homo sapiens
<220>
   <221> CDS
   <222> (34)...(1752)
<400> 24
   tagaggccaa gggagggctc tgtgccagcc ccg atg agg acg ctg ctg acc atc      54
                                        Met Arg Thr Leu Leu Thr Ile
                                        1               5
   ttg act gtg gga tcc ctg gct gct cac gcc cct gag gac ccc tcg gat      102
   Leu Thr Val Gly Ser Leu Ala Ala His Ala Pro Glu Asp Pro Ser Asp
           10                  15                  20
   ctg ctc cag cac gtg aaa ttc cag tcc agc aac ttt gaa aac atc ctg      150
   Leu Leu Gln His Val Lys Phe Gln Ser Ser Asn Phe Glu Asn Ile Leu
       25                  30                  35
   acg tgg gac agc ggg cca gag ggc acc cca gac acg gtc tac agc atc      198
   Thr Trp Asp Ser Gly Pro Glu Gly Thr Pro Asp Thr Val Tyr Ser Ile
   40                  45                  50                  55
   gag tat aag acg tac gga gag agg gac tgg gtg gca aag aag ggc tgt      246
   Glu Tyr Lys Thr Tyr Gly Glu Arg Asp Trp Val Ala Lys Lys Gly Cys
                   60                  65                  70
   cag cgg atc acc cgg aag tcc tgc aac ctg acg gtg gag acg ggc aac      294
   Gln Arg Ile Thr Arg Lys Ser Cys Asn Leu Thr Val Glu Thr Gly Asn
               75                  80                  85
   ctc acg gag ctc tac tat gcc agg gtc acc gct gtc agt gcg gga ggc      342
   Leu Thr Glu Leu Tyr Tyr Ala Arg Val Thr Ala Val Ser Ala Gly Gly
           90                  95                  100
   cgg tca gcc acc aag atg act gac agg ttc agc tct ctg cag cac act      390
   Arg Ser Ala Thr Lys Met Thr Asp Arg Phe Ser Ser Leu Gln His Thr
       105                 110                 115
   acc ctc aag cca cct gat gtg acc tgt atc tcc aaa gtg aga tcg att      438
   Thr Leu Lys Pro Pro Asp Val Thr Cys Ile Ser Lys Val Arg Ser Ile
   120                 125                 130                 135
   cag atg att gtt cat cct acc ccc acg cca atc cgt gca ggc gat ggc      486
   Gln Met Ile Val His Pro Thr Pro Thr Pro Ile Arg Ala Gly Asp Gly
                   140                 145                 150
   cac cgg cta acc ctg gaa gac atc ttc cat gac ctg ttc tac cac tta       534
   His Arg Leu Thr Leu Glu Asp Ile Phe His Asp Leu Phe Tyr His Leu
               155                 160                 165
   gag ctc cag gtc aac cgc acc tac caa atg cac ctt gga ggg aag cag       582
   Glu Leu Gln Val Asn Arg Thr Tyr Gln Met His Leu Gly Gly Lys Gln
           170                 175                 180
   aga gaa tat gag ttc ttc ggc ctg acc cct gac aca gag ttc ctt ggc       630
   Arg Glu Tyr Glu Phe Phe Gly Leu Thr Pro Asp Thr Glu Phe Leu Gly
       185                 190                 195
   acc atc atg att tgc gtt ccc acc tgg gcc aag gag agt gcc ccc tac       678
   Thr Ile Met Ile Cys Val Pro Thr Trp Ala Lys Glu Ser Ala Pro Tyr
   200                 205                 210                 215
   atg tgc cga gtg aag aca ctg cca gac cgg aca tgg acc ggt gga ggc       726
   Met Cys Arg Val Lys Thr Leu Pro Asp Arg Thr Trp Thr Gly Gly Gly
                   220                 225                 230
   ggt tca ggc gga ggt ggc tct ggc ggt ggc gga tcg gcc tcc acc aag       774
   Gly Ser Gly Gly Gly Gly Ser Gly Gly Gly Gly Ser Ala Ser Thr Lys
               235                 240                 245
   ggc cca tcg gtc ttc ccc ctg gca ccc tcc tcc aag agc acc tct ggg       822
   Gly Pro Ser Val Phe Pro Leu Ala Pro Ser Ser Lys Ser Thr Ser Gly
           250                 255                 260
   ggc aca gcg gcc ctg ggc tgc ctg gtc aag gac tac ttc ccc gaa ccg       870
   Gly Thr Ala Ala Leu Gly Cys Leu Val Lys Asp Tyr Phe Pro Glu Pro
       265                 270                 275
   gtg acg gtg tcg tgg aac tca ggc gcc ctg acc agc ggc gtg cac acc       918
   Val Thr Val Ser Trp Asn Ser Gly Ala Leu Thr Ser Gly Val His Thr
   280                 285                 290                 295
   ttc ccg gct gtc cta cag tcc tca gga ctc tac tcc ctc agc agc gtg       966
   Phe Pro Ala Val Leu Gln Ser Ser Gly Leu Tyr Ser Leu Ser Ser Val
                   300                 305                 310
   gtg acc gtg ccc tcc agc agc ttg ggc acc cag acc tac atc tgc aac      1014
   Val Thr Val Pro Ser Ser Ser Leu Gly Thr Gln Thr Tyr Ile Cys Asn
               315                 320                 325
   gtg aat cac aag ccc agc aac acc aag gtg gac aag aaa gtt gag ccc      1062
   Val Asn His Lys Pro Ser Asn Thr Lys Val Asp Lys Lys Val Glu Pro
           330                 335                 340
   aaa tct tgt gac aaa act cac aca tgc cca ccg tgc cca gca cct gaa      1110
   Lys Ser Cys Asp Lys Thr His Thr Cys Pro Pro Cys Pro Ala Pro Glu
       345                 350                 355
   gcc gag ggg gca ccg tca gtc ttc ctc ttc ccc cca aaa ccc aag gac      1158
   Ala Glu Gly Ala Pro Ser Val Phe Leu Phe Pro Pro Lys Pro Lys Asp
   360                 365                 370                 375
   acc ctc atg atc tcc cgg acc cct gag gtc aca tgc gtg gtg gtg gac      1206
   Thr Leu Met Ile Ser Arg Thr Pro Glu Val Thr Cys Val Val Val Asp
                   380                 385                 390
   gtg agc cac gaa gac cct gag gtc aag ttc aac tgg tac gtg gac ggc      1254
   Val Ser His Glu Asp Pro Glu Val Lys Phe Asn Trp Tyr Val Asp Gly
               395                 400                 405
   gtg gag gtg cat aat gcc aag aca aag ccg cgg gag gag cag tac aac      1302
   Val Glu Val His Asn Ala Lys Thr Lys Pro Arg Glu Glu Gln Tyr Asn
           410                 415                 420
   agc acg tac cgt gtg gtc agc gtc ctc acc gtc ctg cac cag gac tgg      1350
   Ser Thr Tyr Arg Val Val Ser Val Leu Thr Val Leu His Gln Asp Trp
       425                 430                 435
   ctg aat ggc aag gag tac aag tgc aag gtc tcc aac aaa gcc ctc cca      1398
   Leu Asn Gly Lys Glu Tyr Lys Cys Lys Val Ser Asn Lys Ala Leu Pro
   440                 445                 450                 455
   tcc tcc atc gag aaa acc atc tcc aaa gcc aaa ggg cag ccc cga gaa      1446
   Ser Ser Ile Glu Lys Thr Ile Ser Lys Ala Lys Gly Gln Pro Arg Glu
                   460                 465                 470
   cca cag gtg tac acc ctg ccc cca tcc cgg gat gag ctg acc aag aac      1494
   Pro Gln Val Tyr Thr Leu Pro Pro Ser Arg Asp Glu Leu Thr Lys Asn
               475                 480                 485
   cag gtc agc ctg acc tgc ctg gtc aaa ggc ttc tat ccc agc gac atc      1542
   Gln Val Ser Leu Thr Cys Leu Val Lys Gly Phe Tyr Pro Ser Asp Ile
           490                 495                 500
   gcc gtg gag tgg gag agc aat ggg cag ccg gag aac aac tac aag acc      1590
   Ala Val Glu Trp Glu Ser Asn Gly Gln Pro Glu Asn Asn Tyr Lys Thr
       505                 510                 515
   acg cct ccc gtg ctg gac tcc gac ggc tcc ttc ttc ctc tac agc aag      1638
   Thr Pro Pro Val Leu Asp Ser Asp Gly Ser Phe Phe Leu Tyr Ser Lys
   520                 525                 530                 535
   ctc acc gtg gac aag agc agg tgg cag cag ggg aac gtc ttc tca tgc      1686
   Leu Thr Val Asp Lys Ser Arg Trp Gln Gln Gly Asn Val Phe Ser Cys
                   540                 545                 550
   tcc gtg atg cat gag gct ctg cac aac cac tac acg cag aag agc ctc      1734
   Ser Val Met His Glu Ala Leu His Asn His Tyr Thr Gln Lys Ser Leu
               555                 560                 565
   tcc ctg tct ccg ggt aaa taatctagat ct                                1764
   Ser Leu Ser Pro Gly Lys
           570
<210> 25
   <211> 573
   <212> PRT
   <213> Homo sapiens
<400> 25
   Met Arg Thr Leu Leu Thr Ile Leu Thr Val Gly Ser Leu Ala Ala His
    1               5                  10                  15
   Ala Pro Glu Asp Pro Ser Asp Leu Leu Gln His Val Lys Phe Gln Ser
               20                  25                  30
   Ser Asn Phe Glu Asn Ile Leu Thr Trp Asp Ser Gly Pro Glu Gly Thr
           35                  40                  45
   Pro Asp Thr Val Tyr Ser Ile Glu Tyr Lys Thr Tyr Gly Glu Arg Asp
           50                  55                  60
   Trp Val Ala Lys Lys Gly Cys Gln Arg Ile Thr Arg Lys Ser Cys Asn
   65                  70                  75                  80
   Leu Thr Val Glu Thr Gly Asn Leu Thr Glu Leu Tyr Tyr Ala Arg Val
                   85                  90                  95
   Thr Ala Val Ser Ala Gly Gly Arg Ser Ala Thr Lys Met Thr Asp Arg
               100                 105                 110
   Phe Ser Ser Leu Gln His Thr Thr Leu Lys Pro Pro Asp Val Thr Cys
           115                 120                 125
   Ile Ser Lys Val Arg Ser Ile Gln Met Ile Val His Pro Thr Pro Thr
       130                 135                 140
   Pro Ile Arg Ala Gly Asp Gly His Arg Leu Thr Leu Glu Asp Ile Phe
   145                 150                 155                 160
   His Asp Leu Phe Tyr His Leu Glu Leu Gln Val Asn Arg Thr Tyr Gln
                   165                 170                 175
   Met His Leu Gly Gly Lys Gln Arg Glu Tyr Glu Phe Phe Gly Leu Thr
               180                 185                 190
   Pro Asp Thr Glu Phe Leu Gly Thr Ile Met Ile Cys Val Pro Thr Trp
           195                 200                 205
   Ala Lys Glu Ser Ala Pro Tyr Met Cys Arg Val Lys Thr Leu Pro Asp
       210                 215                 220
   Arg Thr Trp Thr Gly Gly Gly Gly Ser Gly Gly Gly Gly Ser Gly Gly
   225                 230                 235                 240
   Gly Gly Ser Ala Ser Thr Lys Gly Pro Ser Val Phe Pro Leu Ala Pro
                   245                 250                 255
   Ser Ser Lys Ser Thr Ser Gly Gly Thr Ala Ala Leu Gly Cys Leu Val
               260                 265                 270
   Lys Asp Tyr Phe Pro Glu Pro Val Thr Val Ser Trp Asn Ser Gly Ala
           275                 280                 285
   Leu Thr Ser Gly Val His Thr Phe Pro Ala Val Leu Gln Ser Ser Gly
       290                 295                 300
   Leu Tyr Ser Leu Ser Ser Val Val Thr Val Pro Ser Ser Ser Leu Gly
   305                 310                 315                 320
   Thr Gln Thr Tyr Ile Cys Asn Val Asn His Lys Pro Ser Asn Thr Lys
                   325                 330                 335
   Val Asp Lys Lys Val Glu Pro Lys Ser Cys Asp Lys Thr His Thr Cys
               340                 345                 350
   Pro Pro Cys Pro Ala Pro Glu Ala Glu Gly Ala Pro Ser Val Phe Leu
           355                 360                 365
   Phe Pro Pro Lys Pro Lys Asp Thr Leu Met Ile Ser Arg Thr Pro Glu
       370                 375                 380
   Val Thr Cys Val Val Val Asp Val Ser His Glu Asp Pro Glu Val Lys
   385                 390                 395                 400
   Phe Asn Trp Tyr Val Asp Gly Val Glu Val His Asn Ala Lys Thr Lys
                   405                 410                 415
   Pro Arg Glu Glu Gln Tyr Asn Ser Thr Tyr Arg Val Val Ser Val Leu
               420                 425                 430
   Thr Val Leu His Gln Asp Trp Leu Asn Gly Lys Glu Tyr Lys Cys Lys
           435                 440                 445
   Val Ser Asn Lys Ala Leu Pro Ser Ser Ile Glu Lys Thr Ile Ser Lys
       450                 455                 460
   Ala Lys Gly Gln Pro Arg Glu Pro Gln Val Tyr Thr Leu Pro Pro Ser
   465                 470                 475                 480
   Arg Asp Glu Leu Thr Lys Asn Gln Val Ser Leu Thr Cys Leu Val Lys
                   485                 490                 495
   Gly Phe Tyr Pro Ser Asp Ile Ala Val Glu Trp Glu Ser Asn Gly Gln
               500                 505                 510
   Pro Glu Asn Asn Tyr Lys Thr Thr Pro Pro Val Leu Asp Ser Asp Gly
           515                 520                 525
   Ser Phe Phe Leu Tyr Ser Lys Leu Thr Val Asp Lys Ser Arg Trp Gln
       530                 535                 540
   Gln Gly Asn Val Phe Ser Cys Ser Val Met His Glu Ala Leu His Asn
           545                     550                     555                     560
   His Tyr Thr Gln Lys Ser Leu Ser Leu Ser Pro Gly Lys
                   565                     570
<210> 26
   <211> 556
   <212> PRT
   <213> Homo sapiens
<400> 26
   Pro Glu Asp Pro Ser Asp Leu Leu Gln His Val Lys Phe Gln Ser Ser
   1               5                   10                  15
   Asn Phe Glu Asn Ile Leu Thr Trp Asp Ser Gly Pro Glu Gly Thr Pro
               20                  25                  30
   Asp Thr Val Tyr Ser Ile Glu Tyr Lys Thr Tyr Gly Glu Arg Asp Trp
           35                  40                  45
   Val Ala Lys Lys Gly Cys Gln Arg Ile Thr Arg Lys Ser Cys Asn Leu
       50                  55                  60
   Thr Val Glu Thr Gly Asn Leu Thr Glu Leu Tyr Tyr Ala Arg Val Thr
   65                  70                  75                  80
   Ala Val Ser Ala Gly Gly Arg Ser Ala Thr Lys Met Thr Asp Arg Phe
                   85                  90                  95
   Ser Ser Leu Gln His Thr Thr Leu Lys Pro Pro Asp Val Thr Cys Ile
               100                 105                 110
   Ser Lys Val Arg Ser Ile Gln Met Ile Val His Pro Thr Pro Thr Pro
           115                 120                 125
   Ile Arg Ala Gly Asp Gly His Arg Leu Thr Leu Glu Asp Ile Phe His
       130                 135                 140
   Asp Leu Phe Tyr His Leu Glu Leu Gln Val Asn Arg Thr Tyr Gln Met
   145                 150                 155                 160
   His Leu Gly Gly Lys Gln Arg Glu Tyr Glu Phe Phe Gly Leu Thr Pro
                   165                 170                 175
   Asp Thr Glu Phe Leu Gly Thr Ile Met Ile Cys Val Pro Thr Trp Ala
               180                 185                 190
   Lys Glu Ser Ala Pro Tyr Met Cys Arg Val Lys Thr Leu Pro Asp Arg
           195                 200                 205
   Thr Trp Thr Gly Gly Gly Gly Ser Gly Gly Gly Gly Ser Gly Gly Gly
       210                 215                 220
   Gly Ser Ala Ser Thr Lys Gly Pro Ser Val Phe Pro Leu Ala Pro Ser
   225                 230                 235                 240
   Ser Lys Ser Thr Ser Gly Gly Thr Ala Ala Leu Gly Cys Leu Val Lys
                   245                 250                 255
   Asp Tyr Phe Pro Glu Pro Val Thr Val Ser Trp Asn Ser Gly Ala Leu
               260                 265                 270
   Thr Ser Gly Val His Thr Phe Pro Ala Val Leu Gln Ser Ser Gly Leu
           275                 280                 285
   Tyr Ser Leu Ser Ser Val Val Thr Val Pro Ser Ser Ser Leu Gly Thr
       290                 295                 300
   Gln Thr Tyr Ile Cys Asn Val Asn His Lys Pro Ser Asn Thr Lys Val
   305                 310                 315                 320
   Asp Lys Lys Val Glu Pro Lys Ser Cys Asp Lys Thr His Thr Cys Pro
                   325                 330                 335
   Pro Cys Pro Ala Pro Glu Ala Glu Gly Ala Pro Ser Val Phe Leu Phe
               340                 345                 350
   Pro Pro Lys Pro Lys Asp Thr Leu Met Ile Ser Arg Thr Pro Glu Val
           355                 360                 365
   Thr Cys Val Val Val Asp Val Ser His Glu Asp Pro Glu Val Lys Phe
       370                 375                 380
   Asn Trp Tyr Val Asp Gly Val Glu Val His Asn Ala Lys Thr Lys Pro
   385                 390                 395                 400
   Arg Glu Glu Gln Tyr Asn Ser Thr Tyr Arg Val Val Ser Val Leu Thr
                   405                 410                 415
   Val Leu His Gln Asp Trp Leu Asn Gly Lys Glu Tyr Lys Cys Lys Val
               420                 425                 430
   Ser Asn Lys Ala Leu Pro Ser Ser Ile Glu Lys Thr Ile Ser Lys Ala
           435                 440                 445
   Lys Gly Gln Pro Arg Glu Pro Gln Val Tyr Thr Leu Pro Pro Ser Arg
       450                 455                 460
   Asp Glu Leu Thr Lys Asn Gln Val Ser Leu Thr Cys Leu Val Lys Gly
   465                 470                 475                 480
   Phe Tyr Pro Ser Asp Ile Ala Val Glu Trp Glu Ser Asn Gly Gln Pro
                   485                 490                 495
   Glu Asn Asn Tyr Lys Thr Thr Pro Pro Val Leu Asp Ser Asp Gly Ser
               500                 505                 510
   Phe Phe Leu Tyr Ser Lys Leu Thr Val Asp Lys Ser Arg Trp Gln Gln
           515                 520                 525
   Gly Asn Val Phe Ser Cys Ser Val Met His Glu Ala Leu His Asn His
       530                 535                 540
   Tyr Thr Gln Lys Ser Leu Ser Leu Ser Pro Gly Lys
   545                 550                 555
<210> 27
   <211> 1081
   <212> DNA
   <213> Homo sapiens
<220>
   <221> CDS
   <222> (9)...(1067)
<400> 27
   ggccggcc atg cag act ttc aca atg gtt cta gaa gaa atc tgg aca agt       50
            Met Gln Thr Phe Thr Met Val Leu Glu Glu Ile Trp Thr Ser
             1               5                  10
   ctt ttc atg tgg ttt ttc tac gca ttg att cca tgt ttg ctc aca gat        98
   Leu Phe Met Trp Phe Phe Tyr Ala Leu Ile Pro Cys Leu Leu Thr Asp
    15                  20                  25                  30
   gaa gtg gcc att ctg cct gcc cct cag aac ctc tct gta ctc tca acc       146
   Glu Val Ala Ile Leu Pro Ala Pro Gln Asn Leu Ser Val Leu Ser Thr
                    35                  40                  45
   aac atg aag cat ctc ttg atg tgg agc cca gtg atc gcg cct gga gaa       194
   Asn Met Lys His Leu Leu Met Trp Ser Pro Val Ile Ala Pro Gly Glu
                50                  55                  60
   aca gtg tac tat tct gtc gaa tac cag ggg gag tac gag agc ctg tac       242
   Thr Val Tyr Tyr Ser Val Glu Tyr Gln Gly Glu Tyr Glu Ser Leu Tyr
            65                  70                  75
   acg agc cac atc tgg atc ccc agc agc tgg tgc tca ctc act gaa ggt       290
   Thr Ser His Ile Trp Ile Pro Ser Ser Trp Cys Ser Leu Thr Glu Gly
        80                  85                  90
   cct gag tgt gat gtc act gat gac atc acg gcc act gtg cca tac aac       338
   Pro Glu Cys Asp Val Thr Asp Asp Ile Thr Ala Thr Val Pro Tyr Asn
    95                 100                 105                 110
   ctt cgt gtc agg gcc aca ttg ggc tca cag acc tca gcc tgg agc atc       386
   Leu Arg Val Arg Ala Thr Leu Gly Ser Gln Thr Ser Ala Trp Ser Ile
                   115                 120                 125
   ctg aag cat ccc ttt aat aga aac tca acc atc ctt acc cga cct ggg       434
   Leu Lys His Pro Phe Asn Arg Asn Ser Thr Ile Leu Thr Arg Pro Gly
               130                 135                 140
   atg gag atc ccc aaa cat ggc ttc cac ctg gtt att gag ctg gag gac      482
   Met Glu Ile Pro Lys His Gly Phe His Leu Val Ile Glu Leu Glu Asp
           145                 150                 155
   ctg ggg ccc cag ttt gag ttc ctt gtg gcc tac tgg acg agg gag cct      530
   Leu Gly Pro Gln Phe Glu Phe Leu Val Ala Tyr Trp Thr Arg Glu Pro
       160                 165                 170
   ggt gcc gag gaa cat gtc aaa atg gtg agg agt ggg ggt att cca gtg      578
   Gly Ala Glu Glu His Val Lys Met Val Arg Ser Gly Gly Ile Pro Val
   175                 180                 185                 190
   cac cta gaa acc atg gag cca ggg gct gca tac tgt gtg aag gcc cag      626
   His Leu Glu Thr Met Glu Pro Gly Ala Ala Tyr Cys Val Lys Ala Gln
                   195                 200                 205
   aca ttc gtg aag gcc att ggg agg tac agc gcc ttc agc cag aca gaa      674
   Thr Phe Val Lys Ala Ile Gly Arg Tyr Ser Ala Phe Ser Gln Thr Glu
               210                 215                 220
   tgt gtg gag gtg caa gga gag gcc gga ggt ggt ggc agt gga ggc ggc      722
   Cys Val Glu Val Gln Gly Glu Ala Gly Gly Gly Gly Ser Gly Gly Gly
           225                 230                 235
   ggt agc gga ggc ggt ggc agt cga act gtg gct gca cca tct gtc ttc      770
   Gly Ser Gly Gly Gly Gly Ser Arg Thr Val Ala Ala Pro Ser Val Phe
       240                 245                 250
   atc ttc ccg cca tct gat gag cag ttg aaa tct gga act gcc tct gtt      818
   Ile Phe Pro Pro Ser Asp Glu Gln Leu Lys Ser Gly Thr Ala Ser Val
   255                 260                 265                 270
   gtg tgc ctg ctg aat aac ttc tat ccc aga gag gcc aaa gta cag tgg      866
   Val Cys Leu Leu Asn Asn Phe Tyr Pro Arg Glu Ala Lys Val Gln Trp
                   275                 280                 285
   aag gtg gat aac gcc ctc caa tcg ggt aac tcc cag gag agt gtc aca      914
   Lys Val Asp Asn Ala Leu Gln Ser Gly Asn Ser Gln Glu Ser Val Thr
               290                 295                 300
   gag cag gac agc aag gac agc acc tac agc ctc agc agc acc ctg acg      962
   Glu Gln Asp Ser Lys Asp Ser Thr Tyr Ser Leu Ser Ser Thr Leu Thr
           305                 310                 315
   ctg agc aaa gca gac tac gag aaa cac aaa gtc tac gcc tgc gaa gtc      1010
   Leu Ser Lys Ala Asp Tyr Glu Lys His Lys Val Tyr Ala Cys Glu Val
       320                 325                 330
   acc cat cag ggc ctg agc tcg ccc gtc aca aag agc ttc aac agg gga      1058
   Thr His Gln Gly Leu Ser Ser Pro Val Thr Lys Ser Phe Asn Arg Gly
   335                 340                 345                 350
   gag tgt taa tctagaggcg cgcc                                          1081
   Glu Cys  *
<210> 28
   <211> 352
   <212> PRT
   <213> Homo sapiens
<400> 28
   Met Gln Thr Phe Thr Met Val Leu Glu Glu Ile Trp Thr Ser Leu Phe
    1               5                  10                  15
   Met Trp Phe Phe Tyr Ala Leu Ile Pro Cys Leu Leu Thr Asp Glu Val
               20                  25                  30
   Ala Ile Leu Pro Ala Pro Gln Asn Leu Ser Val Leu Ser Thr Asn Met
           35                  40                  45
   Lys His Leu Leu Met Trp Ser Pro Val Ile Ala Pro Gly Glu Thr Val
       50                  55                  60
   Tyr Tyr Ser Val Glu Tyr Gln Gly Glu Tyr Glu Ser Leu Tyr Thr Ser
   65                  70                  75                  80
   His Ile Trp Ile Pro Ser Ser Trp Cys Ser Leu Thr Glu Gly Pro Glu
                   85                  90                  95
   Cys Asp Val Thr Asp Asp Ile Thr Ala Thr Val Pro Tyr Asn Leu Arg
               100                 105                 110
   Val Arg Ala Thr Leu Gly Ser Gln Thr Ser Ala Trp Ser Ile Leu Lys
           115                 120                 125
   His Pro Phe Asn Arg Asn Ser Thr Ile Leu Thr Arg Pro Gly Met Glu
       130                 135                 140
   Ile Pro Lys His Gly Phe His Leu Val Ile Glu Leu Glu Asp Leu Gly
   145                 150                 155                 160
   Pro Gln Phe Glu Phe Leu Val Ala Tyr Trp Thr Arg Glu Pro Gly Ala
                   165                 170                 175
   Glu Glu His Val Lys Met Val Arg Ser Gly Gly Ile Pro Val His Leu
               180                 185                 190
   Glu Thr Met Glu Pro Gly Ala Ala Tyr Cys Val Lys Ala Gln Thr Phe
           195                 200                 205
   Val Lys Ala Ile Gly Arg Tyr Ser Ala Phe Ser Gln Thr Glu Cys Val
       210                 215                 220
   Glu Val Gln Gly Glu Ala Gly Gly Gly Gly Ser Gly Gly Gly Gly Ser
   225                 230                 235                 240
   Gly Gly Gly Gly Ser Arg Thr Val Ala Ala Pro Ser Val Phe Ile Phe
                   245                 250                 255
   Pro Pro Ser Asp Glu Gln Leu Lys Ser Gly Thr Ala Ser Val Val Cys
               260                 265                 270
   Leu Leu Asn Asn Phe Tyr Pro Arg Glu Ala Lys Val Gln Trp Lys Val
           275                 280                 285
   Asp Asn Ala Leu Gln Ser Gly Asn Ser Gln Glu Ser Val Thr Glu Gln
       290                 295                 300
   Asp Ser Lys Asp Ser Thr Tyr Ser Leu Ser Ser Thr Leu Thr Leu Ser
   305                 310                 315                 320
   Lys Ala Asp Tyr Glu Lys His Lys Val Tyr Ala Cys Glu Val Thr His
                   325                 330                 335
   Gln Gly Leu Ser Ser Pro Val Thr Lys Ser Phe Asn Arg Gly Glu Cys
               340                 345                 350
<210> 29
   <211> 323
   <212> PRT
   <213> Homo sapiens
<400> 29
   Asp Glu Val Ala Ile Leu Pro Ala Pro Gln Asn Leu Ser Val Leu Ser
    1               5                  10                  15
   Thr Asn Met Lys His Leu Leu Met Trp Ser Pro Val Ile Ala Pro Gly
               20                  25                  30
   Glu Thr Val Tyr Tyr Ser Val Glu Tyr Gln Gly Glu Tyr Glu Ser Leu
           35                  40                  45
   Tyr Thr Ser His Ile Trp Ile Pro Ser Ser Trp Cys Ser Leu Thr Glu
       50                  55                  60
   Gly Pro Glu Cys Asp Val Thr Asp Asp Ile Thr Ala Thr Val Pro Tyr
   65                  70                  75                  80
   Asn Leu Arg Val Arg Ala Thr Leu Gly Ser Gln Thr Ser Ala Trp Ser
                   85                  90                  95
   Ile Leu Lys His Pro Phe Asn Arg Asn Ser Thr Ile Leu Thr Arg Pro
               100                 105                 110
   Gly Met Glu Ile Pro Lys His Gly Phe His Leu Val Ile Glu Leu Glu
           115                 120                 125
   Asp Leu Gly Pro Gln Phe Glu Phe Leu Val Ala Tyr Trp Thr Arg Glu
       130                 135                 140
   Pro Gly Ala Glu Glu His Val Lys Met Val Arg Ser Gly Gly Ile Pro
   145                 150                 155                 160
   Val His Leu Glu Thr Met Glu Pro Gly Ala Ala Tyr Cys Val Lys Ala
                   165                 170                 175
   Gln Thr Phe Val Lys Ala Ile Gly Arg Tyr Ser Ala Phe Ser Gln Thr
               180                 185                 190
   Glu Cys Val Glu Val Gln Gly Glu Ala Gly Gly Gly Gly Ser Gly Gly
           195                 200                 205
   Gly Gly Ser Gly Gly Gly Gly Ser Arg Thr Val Ala Ala Pro Ser Val
       210                 215                 220
   Phe Ile Phe Pro Pro Ser Asp Glu Gln Leu Lys Ser Gly Thr Ala Ser
   225                 230                 235                 240
   Val Val Cys Leu Leu Asn Asn Phe Tyr Pro Arg Glu Ala Lys Val Gln
                   245                 250                 255
   Trp Lys Val Asp Asn Ala Leu Gln Ser Gly Asn Ser Gln Glu Ser Val
               260                 265                 270
   Thr Glu Gln Asp Ser Lys Asp Ser Thr Tyr Ser Leu Ser Ser Thr Leu
           275                 280                 285
   Thr Leu Ser Lys Ala Asp Tyr Glu Lys His Lys Val Tyr Ala Cys Glu
       290                 295                 300
   Val Thr His Gln Gly Leu Ser Ser Pro Val Thr Lys Ser Phe Asn Arg
   305                 310                 315                 320
   Gly Glu Cys
<210> 30
   <211> 1714
   <212> DNA
   <213> Homo sapiens
<220>
   <221> CDS
   <222> (34)...(1707)
<400> 30
   tagaggccaa gggagggctc tgtgccagcc ccg atg agg acg ctg ctg acc atc      54
                                        Met Arg Thr Leu Leu Thr Ile
                                        1               5
   ttg act gtg gga tcc ctg gct gct cac gcc cct gag gac ccc tcg gat      102
   Leu Thr Val Gly Ser Leu Ala Ala His Ala Pro Glu Asp Pro Ser Asp
           10                  15                  20
   ctg ctc cag cac gtg aaa ttc cag tcc agc aac ttt gaa aac atc ctg      150
   Leu Leu Gln His Val Lys Phe Gln Ser Ser Asn Phe Glu Asn Ile Leu
       25                  30                  35
   acg tgg gac agc ggg cca gag ggc acc cca gac acg gtc tac agc atc      198
   Thr Trp Asp Ser Gly Pro Glu Gly Thr Pro Asp Thr Val Tyr Ser Ile
    40                  45                  50                  55
   gag tat aag acg tac gga gag agg gac tgg gtg gca aag aag ggc tgt      246
   Glu Tyr Lys Thr Tyr Gly Glu Arg Asp Trp Val Ala Lys Lys Gly Cys
                   60                  65                  70
   cag cgg atc acc cgg aag tcc tgc aac ctg acg gtg gag acg ggc aac      294
   Gln Arg Ile Thr Arg Lys Ser Cys Asn Leu Thr Val Glu Thr Gly Asn
               75                  80                  85
   ctc acg gag ctc tac tat gcc agg gtc acc gct gtc agt gcg gga ggc      342
   Leu Thr Glu Leu Tyr Tyr Ala Arg Val Thr Ala Val Ser Ala Gly Gly
           90                  95                  100
   cgg tca gcc acc aag atg act gac agg ttc agc tct ctg cag cac act      390
   Arg Ser Ala Thr Lys Met Thr Asp Arg Phe Ser Ser Leu Gln His Thr
       105                 110                 115
   acc ctc aag cca cct gat gtg acc tgt atc tcc aaa gtg aga tcg att      438
   Thr Leu Lys Pro Pro Asp Val Thr Cys Ile Ser Lys Val Arg Ser Ile
   120                 125                 130                 135
   cag atg att gtt cat cct acc ccc acg cca atc cgt gca ggc gat ggc      486
   Gln Met Ile Val His Pro Thr Pro Thr Pro Ile Arg Ala Gly Asp Gly
                   140                 145                 150
   cac cgg cta acc ctg gaa gac atc ttc cat gac ctg ttc tac cac tta      534
   His Arg Leu Thr Leu Glu Asp Ile Phe His Asp Leu Phe Tyr His Leu
               155                 160                 165
   gag ctc cag gtc aac cgc acc tac caa atg cac ctt gga ggg aag cag      582
   Glu Leu Gln Val Asn Arg Thr Tyr Gln Met His Leu Gly Gly Lys Gln
           170                 175                 180
   aga gaa tat gag ttc ttc ggc ctg acc cct gac aca gag ttc ctt ggc      630
   Arg Glu Tyr Glu Phe Phe Gly Leu Thr Pro Asp Thr Glu Phe Leu Gly
       185                 190                 195
   acc atc atg att tgc gtt ccc acc tgg gcc aag gag agt gcc ccc tac      678
   Thr Ile Met Ile Cys Val Pro Thr Trp Ala Lys Glu Ser Ala Pro Tyr
   200                 205                 210                 215
   atg tgc cga gtg aag aca ctg cca gac cgg aca tgg acc gct agc acc      726
   Met Cys Arg Val Lys Thr Leu Pro Asp Arg Thr Trp Thr Ala Ser Thr
                   220                 225                 230
   aag ggc cca tcg gtc ttc ccc ctg gca ccc tcc tcc aag agc acc tct      774
   Lys Gly Pro Ser Val Phe Pro Leu Ala Pro Ser Ser Lys Ser Thr Ser
               235                 240                 245
   ggg ggc aca gcg gcc ctg ggc tgc ctg gtc aag gac tac ttc ccc gaa      822
   Gly Gly Thr Ala Ala Leu Gly Cys Leu Val Lys Asp Tyr Phe Pro Glu
           250                 255                 260
   ccg gtg acg gtg tcg tgg aac tca ggc gcc ctg acc agc ggc gtg cac      870
   Pro Val Thr Val Ser Trp Asn Ser Gly Ala Leu Thr Ser Gly Val His
       265                 270                 275
   acc ttc ccg gct gtc cta cag tcc tca gga ctc tac tcc ctc agc agc      918
   Thr Phe Pro Ala Val Leu Gln Ser Ser Gly Leu Tyr Ser Leu Ser Ser
   280                 285                 290                 295
   gtg gtg acc gtg ccc tcc agc agc ttg ggc acc cag acc tac atc tgc      966
   Val Val Thr Val Pro Ser Ser Ser Leu Gly Thr Gln Thr Tyr Ile Cys
                       300                     305                     310
   aac gtg aat cac aag ccc agc aac acc aag gtg gac aag aaa gtt gag        1014
   Asn Val Asn His Lys Pro Ser Asn Thr Lys Val Asp Lys Lys Val Glu
               315                     320                     325
   ccc aaa tct tgt gac aaa act cac aca tgc cca ccg tgc cca gca cct        1062
   Pro Lys Ser Cys Asp Lys Thr His Thr Cys Pro Pro Cys Pro Ala Pro
           330                     335                     340
   gaa ctc ctg ggg gga ccg tca gtc ttc ctc ttc ccc cca aaa ccc aag        1110
   Glu Leu Leu Gly Gly Pro Ser Val Phe Leu Phe Pro Pro Lys Pro Lys
       345                     350                     355
   gac acc ctc atg atc tcc cgg acc cct gag gtc aca tgc gtg gtg gtg        1158
   Asp Thr Leu Met Ile Ser Arg Thr Pro Glu Val Thr Cys Val Val Val
   360                     365                     370                     375
   gac gtg agc cac gaa gac cct gag gtc aag ttc aac tgg tac gtg gac        1206
   Asp Val Ser His Glu Asp Pro Glu Val Lys Phe Asn Trp Tyr Val Asp
                   380                     385                     390
   ggc gtg gag gtg cat aat gcc aag aca aag ccg cgg gag gag cag tac        1254
   Gly Val Glu Val His Asn Ala Lys Thr Lys Pro Arg Glu Glu Gln Tyr
               395                     400                     405
   aac agc acg tac cgt gtg gtc agc gtc ctc acc gtc ctg cac cag gac        1302
   Asn Ser Thr Tyr Arg Val Val Ser Val Leu Thr Val Leu His Gln Asp
           410                     415                     420
   tgg ctg aat ggc aag gag tac aag tgc aag gtc tcc aac aaa gcc ctc        1350
   Trp Leu Asn Gly Lys Glu Tyr Lys Cys Lys Val Ser Asn Lys Ala Leu
       425                     430                     435
   cca gcc ccc atc gag aaa acc atc tcc aaa gcc aaa ggg cag ccc cga        1398
   Pro Ala Pro Ile Glu Lys Thr Ile Ser Lys Ala Lys Gly Gln Pro Arg
   440                     445                     450                     455
   gaa cca cag gtg tac acc ctg ccc cca tcc cgg gat gag ctg acc aag        1446
   Glu Pro Gln Val Tyr Thr Leu Pro Pro Ser Arg Asp Glu Leu Thr Lys
                   460                     465                     470
   aac cag gtc agc ctg acc tgc ctg gtc aaa ggc ttc tat ccc agc gac        1494
   Asn Gln Val Ser Leu Thr Cys Leu Val Lys Gly Phe Tyr Pro Ser Asp
               475                     480                     485
   atc gcc gtg gag tgg gag agc aat ggg cag ccg gag aac aac tac aag        1542
   Ile Ala Val Glu Trp Glu Ser Asn Gly Gln Pro Glu Asn Asn Tyr Lys
           490                     495                     500
   acc acg cct ccc gtg ctg gac tcc gac ggc tcc ttc ttc ctc tac agc        1590
   Thr Thr Pro Pro Val Leu Asp Ser Asp Gly Ser Phe Phe Leu Tyr Ser
       505                     510                     515
   aag ctc acc gtg gac aag agc agg tgg cag cag ggg aac gtc ttc tca        1638
   Lys Leu Thr Val Asp Lys Ser Arg Trp Gln Gln Gly Asn Val Phe Ser
   520                     525                     530                     535
   tgc tcc gtg atg cat gag gct ctg cac aac cac tac acg cag aag agc        1686
   Cys Ser Val Met His Glu Ala Leu His Asn His Tyr Thr Gln Lys Ser
                   540                     545                     550
   ctc tcc ctg tct ccg ggt aaa tgacgcg                                  1714
   Leu Ser Leu Ser Pro Gly Lys
               555
<210> 31
   <211> 558
   <212> PRT
   <213> Homo sapiens
<400> 31
   Met Arg Thr Leu Leu Thr Ile Leu Thr Val Gly Ser Leu Ala Ala His
    1               5                  10                  15
   Ala Pro Glu Asp Pro Ser Asp Leu Leu Gln His Val Lys Phe Gln Ser
               20                  25                  30
   Ser Asn Phe Glu Asn Ile Leu Thr Trp Asp Ser Gly Pro Glu Gly Thr
           35                  40                  45
   Pro Asp Thr Val Tyr Ser Ile Glu Tyr Lys Thr Tyr Gly Glu Arg Asp
       50                  55                  60
   Trp Val Ala Lys Lys Gly Cys Gln Arg Ile Thr Arg Lys Ser Cys Asn
   65                  70                  75                  80
   Leu Thr Val Glu Thr Gly Asn Leu Thr Glu Leu Tyr Tyr Ala Arg Val
                   85                  90                  95
   Thr Ala Val Ser Ala Gly Gly Arg Ser Ala Thr Lys Met Thr Asp Arg
               100                 105                 110
   Phe Ser Ser Leu Gln His Thr Thr Leu Lys Pro Pro Asp Val Thr Cys
           115                 120                 125
   Ile Ser Lys Val Arg Ser Ile Gln Met Ile Val His Pro Thr Pro Thr
       130                 135                 140
   Pro Ile Arg Ala Gly Asp Gly His Arg Leu Thr Leu Glu Asp Ile Phe
   145                 150                 155                 160
   His Asp Leu Phe Tyr His Leu Glu Leu Gln Val Asn Arg Thr Tyr Gln
                   165                 170                 175
   Met His Leu Gly Gly Lys Gln Arg Glu Tyr Glu Phe Phe Gly Leu Thr
               180                 185                 190
   Pro Asp Thr Glu Phe Leu Gly Thr Ile Met Ile Cys Val Pro Thr Trp
           195                 200                 205
   Ala Lys Glu Ser Ala Pro Tyr Met Cys Arg Val Lys Thr Leu Pro Asp
       210                 215                 220
   Arg Thr Trp Thr Ala Ser Thr Lys Gly Pro Ser Val Phe Pro Leu Ala
   225                 230                 235                 240
   Pro Ser Ser Lys Ser Thr Ser Gly Gly Thr Ala Ala Leu Gly Cys Leu
                   245                 250                 255
   Val Lys Asp Tyr Phe Pro Glu Pro Val Thr Val Ser Trp Asn Ser Gly
               260                 265                 270
   Ala Leu Thr Ser Gly Val His Thr Phe Pro Ala Val Leu Gln Ser Ser
           275                 280                 285
   Gly Leu Tyr Ser Leu Ser Ser Val Val Thr Val Pro Ser Ser Ser Leu
       290                 295                 300
   Gly Thr Gln Thr Tyr Ile Cys Asn Val Asn His Lys Pro Ser Asn Thr
   305                 310                 315                 320
   Lys Val Asp Lys Lys Val Glu Pro Lys Ser Cys Asp Lys Thr His Thr
                   325                 330                 335
   Cys Pro Pro Cys Pro Ala Pro Glu Leu Leu Gly Gly Pro Ser Val Phe
               340                 345                 350
   Leu Phe Pro Pro Lys Pro Lys Asp Thr Leu Met Ile Ser Arg Thr Pro
           355                 360                 365
   Glu Val Thr Cys Val Val Val Asp Val Ser His Glu Asp Pro Glu Val
       370                 375                 380
   Lys Phe Asn Trp Tyr Val Asp Gly Val Glu Val His Asn Ala Lys Thr
   385                 390                 395                 400
   Lys Pro Arg Glu Glu Gln Tyr Asn Ser Thr Tyr Arg Val Val Ser Val
                   405                     410                     415
   Leu Thr Val Leu His Gln Asp Trp Leu Asn Gly Lys Glu Tyr Lys Cys
               420                     425                     430
   Lys Val Ser Asn Lys Ala Leu Pro Ala Pro Ile Glu Lys Thr Ile Ser
           435                     440                     445
   Lys Ala Lys Gly Gln Pro Arg Glu Pro Gln Val Tyr Thr Leu Pro Pro
       450                     455                     460
   Ser Arg Asp Glu Leu Thr Lys Asn Gln Val Ser Leu Thr Cys Leu Val
   465                     470                     475                     480
   Lys Gly Phe Tyr Pro Ser Asp Ile Ala Val Glu Trp Glu Ser Asn Gly
                   485                     490                     495
   Gln Pro Glu Asn Asn Tyr Lys Thr Thr Pro Pro Val Leu Asp Ser Asp
               500                     505                     510
   Gly Ser Phe Phe Leu Tyr Ser Lys Leu Thr Val Asp Lys Ser Arg Trp
           515                     520                     525
   Gln Gln Gly Asn Val Phe Ser Cys Ser Val Met His Glu Ala Leu His
       530                     535                     540
   Asn His Tyr Thr Gln Lys Ser Leu Ser Leu Ser Pro Gly Lys
   545                     550                     555
<210> 32
   <211> 541
   <212> PRT
   <213> Homo sapiens
<400> 32
   Pro Glu Asp Pro Ser Asp Leu Leu Gln His Val Lys Phe Gln Ser Ser
    1               5                  10                  15
   Asn Phe Glu Asn Ile Leu Thr Trp Asp Ser Gly Pro Glu Gly Thr Pro
               20                  25                  30
   Asp Thr Val Tyr Ser Ile Glu Tyr Lys Thr Tyr Gly Glu Arg Asp Trp
           35                  40                  45
   Val Ala Lys Lys Gly Cys Gln Arg Ile Thr Arg Lys Ser Cys Asn Leu
       50                  55                  60
   Thr Val Glu Thr Gly Asn Leu Thr Glu Leu Tyr Tyr Ala Arg Val Thr
   65                  70                  75                  80
   Ala Val Ser Ala Gly Gly Arg Ser Ala Thr Lys Met Thr Asp Arg Phe
                   85                  90                  95
   Ser Ser Leu Gln His Thr Thr Leu Lys Pro Pro Asp Val Thr Cys Ile
               100                 105                 110
   Ser Lys Val Arg Ser Ile Gln Met Ile Val His Pro Thr Pro Thr Pro
           115                 120                 125
   Ile Arg Ala Gly Asp Gly His Arg Leu Thr Leu Glu Asp Ile Phe His
       130                 135                 140
   Asp Leu Phe Tyr His Leu Glu Leu Gln Val Asn Arg Thr Tyr Gln Met
   145                 150                 155                 160
   His Leu Gly Gly Lys Gln Arg Glu Tyr Glu Phe Phe Gly Leu Thr Pro
                   165                 170                 175
   Asp Thr Glu Phe Leu Gly Thr Ile Met Ile Cys Val Pro Thr Trp Ala
               180                 185                 190
   Lys Glu Ser Ala Pro Tyr Met Cys Arg Val Lys Thr Leu Pro Asp Arg
           195                 200                 205
   Thr Trp Thr Ala Ser Thr Lys Gly Pro Ser Val Phe Pro Leu Ala Pro
       210                 215                 220
   Ser Ser Lys Ser Thr Ser Gly Gly Thr Ala Ala Leu Gly Cys Leu Val
   225                 230                 235                 240
   Lys Asp Tyr Phe Pro Glu Pro Val Thr Val Ser Trp Asn Ser Gly Ala
                   245                 250                 255
   Leu Thr Ser Gly Val His Thr Phe Pro Ala Val Leu Gln Ser Ser Gly
               260                 265                 270
   Leu Tyr Ser Leu Ser Ser Val Val Thr Val Pro Ser Ser Ser Leu Gly
           275                 280                 285
   Thr Gln Thr Tyr Ile Cys Asn Val Asn His Lys Pro Ser Asn Thr Lys
       290                 295                 300
   Val Asp Lys Lys Val Glu Pro Lys Ser Cys Asp Lys Thr His Thr Cys
   305                 310                 315                 320
   Pro Pro Cys Pro Ala Pro Glu Leu Leu Gly Gly Pro Ser Val Phe Leu
                   325                 330                 335
   Phe Pro Pro Lys Pro Lys Asp Thr Leu Met Ile Ser Arg Thr Pro Glu
               340                 345                 350
   Val Thr Cys Val Val Val Asp Val Ser His Glu Asp Pro Glu Val Lys
           355                 360                 365
   Phe Asn Trp Tyr Val Asp Gly Val Glu Val His Asn Ala Lys Thr Lys
       370                 375                 380
   Pro Arg Glu Glu Gln Tyr Asn Ser Thr Tyr Arg Val Val Ser Val Leu
   385                 390                 395                 400
   Thr Val Leu His Gln Asp Trp Leu Asn Gly Lys Glu Tyr Lys Cys Lys
                   405                 410                 415
   Val Ser Asn Lys Ala Leu Pro Ala Pro Ile Glu Lys Thr Ile Ser Lys
               420                 425                 430
   Ala Lys Gly Gln Pro Arg Glu Pro Gln Val Tyr Thr Leu Pro Pro Ser
           435                 440                 445
   Arg Asp Glu Leu Thr Lys Asn Gln Val Ser Leu Thr Cys Leu Val Lys
       450                 455                 460
   Gly Phe Tyr Pro Ser Asp Ile Ala Val Glu Trp Glu Ser Asn Gly Gln
   465                 470                 475                 480
   Pro Glu Asn Asn Tyr Lys Thr Thr Pro Pro Val Leu Asp Ser Asp Gly
                   485                 490                 495
   Ser Phe Phe Leu Tyr Ser Lys Leu Thr Val Asp Lys Ser Arg Trp Gln
               500                 505                 510
   Gln Gly Asn Val Phe Ser Cys Ser Val Met His Glu Ala Leu His Asn
           515                 520                 525
   His Tyr Thr Gln Lys Ser Leu Ser Leu Ser Pro Gly Lys
       530                 535                 540
<210> 33
   <211> 1011
   <212> DNA
   <213> Homo sapiens
<220>
   <221> CDS
   <222> (1)...(1008)
<400> 33
   atg cag act ttc aca atg gtt cta gaa gaa atc tgg aca agt ctt ttc      48
   Met Gln Thr Phe Thr Met Val Leu Glu Glu Ile Trp Thr Ser Leu Phe
    1               5                  10                  15
   atg tgg ttt ttc tac gca ttg att cca tgt ttg ctc aca gat gaa gtg      96
   Met Trp Phe Phe Tyr Ala Leu Ile Pro Cys Leu Leu Thr Asp Glu Val
               20                  25                  30
   gcc att ctg cct gcc cct cag aac ctc tct gta ctc tca acc aac atg     144
   Ala Ile Leu Pro Ala Pro Gln Asn Leu Ser Val Leu Ser Thr Asn Met
           35                  40                  45
   aag cat ctc ttg atg tgg agc cca gtg atc gcg cct gga gaa aca gtg     192
   Lys His Leu Leu Met Trp Ser Pro Val Ile Ala Pro Gly Glu Thr Val
       50                  55                  60
   tac tat tct gtc gaa tac cag ggg gag tac gag agc ctg tac acg agc     240
   Tyr Tyr Ser Val Glu Tyr Gln Gly Glu Tyr Glu Ser Leu Tyr Thr Ser
    65                  70                  75                  80
   cac atc tgg atc ccc agc agc tgg tgc tca ctc act gaa ggt cct gag       288
   His Ile Trp Ile Pro Ser Ser Trp Cys Ser Leu Thr Glu Gly Pro Glu
                    85                  90                  95
   tgt gat gtc act gat gac atc acg gcc act gtg cca tac aac ctt cgt       336
   Cys Asp Val Thr Asp Asp Ile Thr Ala Thr Val Pro Tyr Asn Leu Arg
               100                 105                 110
   gtc agg gcc aca ttg ggc tca cag acc tca gcc tgg agc atc ctg aag       384
   Val Arg Ala Thr Leu Gly Ser Gln Thr Ser Ala Trp Ser Ile Leu Lys
           115                 120                 125
   cat ccc ttt aat aga aac tca acc atc ctt acc cga cct ggg atg gag       432
   His Pro Phe Asn Arg Asn Ser Thr Ile Leu Thr Arg Pro Gly Met Glu
       130                 135                 140
   atc acc aaa gat ggc ttc cac ctg gtt att gag ctg gag gac ctg ggg       480
   Ile Thr Lys Asp Gly Phe His Leu Val Ile Glu Leu Glu Asp Leu Gly
   145                 150                 155                 160
   ccc cag ttt gag ttc ctt gtg gcc tac tgg agg agg gag cct ggt gcc       528
   Pro Gln Phe Glu Phe Leu Val Ala Tyr Trp Arg Arg Glu Pro Gly Ala
                   165                 170                 175
   gag gaa cat gtc aaa atg gtg agg agt ggg ggt att cca gtg cac cta       576
   Glu Glu His Val Lys Met Val Arg Ser Gly Gly Ile Pro Val His Leu
               180                 185                 190
   gaa acc atg gag cca ggg gct gca tac tgt gtg aag gcc cag aca ttc       624
   Glu Thr Met Glu Pro Gly Ala Ala Tyr Cys Val Lys Ala Gln Thr Phe
           195                 200                 205
   gtg aag gcc att ggg agg tac agc gcc ttc agc cag aca gaa tgt gtg       672
   Val Lys Ala Ile Gly Arg Tyr Ser Ala Phe Ser Gln Thr Glu Cys Val
       210                 215                 220
   gag gtg caa gga gag gcc act gtg gct gca cca tct gtc ttc atc ttc       720
   Glu Val Gln Gly Glu Ala Thr Val Ala Ala Pro Ser Val Phe Ile Phe
   225                 230                 235                 240
   ccg cca tct gat gag cag ttg aaa tct ggt acc gcc tct gtt gtg tgc       768
   Pro Pro Ser Asp Glu Gln Leu Lys Ser Gly Thr Ala Ser Val Val Cys
                   245                 250                 255
   ctg ctg aat aac ttc tat ccc aga gag gcc aaa gta cag tgg aag gtg       816
   Leu Leu Asn Asn Phe Tyr Pro Arg Glu Ala Lys Val Gln Trp Lys Val
               260                 265                 270
   gat aac gcc ctc caa tcg ggt aac tcc cag gag agt gtc aca gag cag       864
   Asp Asn Ala Leu Gln Ser Gly Asn Ser Gln Glu Ser Val Thr Glu Gln
           275                 280                 285
   gac agc aag gac agc acc tac agc ctc agc agc acc ctg acg ctg agc       912
   Asp Ser Lys Asp Ser Thr Tyr Ser Leu Ser Ser Thr Leu Thr Leu Ser
       290                 295                 300
   aaa gca gac tac gag aaa cac aaa gtc tac gcc tgc gaa gtc acc cat       960
   Lys Ala Asp Tyr Glu Lys His Lys Val Tyr Ala Cys Glu Val Thr His
   305                 310                 315                 320
   cag ggc ctg agc tcg ccc gtc aca aag agc ttc aac agg gga gag tgt      1008
   Gln Gly Leu Ser Ser Pro Val Thr Lys Ser Phe Asn Arg Gly Glu Cys
                   325                 330                 335
   tag                                                                 1011
<210> 34
   <211> 336
   <212> PRT
   <213> Homo sapiens
<400> 34
   Met Gln Thr Phe Thr Met Val Leu Glu Glu Ile Trp Thr Ser Leu Phe
   1               5                   10                  15
   Met Trp Phe Phe Tyr Ala Leu Ile Pro Cys Leu Leu Thr Asp Glu Val
               20                  25                  30
   Ala Ile Leu Pro Ala Pro Gln Asn Leu Ser Val Leu Ser Thr Asn Met
           35                  40                  45
   Lys His Leu Leu Met Trp Ser Pro Val Ile Ala Pro Gly Glu Thr Val
       50                  55                  60
   Tyr Tyr Ser Val Glu Tyr Gln Gly Glu Tyr Glu Ser Leu Tyr Thr Ser
   65                  70                  75                  80
   His Ile Trp Ile Pro Ser Ser Trp Cys Ser Leu Thr Glu Gly Pro Glu
                   85                  90                  95
   Cys Asp Val Thr Asp Asp Ile Thr Ala Thr Val Pro Tyr Asn Leu Arg
               100                 105                 110
   Val Arg Ala Thr Leu Gly Ser Gln Thr Ser Ala Trp Ser Ile Leu Lys
           115                 120                 125
   His Pro Phe Asn Arg Asn Ser Thr Ile Leu Thr Arg Pro Gly Met Glu
       130                 135                 140
   Ile Thr Lys Asp Gly Phe His Leu Val Ile Glu Leu Glu Asp Leu Gly
   145                 150                 155                 160
   Pro Gln Phe Glu Phe Leu Val Ala Tyr Trp Arg Arg Glu Pro Gly Ala
                   165                 170                 175
   Glu Glu His Val Lys Met Val Arg Ser Gly Gly Ile Pro Val His Leu
               180                 185                 190
   Glu Thr Met Glu Pro Gly Ala Ala Tyr Cys Val Lys Ala Gln Thr Phe
           195                 200                 205
   Val Lys Ala Ile Gly Arg Tyr Ser Ala Phe Ser Gln Thr Glu Cys Val
       210                 215                 220
   Glu Val Gln Gly Glu Ala Thr Val Ala Ala Pro Ser Val Phe Ile Phe
   225                 230                 235                 240
   Pro Pro Ser Asp Glu Gln Leu Lys Ser Gly Thr Ala Ser Val Val Cys
                   245                 250                 255
   Leu Leu Asn Asn Phe Tyr Pro Arg Glu Ala Lys Val Gln Trp Lys Val
               260                 265                 270
   Asp Asn Ala Leu Gln Ser Gly Asn Ser Gln Glu Ser Val Thr Glu Gln
           275                 280                 285
   Asp Ser Lys Asp Ser Thr Tyr Ser Leu Ser Ser Thr Leu Thr Leu Ser
       290                 295                 300
   Lys Ala Asp Tyr Glu Lys His Lys Val Tyr Ala Cys Glu Val Thr His
   305                 310                 315                 320
   Gln Gly Leu Ser Ser Pro Val Thr Lys Ser Phe Asn Arg Gly Glu Cys
                   325                 330                 335
<210> 35
   <211> 307
   <212> PRT
   <213> Homo sapiens
<400> 35
   Asp Glu Val Ala Ile Leu Pro Ala Pro Gln Asn Leu Ser Val Leu Ser
   1 5 10 15
   Thr Asn Met Lys His Leu Leu Met Trp Ser Pro Val Ile Ala Pro Gly
   20 25 30
   Glu Thr Val Tyr Tyr Ser Val Glu Tyr Gln Gly Glu Tyr Glu Ser Leu
   35 40 45
   Tyr Thr Ser His Ile Trp Ile Pro Ser Ser Trp Cys Ser Leu Thr Glu
   50 55 60
   Gly Pro Glu Cys Asp Val Thr Asp Asp Ile Thr Ala Thr Val Pro Tyr
   65 70 75 80
   Asn Leu Arg Val Arg Ala Thr Leu Gly Ser Gln Thr Ser Ala Trp Ser
   85 90 95
   Ile Leu Lys His Pro Phe Asn Arg Asn Ser Thr Ile Leu Thr Arg Pro
   100 105 110
   Gly Met Glu Ile Thr Lys Asp Gly Phe His Leu Val Ile Glu Leu Glu
   115 120 125
   Asp Leu Gly Pro Gln Phe Glu Phe Leu Val Ala Tyr Trp Arg Arg Glu
   130 135 140
   Pro Gly Ala Glu Glu His Val Lys Met Val Arg Ser Gly Gly Ile Pro
   145 150 155 160
   Val His Leu Glu Thr Met Glu Pro Gly Ala Ala Tyr Cys Val Lys Ala
   165 170 175
   Gln Thr Phe Val Lys Ala Ile Gly Arg Tyr Ser Ala Phe Ser Gln Thr
   180 185 190
   Glu Cys Val Glu Val Gln Gly Glu Ala Thr Val Ala Ala Pro Ser Val
   195 200 205
   Phe Ile Phe Pro Pro Ser Asp Glu Gln Leu Lys Ser Gly Thr Ala Ser
   210 215 220
   Val Val Cys Leu Leu Asn Asn Phe Tyr Pro Arg Glu Ala Lys Val Gln
   225 230 235 240
   Trp Lys Val Asp Asn Ala Leu Gln Ser Gly Asn Ser Gln Glu Ser Val
   245 250 255
   Thr Glu Gln Asp Ser Lys Asp Ser Thr Tyr Ser Leu Ser Ser Thr Leu
   260 265 270
   Thr Leu Ser Lys Ala Asp Tyr Glu Lys His Lys Val Tyr Ala Cys Glu
   275 280 285
   Val Thr His Gln Gly Leu Ser Ser Pro Val Thr Lys Ser Phe Asn Arg
   290 295 300
   Gly Glu Cys
   305
<210> 36
   <211> 15
   <212> PRT
   <213> Homo sapiens
<400> 36
   Gly Gly Gly Gly Ser Gly Gly Gly Gly Ser Gly Gly Gly Gly Ser
   1 5 10 15
<210> 37
   <211> 32
   <212> DNA
   <213> Homo sapiens
<400> 37
   cgctcgagcc ttccgcaaac ctatgagatc ca 32
<210> 38
   <211> 33
   <212> DNA
   <213> Homo sapiens
<400> 38
   gcgaattcga gtctaccaaa tgcagacttt cac 33
<210> 39
   <211> 18
   <212> DNA
   <213> Mouse
<400> 39
   cgccgcgttc ccgagatg 18
<210> 40
   <211> 24
   <212> DNA
   <213> mouse
<400> 40
   ggatgaggca gggctgacaa agtt 24

## Description

Cytokines are soluble proteins that influence the growth and differentiation of many cell types. Their receptors are composed of one or more integral membrane proteins that bind the cytokine with high affinity and transduce this binding event to the cell through the cytoplasmic portions of the certain receptor subunits. Cytokine receptors have been grouped into several classes on the basis of similarities in their extracellular ligand binding domains. For example, the receptor chains responsible for binding and/or transducing the effect of interferons (IFNs) are members of the type II cytokine receptor family (CRF2), based upon a characteristic 200 residue extracellular domain. The demonstrated *in vivo* activities of these interferons illustrate the enormous clinical potential of, and need for, other cytokines, cytokine agonists, and cytokine antagonists. Some cytokines are involved in the inflammatory cascade and can promote such diseases as rheumatoid arthritis, Crohn's disease, psoriasis, heart disease etc. Thus, there is a need to discover cytokines and their receptors that are involved in inflammation. One can then use the isolated soluble receptors of the cytokine to inhibit the cytokine-mediated inflammation.

### BRIEF DESCRIPTION OF THE DRAWINGS

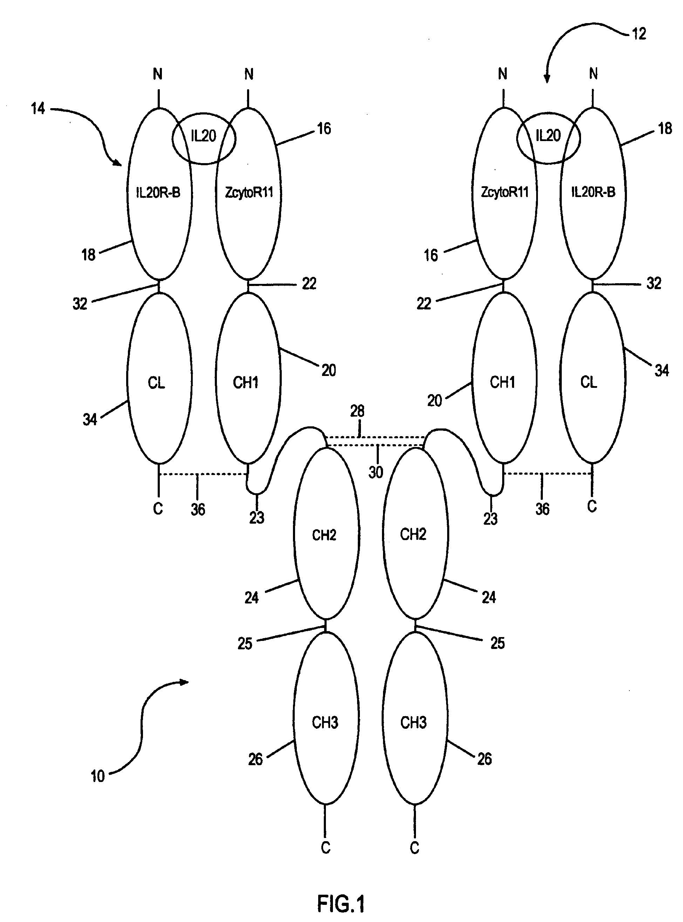

FIGURES 1-8 are schematic representations of different embodiments of the soluble receptor of the present invention.

The present invention provides an isolated soluble receptor comprising an IL-22R subunit and an IL-20RB subunit, wherein the IL-22R subunit comprises a polypeptide comprising an amino acid sequence selected from the group consisting of SEQ ID NOs: 12, 13, 25, 26, 31, and 32, and the IL-20RB subunit comprises a polypeptide comprising an amino acid sequence selected from the group consisting of SEQ ID NOs: 15, 16, 17, 18, 19, 20, 21, 23, 28, 29, 34, and 35.

The soluble receptor can be used to down-regulate IL-20 and thus treat inflammatory diseases such as psoriasis and inflammatory lung diseases.

The present invention also provides a method for producing a soluble receptor comprising extracellular domains of IL-22R and IL-20RB comprising (a) introducing into a host cell a first DNA sequence comprising a DNA sequence that encodes the extracellular portion of IL-22R and the DNA that encodes an immunoglobulin light chain constant region;(b) introducing into the host cell a second DNA construct comprising a DNA sequence that encodes the extracellular portion of IL-20RB and a DNA sequence that encodes an immunoglobulin heavy chain constant region domain; (c) growing the host cell in an appropriate growth medium under physiological conditions to allow production of a fusion protein comprising the extracellular domain of IL-22R and IL-20RB; and (d) isolating the polypeptide from the host cell, wherein the extracellular domain of IL-22R is a polypeptide selected from the group consisting of SEQ ID NOs: 12 and 13, and the extracellular domain of IL-20RB is a polypeptide selected from the group consisting of SEQ ID NOs: 16, 17, 18, 19, 20, and 21.

IL-20 was formally called "Zcyto10" (International patent publication NO. WO 99/27103) and has the amino acid sequences of SEQ ID NOs: 1-9. A heterodimeric receptor that binds to IL-20 is composed of two chains, an alpha chain and a beta chain. The alpha chain is referred to as IL-22R (formerly called Zcytor11).

See U.S. Patent No. 5,965,704. The beta chain, hereinafter referred to as IL-20RB, was formally called DIRS1. See International Patent Application No. PCT/US99/03735. The present invention is a soluble receptor comprised of the extracellular domain of IL-22R and the extracellular domain of IL-20RB,

The present invention encompasses an isolated soluble receptor as defined in the claims comprised of a 'IL-22R' subunit and an 'IL-20RB' subunit, wherein the IL-22R subunit is comprised of a polypeptide having an amino acid sequence selected from the group consisting of SEQ ID NOs: 11, 12 and 13, and the IL-20RB subunit is comprised of a polypeptide having an amino acid sequence selected from the group consisting of SEQ ID NOs: 14-23. The IL-22R and IL-20RB subunits are generally linked together by a polypeptide linker. The linking can be by any means but generally by a peptide bond or a disulfide bond between a polypeptide connected to the IL-22R subunit and a polypeptide connected to the IL-20RB subunit. The present application also describes isolated polynucleotides that encode the novel IL-22R and IL-20RB polypeptides of the present invention.

In one embodiment the IL-22R subunit is fused to the constant region of the heavy chain of an immunoglobulin (Ig) molecule or a portion thereof and the IL-20RB subunit is fused to the constant region of the light chain of an Ig molecule such that the constant region of the light chain is disulfide bonded to the constant region of the heavy chain, generally to a cysteine residue on the hinge region of the heavy chain. Also the opposite can occur, the IL-22R subunit can be fused to the constant region of the light chain of an Ig molecule and the IL-20RB subunit can be fused to the constant region of the heavy chain of an Ig molecule.

In one embodiment of the soluble receptor of the present invention, the IL-22R subunit fused to the constant region of the heavy chain is comprised of an amino acid sequence selected from the group consisting of SEQ ID NOs: 25, 26, 31 and 32 and the IL-20RB subunit fused to the constant region of the light chain of the Ig molecule is comprised of an amino acid sequence selected from the group consisting of SEQ ID NOs: 28 and 29.

The present application also describes a method for inhibiting interleukin-20 (IL-20) comprising administering to an individual a soluble IL-22R/IL-20RB heterodimeric polypeptide.

The present application also describes a method for inhibiting IL-20 comprising administering to an antibody that binds to IL-22R.

The present application further describes a polynucleotide encoding for the extracellular domain of IL-22R and the extracellular domain of IL-20RB. An example of such a polynucleotide is a vector or plasmid containing a polynucleotide that encodes for IL-22R and IL-20RB.

### Definitions

Prior to setting forth the invention in more detail, it may be helpful to the understanding thereof to define the following terms.

The terms "amino-terminal" and "carboxyl-terminal" are used herein to denote positions within polypeptides. Where the context allows, these terms are used with reference to a particular sequence or portion of a polypeptide to denote proximity or relative position. For example, a certain sequence positioned carboxyl-terminal to a reference sequence within a polypeptide is located proximal to the carboxyl terminus of the reference sequence, but is not necessarily at the carboxyl terminus of the complete polypeptide.

As used herein, the term "antibody fusion protein" refers to a recombinant molecule that comprises an antibody component and a therapeutic agent. Examples of therapeutic agents suitable for such fusion proteins include immunomodulators ("antibody-immunomodulator fusion protein") and toxins ("antibody-toxin fusion protein").

The term "complement/anti-complement pair" denotes non-identical moieties that form a non-covalently associated, stable pair under appropriate conditions. For instance, biotin and avidin (or streptavidin) are prototypical members of a complement/anti-complement pair. Other exemplary complement/anti-complement pairs include receptor/ligand pairs, antibody/antigen (or hapten or epitope) pairs, sense/antisense polynucleotide pairs, and the like. Where subsequent dissociation of the complement/anti-complement pair is desirable, the complement/anti-complement pair preferably has a binding affinity of <10⁹ M⁻¹.

The term "complements of a polynucleotide molecule" is a polynucleotide molecule having a complementary base sequence and reverse orientation as compared to a reference sequence.

The term "config" denotes a polynucleotide that has a contiguous stretch of identical or complementary sequence to another polynucleotide. Contiguous sequences are said to "overlap" a given stretch of polynucleotide sequence either in their entirety or along a partial stretch of the polynucleotide.

The term "degenerate nucleotide sequence" denotes a sequence of nucleotides that includes one or more degenerate codons (as compared to a reference polynucleotide molecule that encodes a polypeptide). Degenerate codons contain different triplets of nucleotides, but encode the same amino acid residue (i.e., GAU and GAC triplets each encode Asp).

The term "expression vector" is used to denote a DNA molecule, linear or circular, that comprises a segment encoding a polypeptide of interest operably linked to additional segments that provide for its transcription. Such additional segments include promoter and terminator sequences, and may also include one or more origins of replication, one or more selectable markers, an enhancer, a polyadenylation signal, etc. Expression vectors are generally derived from plasmid or viral DNA, or may contain elements of both.

The term "isolated", when applied to a polynucleotide, denotes that the polynucleotide has been removed from its natural genetic milieu and is thus free of other extraneous or unwanted coding sequences, and is in a form suitable for use within genetically engineered protein production systems. Such isolated molecules are those that are separated from their natural environment and include cDNA and genomic clones. Isolated DNA molecules of the present invention are free of other genes with which they are ordinarily associated, but may include naturally occurring 5' and 3' untranslated regions such as promoters and terminators. The identification of associated regions will be evident to one of ordinary skill in the art (see for example, Dynan and Tijan, Nature 316:774-78 (1985).

An "isolated" polypeptide or protein is a polypeptide or protein that is found in a condition other than its native environment, such as apart from blood and animal tissue. In a preferred form, the isolated polypeptide is substantially free of other polypeptides, particularly other polypeptides of animal origin. It is preferred to provide the polypeptides in a highly purified form, i.e. greater than 95% pure, more preferably greater than 99% pure. When used in this context, the term "isolated" does not exclude the presence of the same polypeptide in alternative physical forms, such as dimers or alternatively glycosylated or derivatized forms.

The term "operably linked", when referring to DNA segments, indicates that the segments are arranged so that they function in concert for their intended purposes, *e*.*g*., transcription initiates in the promoter and proceeds through the coding segment to the terminator.

A "polynucleotide" is a single- or double-stranded polymer of deoxyribonucleotide or ribonucleotide bases read from the 5' to the 3' end. Polynucleotides include RNA and DNA, and may be isolated from natural sources, synthesized *in vitro,* or prepared from a combination of natural and synthetic molecules. Sizes of polynucleotides are expressed as base pairs (abbreviated "bp"), nucleotides ("nt"), or kilobases ("kb"). Where the context allows, the latter two terms may describe polynucleotides that are single-stranded or double-stranded. When the term is applied to double-stranded molecules it is used to denote overall length and will be understood to be equivalent to the term "base pairs". It will be recognized by those skilled in the art that the two strands of a double-stranded polynucleotide may differ slightly in length and that the ends thereof may be staggered as a result of enzymatic cleavage; thus all nucleotides within a double-stranded polynucleotide molecule may not be paired. Such unpaired ends will in general not exceed 20 nucleotides in length.

A "polypeptide" is a polymer of amino acid residues joined by peptide bonds, whether produced naturally or synthetically. Polypeptides of less than about 10 amino acid residues are commonly referred to as "peptides".

The term "promoter" is used herein for its art-recognized meaning to denote a portion of a gene containing DNA sequences that provide for the binding of RNA polymerase and initiation of transcription. Promoter sequences are commonly, but not always, found in the 5' non-coding regions of genes.

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced, and will vary with the type of cell. Proteins are defined herein in terms of their amino acid backbone structures; substituents such as carbohydrate groups are generally not specified, but may be present nonetheless.

The term "receptor" denotes a cell-associated protein that binds to a bioactive molecule (i.e., a ligand) and mediates the effect of the ligand on the cell. Membrane-bound receptors are characterized by a multi-domain structure comprising an extracellular ligand-binding domain and an intracellular effector domain that is typically involved in signal transduction. Binding of ligand to receptor results in a conformational change in the receptor that causes an interaction between the effector domain and other molecule(s) in the cell. This interaction in turn leads to an alteration in the metabolism of the cell. Metabolic events that are linked to receptor-ligand interactions include gene transcription, phosphorylation, dephosphorylation, increases in cyclic AMP production, mobilization of cellular calcium, mobilization of membrane lipids, cell adhesion, hydrolysis of inositol lipids and hydrolysis of phospholipids. In general, receptors can be membrane bound, cytosolic or nuclear, monomeric (*e*.*g*., thyroid stimulating hormone receptor, beta-adrenergic receptor) or multimeric (*e*.*g*., PDGF receptor, growth hormone receptor, IL-3 receptor, GM-CSF receptor, G-CSF receptor, erythropoietin receptor and IL-6 receptor).

The term "secretory signal sequence" denotes a DNA sequence that encodes a polypeptide (a "secretory peptide") that, as a component of a larger polypeptide, directs the larger polypeptide through a secretory pathway of a cell in which it is synthesized. The larger polypeptide is commonly cleaved to remove the secretory peptide during transit through the secretory pathway.

The term "splice variant" is used herein to denote alternative forms of RNA transcribed from a gene. Splice variation arises naturally through use of alternative splicing sites within a transcribed RNA molecule, or less commonly between separately transcribed RNA molecules, and may result in several mRNAs transcribed from the same gene. Splice variants may encode polypeptides having altered amino acid sequence. The term splice variant is also used herein to denote a protein encoded by a splice variant of an mRNA transcribed from a gene.

Molecular weights and lengths of polymers determined by imprecise analytical methods (*e*.*g*., gel electrophoresis) will be understood to be approximate values. When such a value is expressed as "about" X or "approximately" X, the stated value of X will be understood to be accurate to ±10%.

As was stated above, IL-20 (formally called Zcyto10) is defined and methods for producing it and antibodies to IL-20 are contained in International Patent Application No. PCT/US98/25228, publication no. WO 99/27103,published November 25, 1998 and U.S. Patent Application No. 09/313,458 filed May 17, 1999. The polynucleotide and polypeptide of human IL-20 are represented by SEQ ID NOs: 1 - 4, and mouse IL-20 by SEQ ID NOs: 5-9.

A receptor that binds to IL-20 has been discovered and is a heterodimer comprised of the polypeptide termed 'IL-22R' and a polypeptide termed 'IL-20RB'. The IL-22R, also called ZcytoR11, polypeptide, nucleic acid that encodes it, antibodies to IL-22R, and methods for producing it are disclosed in U.S. Patent No. 5,965,704 issued October 12, 1999. SEQ ID NOs: 10 - 12 are the IL-22R polynucleotides and polypeptides. The extracellular domain of the human IL-22R is comprised of either SEQ ID NO: 12 or SEQ ID NO: 13.

The extracellular domain of IL-20RB (SEQ ID NOs: 14-15, and a variant SEQ ID NOs: 22 and 23) is comprised of a polypeptide selected from the group consisting of SEQ ID NOs: 16-21. Preferably, the extracellular domain of the IL-22R polypeptide and the extracellular domain of the IL-20RB polypeptide are covalently linked together. In a preferred embodiment one extracellular subunit polypeptide has a constant region of a heavy chain of an immunoglobulin fused to its carboxy terminus and the other extracellular subunit has a constant light chain of an immunoglobulin (Ig) fused to its carboxy terminus such that the two polypeptides come together to form a soluble receptor and a disulfide bond is formed between the heavy and the light Ig chains. In another embodiment, a peptide linker could be fused to the two carboxy-termini of the polypeptides to form a covalently bonded soluble receptor.

SEQ ID NOs: 24 and 25 are constructs of the extracellular domain of IL-22R fused to a mutated human immunoglobulin gamma 1 constant region. SEQ ID NO: 26 is the predicted mature sequence without the signal sequence. SEQ ID NOs: 27 and 28 are constructs of the extracellular domain of IL-20RB fused to wild type human immunoglobulin kappa light chain constant region. SEQ ID NO: 29 is the predicted mature sequence without the signal sequence. Figure 1 is a schematic representation of the heterotetramer.

SEQ ID NOs: 30 and 31 are constructs of the extracellular domain of IL-22R fused to a mutated human immunoglobulin gamma 1 constant region. SEQ ID NO: 32 is the predicted mature sequence without the signal sequence. SEQ ID NOs: 33 and 34 are constructs of the extracellular domain of IL-20RB fused to wild type human immunoglobulin kappa light chain constant region produced according to the procedure of example 12. SEQ ID NO: 35 is the predicted mature sequence without the signal sequence. The resultant heterotetramer does not have a polypeptide linker between the extracellular domains and the beginning of the Ig constant regions, 22 in Figure 1. Hereinafter, the term "extracellular domain of a receptor" means the extracellular domain of the receptor or a portion of the extracellular domain that is necessary for binding to its ligand, in this case the ligand being IL-20.

One can link together the extracellular domains of IL-22R and IL-20RB in a number of ways such that the resultant soluble receptor can bind to IL-20. Figures 1-8 illustrate a representative number of embodiments of the present invention. Common elements in each of the drawings are given the same number. Figure 1 represents the embodiment of the present invention of SEQ ID NOs: 24, 25, 26, 27, 28 and 29. The soluble receptor construct, designated 10, is comprised of two IL-20 binding site polypeptide chains designated 12 and 14. Each binding site is comprised of the extracellular domain of IL-22R, designated 16, and the extracellular domain of IL-20RB designated 18.

The extracellular domain, 16, of IL-22R is linked to the constant heavy one (CH1) domain, 20, of the human immunoglobulin gamma 1 heavy chain constant region via linker 22, which is SEQ ID NO: 36. The CH1 domain, 20, is then linked to the CH2 domain, 24, via hinge region 23. The CH2 domain, 24, is linked to the CH3 domain, 26, via hinge region 25.

Comparing the construct of Figure 1 with SEQ ID NO:25, the mature extracellular domain, 16, of IL-22R extends from amino acid residues 18, a proline, to and including amino acid residue 228, a threonine of SEQ ID NO:25. Polypeptide linker, 22, extends from amino acid residue 229, a glycine to and including amino acid residue 243, a serine, of SEQ ID NO:25. The CH1 domain, 22 of Figure 1, extends from amino acid residue 244, an alanine, to and including amino acid residue 341, a valine, of SEQ ID NO: 25. Hinge region 23 of Figure 1 extends from amino acid residue 342, a glutamic acid to and including amino acid residue 356, a proline, of SEQ ID NO: 25. Chains 12 and 14 are disulfide-bonded together by means of disulfide bonds 28 and 30. The disulfide bonds are formed between the heavy chains by the cysteine residues at positions 352 and 356 of SEQ ID NO: 25 of each of the two heavy chains.

Extracellular domain, 18, of IL-20RB is linked to the constant region of the human kappa light chain (CL), 34 of Figure 1 via polypeptide linker 32, which is the polypeptide SEQ ID NO: 36. The extracellular domain, 18, of IL-20RB extends from amino acid residue 30, a valine, to and including amino acid residue 230, an alanine, of SEQ ID NO: 28. Polypeptide linker, 32, extends from amino acid residue 231, a glycine, to and including amino acid residue 245, a serine, of SEQ ID NO: 28. The kappa constant light region, 34, extends from amino acid residue 246, an arginine, to and including the final amino acid residue 352, a cysteine, of SEQ ID NO: 28. The cysteine at position 352 of SEQ ID NO: 28 forms a disulfide bond, 36 in Figure 1, with the cysteine at position 346 of SEQ ID NO: 25. The constant light chain 34 is thus linked to the hinge region, 23, by disulfide bond, 36. In this way, the extracellular domain, 16, of IL-22R is linked to the extracellular domain, 18, of IL-20RB to form a soluble receptor.

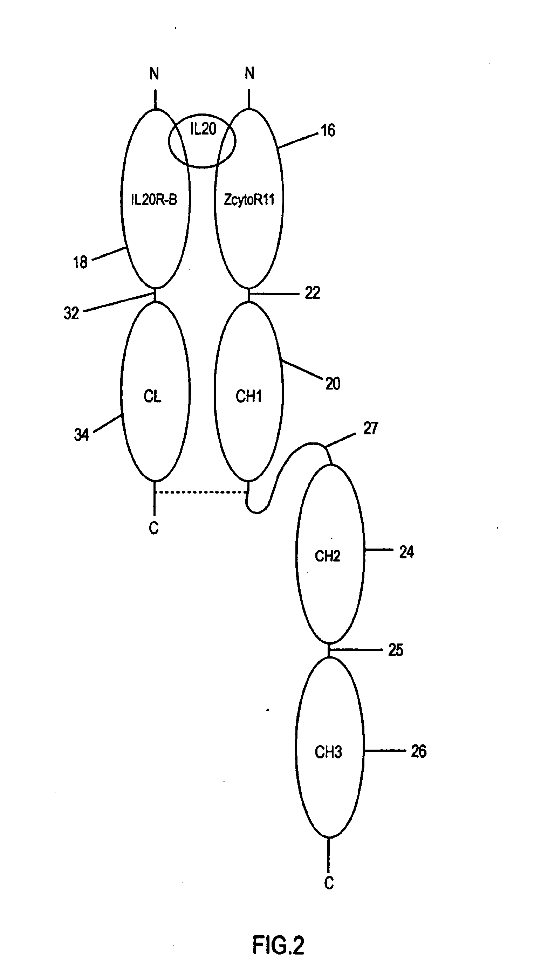

If the cysteine residues at positions 352 and 356 of SEQ ID NO: 25 were changed to different amino acid residues, the two IL-20 binding polypeptides, 12 and 14, would not be disulfide bonded together and would form a construct shown in Figure 2 having hinge region, 27.

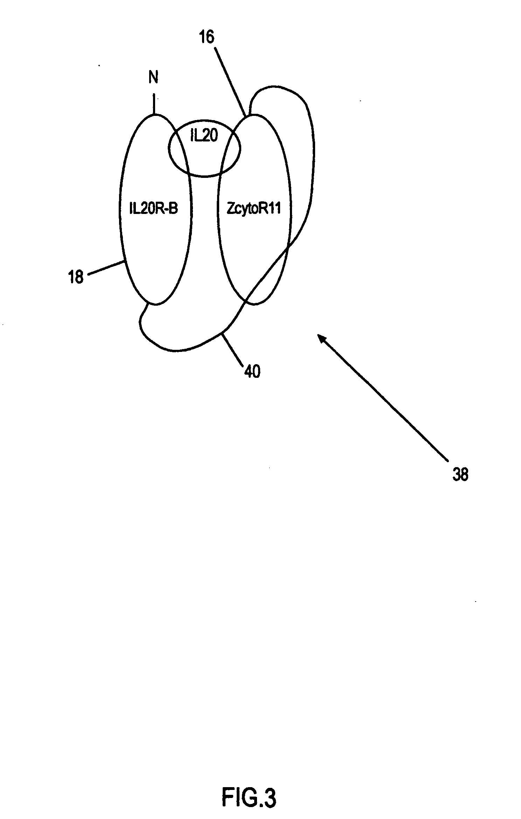

Figure 3 shows a very simple soluble receptor 38 of the present invention wherein extracellular domain, 16, of IL-22R is connected to the extracellular domain, 18, of IL-20RB by means of a polypeptide linker, 40. The polypeptide linker extends from the amino terminus of extracellular domain, 16, of IL-22R and is connected to the carboxyl terminus of the extracellular domain, 18, of IL-20RB. The polypeptide linker should be between 100-240 amino acids in length, preferably about 170 amino acid residues in length. A suitable linker would be comprised of glycine and serine residues. A possible linker would be multiple units of SEQ ID NO: 36, preferably about 12.

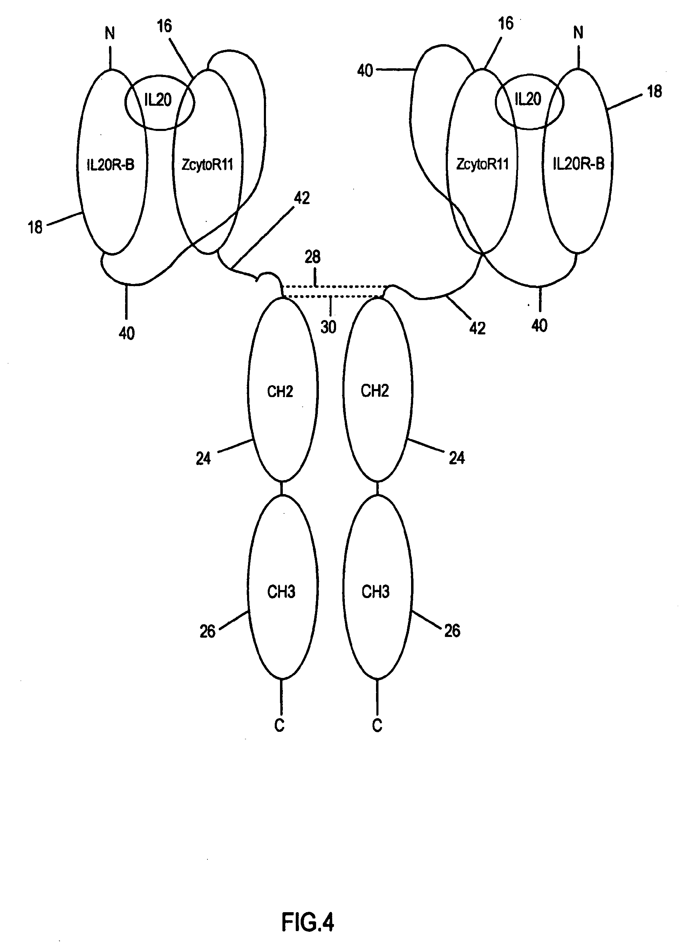

Figure 4 shows an embodiment that has the extracellular domain, 16, of IL-22R linked to the extracellular domain, 18, of IL-20RB by means of linker 40, as in Figure 3. While the extracellular domain, 16, of IL-22R is linked to the CH1 domain, 20, as in Figure 1 by means of polypeptide linker 42, which should be about 30 amino acid residues in length. An ideal linker would be comprised of glycine and serine as in SEQ ID NO: 72, and the hinge sequence, 23 of Figure 1.

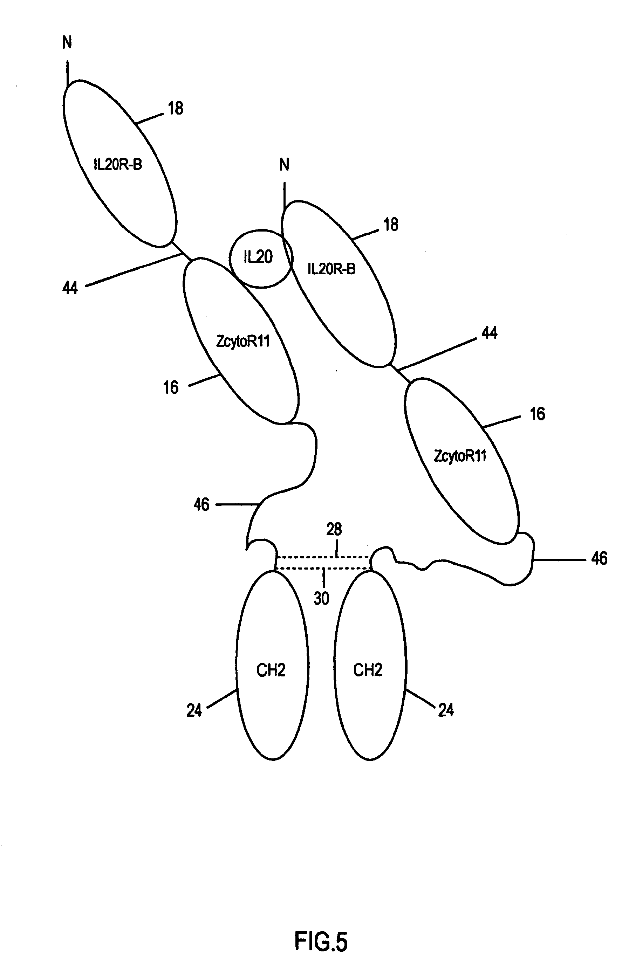

Figure 5 shows another possible embodiment of the present invention. In this embodiment, a polypeptide linker 44 of about 15 amino acid residue, *e*.*g*. SEQ ID NO: 36, links the carboxyl terminus of the extracellular domain, 18, of IL-20RB with the amino terminus of the extracellular domain, 16, of IL-22R. A polypeptide linker 46 of about 30 amino acid residues extends from the carboxy terminus of the extracellular domain, 16, of IL-22R to the CH2 domain. The carboxyl terminus of linker 46 would preferably be comprised of the hinge region extending from amino acid residue 342, a glutamic acid to and including amino acid residue 356, a proline, of SEQ ID NO: 25. Nonetheless, polypeptide linker 46 would ideally have at least one cysteine residue at its carboxyl terminus so a disulfide bond could be formed.

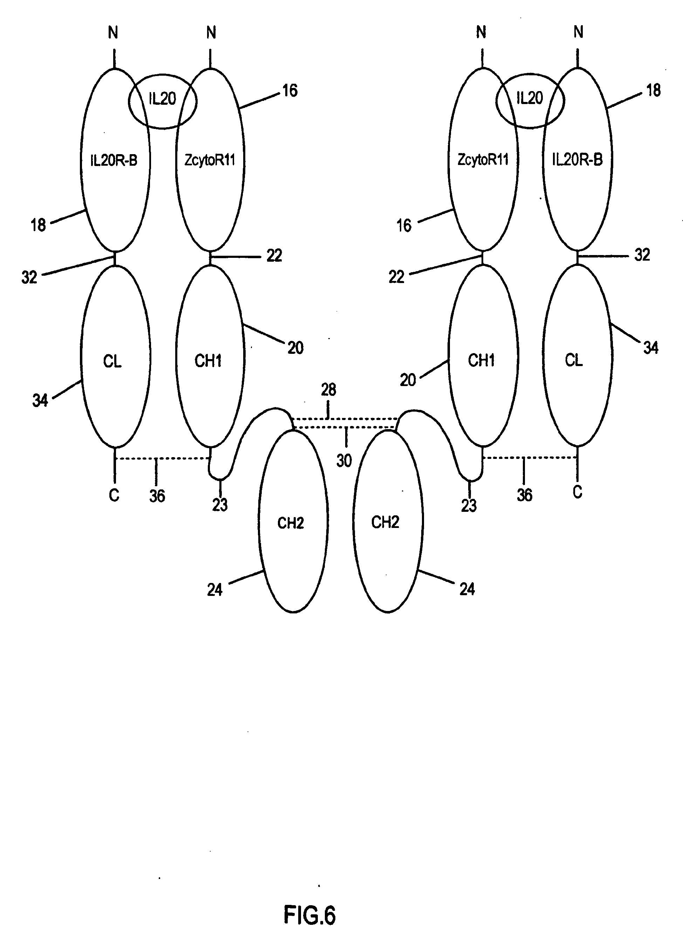

The soluble IL-20 receptor of Figure 6 is identical to that of Figure 1 except for the CH3 domain, 26 of Figure 1, is not present on the embodiment of Figure 6. The CH3 region begins at amino acid residue 467, a glycine, and extends to the last residue 573 of SEQ ID NO: 25.

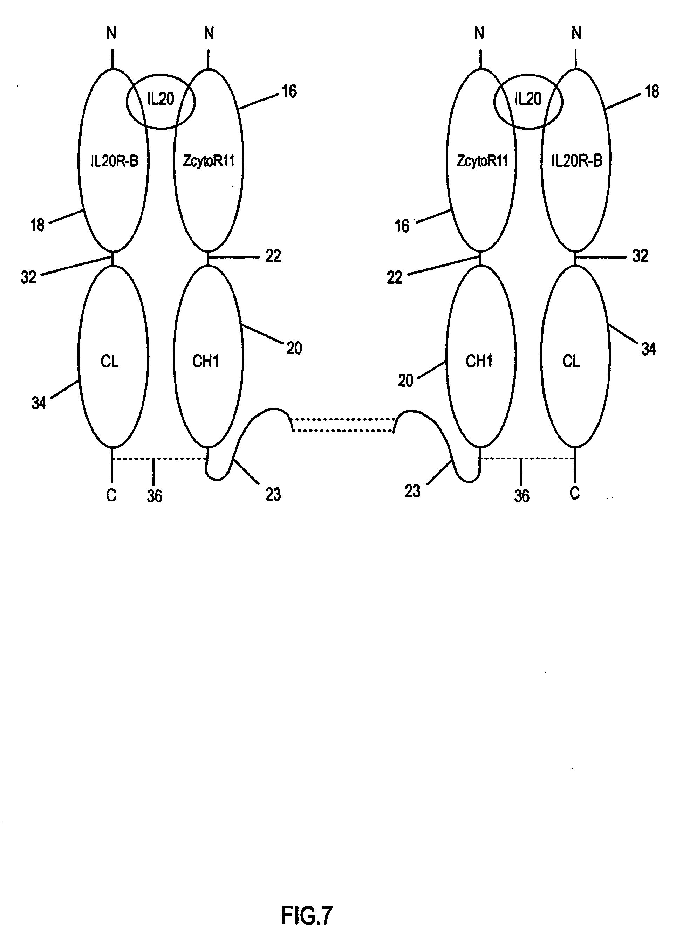

Figure 7 shows a soluble IL-20 receptor construct that is identical to the construct of Figure 1 except both the CH2, and CH3 domains are absent. The CH2 and CH3 domains run from amino acid residue 357, an alanine, to the end of the polypeptide sequence of SEQ ID NO: 25.

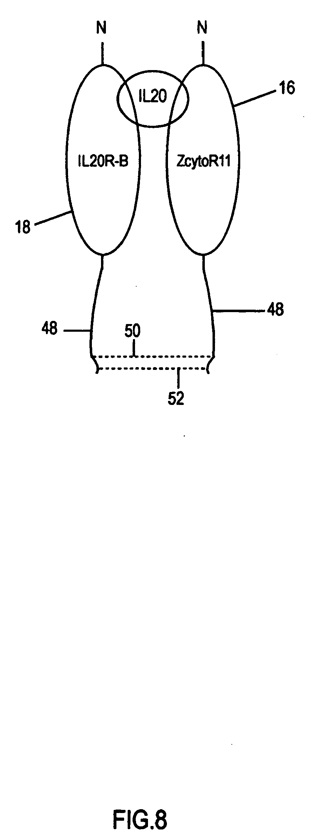

Figure 8 shows a construct wherein both IL-22R, 16, and IL-20RB have a polypeptide linker, 48, fused to their respective carboxyl termini. Each polypeptide linker has two cysteine residues such that when they are expressed the cysteines form two disulfide bonds, 50 and 52. In this case the polypeptide linker is comprised of the hinge region, 23 in Figure 1. The hinge region is comprised of amino acid residues 342, a glutamic acid, to and including amino acid residue 356 of SEQ ID NO: 25.

In another aspect of the invention, a method is provided for producing a soluble receptor comprised of extracellular domains of IL-22R and IL-20RB (as defined in the claims), comprising (a) introducing into a host cell a first DNA sequence comprised of a transcriptional promoter operatively linked to a first secretory signal sequence followed downstream by and in proper reading frame the DNA that encodes the extracellular portion of IL-22R and the DNA that encodes an immunoglobulin light chain constant region;(b) introducing into the host cell a second DNA construct comprised of a transcriptional promoter operatively linked to a second secretory signal followed downstream by and in proper reading frame a DNA sequence that encodes the extracellular portion of IL-20RB and a DNA sequence that encodes an immunoglobulin heavy chain constant region domain selected from the group consisting of C_{H}1, C_{H}2, C_{H}3 and C_{H}4; (c) growing the host cell in an appropriate growth medium under physiological conditions to allow the secretion of a fusion protein comprised of the extracellular domain of IL-22R and IL-20RB; and (d) isolating the polypeptide from the host cell. In one embodiment, the second DNA sequence further encodes an immunoglobulin heavy chain hinge region wherein the hinge region is joined to the heavy chain constant region domain. In another embodiment, the second DNA sequence further encodes an immunoglobulin variable region joined upstream of and in proper reading frame with the immunoglobulin heavy chain constant region.

In an alternative embodiment, a method is provided for producing a soluble receptor comprised of the extracellular domains of IL-22R and IL-20RB (as defined in the claims), comprising (a) introducing into a host cell a first DNA sequence comprised of a transcriptional promoter operatively linked to a first secretory signal sequence followed downstream by and in proper reading frame the DNA that encodes the extracellular portion of IL-20RB and the DNA that encodes an immunoglobulin light chain constant region;(b) introducing into the host cell a second DNA construct comprised of a transcriptional promoter operatively linked to a second secretory signal followed downstream by and in proper reading frame a DNA sequence that encodes the extracellular portion of IL-22R and a DNA sequence that encodes an immunoglobulin heavy chain constant region domain selected from the group consisting of C_{H}1, C_{H}2, C_{H}3 and C_{H}4; (c) growing the host cell in an appropriate growth medium under physiological conditions to allow the production of a dimerized heterodimeric fusion protein comprised of the extracellular domain of IL-22R and IL-20RB; and (d) isolating the dimerized polypeptide from the host cell. In one embodiment, the second DNA sequence further encodes an immunoglobulin heavy chain hinge region wherein the hinge region is joined to the heavy chain constant region domain. In another embodiment, the second DNA sequence further encodes an immunoglobulin variable region joined upstream of and in proper reading frame with the immunoglobulin heavy chain constant region. (See U.S. Patent No. 5,843,725.)

In another embodiment, a method is provided for producing a soluble receptor comprised of the extracellular domains of IL-22R and IL-20RB (as defined in the claims), comprising (a) introducing into a host cell a DNA construct containing a DNA construct that encodes the extracellular portion of IL-20RB and a DNA construct of the extracellular portion of IL-22R, (b) growing the host cell in an appropriate medium under physiological conditions to allow the production of the extracellular domain of IL-22R and the extracellular domain of IL-20RB; and (d) isolating the polypeptides from the host cell.

Other aspects of the present invention include host cells transformed or transfected with a DNA construct that encodes the extracellular domain of IL-22RB and a DNA construct that encodes the extracellular domain of IL-20RB, as defined in the claims. Both constructs can be on one vector or on separate vectors.

A polynucleotide, generally a cDNA sequence, encodes the described polypeptides herein. A cDNA sequence that encodes a polypeptide of the present invention is comprised of a series of codons, each amino acid residue of the polypeptide being encoded by a codon and each codon being comprised of three nucleotides. The amino acid residues are encoded by their respective codons as follows.
Alanine (Ala) is encoded by GCA, GCC, GCG or GCT.
Cysteine (Cys) is encoded by TGC or TGT.
Aspartic acid (Asp) is encoded by GAC or GAT.
Glutamic acid (Glu) is encoded by GAA or GAG.
Phenylalanine (Phe) is encoded by TTC or TTT.
Glycine (Gly) is encoded by GGA, GGC, GGG or GGT.
Histidine (His) is encoded by CAC or CAT.
Isoleucine (Ile) is encoded by ATA, ATC or ATT.
Lysine (Lys) is encoded by AAA, or AAG.
Leucine (Leu) is encoded by TTA, TTG, CTA, CTC, CTG or CTT.
Methionine (Met) is encoded by ATG.
Asparagine (Asn) is encoded by AAC or AAT.
Proline (Pro) is encoded by CCA, CCC, CCG or CCT.
Glutamine (Gln) is encoded by CAA or CAG.
Arginine (Arg) is encoded by AGA, AGG, CGA, CGC, CGG or CGT.
Serine (Ser) is encoded by AGC, AGT, TCA, TCC, TCG or TCT.
Threonine (Thr) is encoded by ACA, ACC, ACG or ACT.
Valine (Val) is encoded by GTA, GTC, GTG or GTT.
Tryptophan (Trp) is encoded by TGG.
Tyrosine (Tyr) is encoded by TAC or TAT.

It is to be recognized that according to the present invention, when a polynucleotide is claimed as described herein, it is understood that what is claimed are both the sense strand, the anti-sense strand, and the DNA as double-stranded having both the sense and anti-sense strand annealed together by their respective hydrogen bonds. Also claimed is the messenger RNA (mRNA) that encodes the polypeptides of the president invention, and which mRNA is encoded by the cDNA described herein. Messenger RNA (mRNA) will encode a polypeptide using the same codons as those defined herein, with the exception that each thymine nucleotide (T) is replaced by a uracil nucleotide (U).

One of ordinary skill in the art will also appreciate that different species can exhibit "preferential codon usage." In general, see, Grantham, et al., Nuc. Acids Res. 8:1893-1912 (1980); Haas, et al. Curr. Biol. 6:315-324 (1996); Wain-Hobson, et al., Gene 13:355-364 (1981); Grosjean and Fiers, Gene 18:199-209 (1982); Holm, Nuc. Acids Res. 14:3075-3087 (1986); Ikemura, J. Mol. Biol. 158:573-597 (1982). As used herein, the term "preferential codon usage" or "preferential codons" is a term of art referring to protein translation codons that are most frequently used in cells of a certain species, thus favoring one or a few representatives of the possible codons encoding each amino acid. For example, the amino acid Threonine (Thr) may be encoded by ACA, ACC, ACG, or ACT, but in mammalian cells ACC is the most commonly used codon; in other species, for example, insect cells, yeast, viruses or bacteria, different Thr codons may be preferential. Preferential codons for a particular species can be introduced into the polynucleotides of the present invention by a variety of methods known in the art. Introduction of preferential codon sequences into recombinant DNA can, for example, enhance production of the protein by making protein translation more efficient within a particular cell type or species. Sequences containing preferential codons can be tested and optimized for expression in various species, and tested for functionality as disclosed herein.

Methods for synthesizing amino acids and aminoacylating tRNA are known in the art. Transcription and translation of plasmids containing nonsense mutations is carried out in a cell-free system comprising an E. *coli* S30 extract and commercially available enzymes and other reagents. Proteins are purified by chromatography. See, for example, Robertson et al., J. Am. Chem. Soc. 113:2722 (1991); Ellman et al., Methods Enzymol. 202:301 (1991; Chung et al., Science 259:806-809 (1993); and Chung et al., Proc. Natl. Acad. Sci. USA 90:10145-1019 (1993). In a second method, translation is carried out in *Xenopus* oocytes by microinjection of mutated mRNA and chemically aminoacylated suppressor tRNAs, Turcatti et al., J. Biol. Chem. 271:19991-19998 (1996). Within a third method, E. *coli* cells are cultured in the absence of a natural amino acid that is to be replaced (*e*.*g*., phenylalanine) and in the presence of the desired non-naturally occurring amino acid(s) (*e*.*g*., 2-azaphenylalanine, 3-azaphenylalanine, 4-azaphenylalanine, or 4-fluorophenylalanine). The non-naturally occurring amino acid is incorporated into the protein in place of its natural counterpart. See, Koide et al., Biochem. 33:7470-7476 (1994). Naturally occurring amino acid residues can be converted to non-naturally occurring species by *in vitro* chemical modification. Chemical modification can be combined with site-directed mutagenesis to further expand the range of substitutions, Wynn and Richards, Protein Sci. 2:395-403 (1993).

A limited number of non-conservative amino acids, amino acids that are not encoded by the genetic code, non-naturally occurring amino acids, and unnatural amino acids may be substituted for amino acid residues.

Essential amino acids in the polypeptides of the present invention can be identified according to procedures known in the art, such as site-directed mutagenesis or alanine-scanning mutagenesis, Cunningham and Wells, Science 244: 1081-1085 (1989); Bass et al., Proc. Natl. Acad. Sci. USA 88:4498-502 (1991). In the latter technique, single alanine mutations are introduced at every residue in the molecule, and the resultant mutant molecules are tested for biological activity as disclosed below to identify amino acid residues that are critical to the activity of the molecule. See also, Hilton et al., J. Biol. Chem. 271:4699-708, 1996. Sites of ligand-receptor interaction can also be determined by physical analysis of structure, as determined by such techniques as nuclear magnetic resonance, crystallography, electron diffraction or photoaffinity labeling, in conjunction with mutation of putative contact site amino acids. See, for example, de Vos et al., Science 255:306-312 (1992); Smith et al., J. Mol. Biol. 224:899-904 (1992); Wlodaver et al., FEBS Lett. 309:59-64 (1992).

Multiple amino acid substitutions can be made and tested using known methods of mutagenesis and screening, such as those disclosed by Reidhaar-Olson and Sauer, Science 241:53-57 (1988) or Bowie and Sauer, Proc. Natl. Acad. Sci. USA 86:2152-2156 (1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display, *e.g.,* Lowman et al., Biochem. 30:10832-10837 (1991); Ladner et al., U.S. Patent No. 5,223,409; Huse, WIPO Publication WO 92/06204) and region-directed mutagenesis, Derbyshire et al., Gene 46:145 (1986); Ner et al., DNA 7:127 (1988).

Variants of the disclosed *IL-20*, IL-22R and IL-20RB DNA and polypeptide sequences can be generated through DNA shuffling as disclosed by Stemmer, Nature 370:389-391, (1994), Stemmer, Proc. Natl. Acad. Sci. USA 91:10747-10751 (1994) and WIPO Publication WO 97/20078. Briefly, variant DNAs are generated by *in vitro* homologous recombination by random fragmentation of a parent DNA followed by reassembly using PCR, resulting in randomly introduced point mutations. This technique can be modified by using a family of parent DNAs, such as allelic variants or DNAs from different species, to introduce additional variability into the process. Selection or screening for the desired activity, followed by additional iterations of mutagenesis and assay provides for rapid "evolution" of sequences by selecting for desirable mutations while simultaneously selecting against detrimental changes.

Mutagenesis methods as disclosed herein can be combined with high-throughput, automated screening methods to detect activity of cloned, mutagenized polypeptides in host cells. Mutagenized DNA molecules that encode active polypeptides can be recovered from the host cells and rapidly sequenced using modem equipment. These methods allow the rapid determination of the importance of individual amino acid residues in a polypeptide of interest, and can be applied to polypeptides of unknown structure.

### PROTEIN PRODUCTION

Polypeptides can be produced in genetically engineered host cells according to conventional techniques. Suitable host cells are those cell types that can be transformed or transfected with exogenous DNA and grown in culture, and include bacteria, fungal cells, and cultured higher eukaryotic cells. Eukaryotic cells, particularly cultured cells of multicellular organisms, are preferred. Techniques for manipulating cloned DNA molecules and introducing exogenous DNA into a variety of host cells are disclosed by Sambrook et al., Molecular Cloning: A Laboratory Manual, 2nd ed., (Cold Spring Harbor Laboratory Press, Cold Spring Harbor, NY, 1989), and Ausubel et al., eds., Current Protocols in Molecular Biology (John Wiley and Sons, Inc., NY, 1987).

In general, a DNA sequence encoding a polypeptide is operably linked to other genetic elements required for its expression, generally including a transcription promoter and terminator, within an expression vector. The vector will also commonly contain one or more selectable markers and one or more origins of replication, although those skilled in the art will recognize that within certain systems selectable markers may be provided on separate vectors, and replication of the exogenous DNA may be provided by integration into the host cell genome. Selection of promoters, terminators, selectable markers, vectors and other elements is a matter of routine design within the level of ordinary skill in the art. Many such elements are described in the literature and are available through commercial suppliers.

To direct a polypeptide into the secretory pathway of a host cell, a secretory signal sequence (also known as a leader sequence, prepro sequence or pre sequence) is provided in the expression vector. The secretory signal sequence may be that of the native polypeptides, or may be derived from another secreted protein (*e*.*g*., t-PA) or synthesized *de novo.* The secretory signal sequence is operably linked to the DNA sequence, i.e., the two sequences are joined in the correct reading frame and positioned to direct the newly synthesized polypeptide into the secretory pathway of the host cell. Secretory signal sequences are commonly positioned 5' to the DNA sequence encoding the polypeptide of interest, although certain secretory signal sequences may be positioned elsewhere in the DNA sequence of interest (see, *e.g.,* Welch et al., U.S. Patent No. 5,037,743; Holland et al., U.S. Patent No. 5,143,830).

Alternatively, the secretory signal sequence contained in the polypeptides of the present invention is used to direct other polypeptides into the secretory pathway. The present invention provides for such fusion polypeptides. The secretory signal sequence contained in the fusion polypeptides of the present invention is preferably fused amino-terminally to an additional peptide to direct the additional peptide into the secretory pathway. Such constructs have numerous applications known in the art. For example, these novel secretory signal sequence fusion constructs can direct the secretion of an active component of a normally non-secreted protein, such as a receptor. Such fusions may be used *in vivo* or *in vitro* to direct peptides through the secretory pathway.

Cultured mammalian cells are suitable hosts within the present invention. Methods for introducing exogenous DNA into mammalian host cells include calcium phosphate-mediated transfection, Wigler et al., Cell 14:725 (1978), Corsaro and Pearson, Somatic Cell Genetics 7:603 (1981); Graham and Van der Eb, Virology 52:456 (1973), electroporation, Neumann et al., EMBO J. 1:841-845 (1982), DEAE-dextran mediated transfection (Ausubel *et al., ibid.,* and liposome-mediated transfection, Hawley-Nelson et al., Focus 15:73 (1993); Ciccarone et al., Focus 15:80 (1993), and viral vectors, Miller and Rosman, BioTechniques 7:980(1989); Wang and Finer, Nature Med. 2:714 (1996). The production of recombinant polypeptides in cultured mammalian cells is disclosed, for example, by Levinson et al., U.S. Patent No. 4,713,339; Hagen et al., U.S. Patent No. 4,784,950; Palmiter et al., U.S. Patent No. 4,579,821; and Ringold, U.S. Patent No. 4,656,134. Suitable cultured mammalian cells include the COS-1 (ATCC No. CRL 1650), COS-7 (ATCC No. CRL 1651), BHK (ATCC No. CRL 1632), BHK 570 (ATCC No. CRL 10314),293 (ATCC No. CRL 1573; Graham et al., J. Gen. Virol. 36:59 (1977) and Chinese hamster ovary (*e.g.* CHO-K1; ATCC No. CCL 61) cell lines. Additional suitable cell lines are known in the art and available from public depositories such as the American Type Culture Collection, Rockville, Maryland. In general, strong transcription promoters are preferred, such as promoters from SV-40 or cytomegalovirus. See, *e.g.,* U.S. Patent No. 4,956,288. Other suitable promoters include those from metallothionein genes (U.S. Patent Nos. 4,579,821 and 4,601,978) and the adenovirus major late promoter.

Drug selection is generally used to select for cultured mammalian cells into which foreign DNA has been inserted. Such cells are commonly referred to as "transfectants". Cells that have been cultured in the presence of the selective agent and are able to pass the gene of interest to their progeny are referred to as "stable transfectants." A preferred selectable marker is a gene encoding resistance to the antibiotic neomycin. Selection is carried out in the presence of a neomycin-type drug, such as G-418 or the like. Selection systems can also be used to increase the expression level of the gene of interest, a process referred to as "amplification." Amplification is carried out by culturing transfectants in the presence of a low level of the selective agent and then increasing the amount of selective agent to select for cells that produce high levels of the products of the introduced genes. A preferred amplifiable selectable marker is dihydrofolate reductase, which confers resistance to methotrexate. Other drug resistance genes (*e*.*g*. hygromycin resistance, multi-drug resistance, puromycin acetyltransferase) can also be used. Alternative markers that introduce an altered phenotype, such as green fluorescent protein, or cell surface proteins such as CD4, CD8, Class I MHC, placental alkaline phosphatase may be used to sort transfected cells from untransfected cells by such means as FACS sorting or magnetic bead separation technology.

Other higher eukaryotic cells can also be used as hosts, including plant cells, insect cells and avian cells. The use of *Agrobacterium rhizogenes* as a vector for expressing genes in plant cells has been reviewed by Sinkar et al., J. Biosci. (Bangalore) 11:47 (1987). Transformation of insect cells and production of foreign polypeptides therein is disclosed by Guarino et al., U.S. Patent No. 5,162,222 and WIPO publication WO 94/06463. Insect cells can be infected with recombinant baculovirus, commonly derived from Autographa californica nuclear polyhedrosis virus (AcNPV). DNA encoding a polypeptide is inserted into the baculoviral genome in place of the AcNPV polyhedrin gene coding sequence by one of two methods. The first is the traditional method of homologous DNA recombination between wild-type AcNPV and a transfer vector containing the gene flanked by AcNPV sequences. Suitable insect cells, *e.g.* SF9 cells, are infected with wild-type AcNPV and transfected with a transfer vector comprising a polynucleotide operably linked to an AcNPV polyhedrin gene promoter, terminator, and flanking sequences. See, King, L.A. and Possee, R.D., The Baculovirus Expression System: A Laboratory Guide, (Chapman & Hall, London); O'Reilly, D.R. et al., Baculovirus Expression Vectors: A Laboratory Manual (Oxford University Press, New York, New York, 1994); and, Richardson, C. D., Ed., Baculovirus Expression Protocols. Methods in Molecular Biology, (Humana Press, Totowa, NJ 1995). Natural recombination within an insect cell will result in a recombinant baculovirus that contains coding sequences driven by the polyhedrin promoter. Recombinant viral stocks are made by methods commonly used in the art.

The second method of making recombinant baculovirus utilizes a transposon-based system described by Luckow, V.A, et al., J Virol 67:4566 (1993). This system is sold in the Bac-to-Bac kit (Life Technologies, Rockville, MD). This system utilizes a transfer vector, pFastBacl™ (Life Technologies) containing a Tn7 transposon to move the DNA encoding the polypeptide into a baculovirus genome maintained in E. *coli* as a large plasmid called a "bacmid." The pFastBacl™ transfer vector utilizes the AcNPV polyhedrin promoter to drive the expression of the gene of interest. However, pFastBacl™ can be modified to a considerable degree. The polyhedrin promoter can be removed and substituted with the baculovirus basic protein promoter (also known as P*cor,* p6.9 or MP promoter), which is expressed earlier in the baculovirus infection, and has been shown to be advantageous for expressing secreted proteins. See, Hill-Perkins, M.S. and Possee, R.D., J Gen Virol 71:971 (1990); Bonning, B.C. et al., J Gen Virol 75:1551 (1994); and, Chazenbalk, G.D., and Rapoport, B., J Biol Chem 270:1543 (1995). In such transfer vector constructs, a short or long version of the basic protein promoter can be used. Moreover, transfer vectors can be constructed that replace the native secretory signal sequences with secretory signal sequences derived from insect proteins. For example, a secretory signal sequence from Ecdysteroid Glucosyltransferase (EGT), honey bee Melittin (Invitrogen, Carlsbad, CA), or baculovirus gp67 (PharMingen, San Diego, CA) can be used in constructs to replace the native secretory signal sequence. In addition, transfer vectors can include an in-frame fusion with DNA encoding an epitope tag at the C- or N-terminus of the expressed polypeptide, for example, a Glu-Glu epitope tag, Grussenmeyer, T. et al., Proc Natl Acad Sci. 82:7952 (1985). Using a technique known in the art, a transfer vector containing a recombinant gene is transformed into E. *coli,* and screened for bacmids that contain an interrupted lacZ gene indicative of recombinant baculovirus. The bacmid DNA containing the recombinant baculovirus genome is isolated, using common techniques, and used to transfect *Spodoptera frugiperda* cells, *e*.*g*. Sf9 cells. Recombinant virus that expresses the polypeptide is subsequently produced. Recombinant viral stocks are made by methods commonly used the art.

The recombinant virus is used to infect host cells, typically a cell line derived from the fall armyworm, *Spodoptera frugiperda.* See, in general, Glick and Pasternak, Molecular Biotechnology: Principles and Applications of Recombinant DNA (ASM Press, Washington, D.C., 1994). Another suitable cell line is the High FiveO™ cell line (Invitrogen) derived from *Trichoplusia ni* (U.S. Patent #5,300,435). Commercially available serum-free media are used to grow and maintain the cells. Suitable media are Sf900 II™ (Life Technologies) or ESF 921™ (Expression Systems) for the Sf9 cells; and Ex-ce110405™ (JRH Biosciences, Lenexa, KS) or Express FiveO™ (Life Technologies) for the *T. ni* cells. The cells are grown up from an inoculation density of approximately 2-5 x 10⁵ cells to a density of 1-2 x 10⁶ cells at which time a recombinant viral stock is added at a multiplicity of infection (MOI) of 0.1 to 10, more typically near 3. The recombinant virus-infected cells typically produce the recombinant polypeptide at 12-72 hours post-infection and secrete it with varying efficiency into the medium. The culture is usually harvested 48 hours post-infection. Centrifugation is used to separate the cells from the medium (supernatant). The supernatant containing the polypeptide is filtered through micropore filters, usually 0.45 µm pore size. Procedures used are generally described in available laboratory manuals (King, L. A. and Possee, R.D., *ibid.,* O'Reilly, D.R. *et al., ibid.;* Richardson, C. D., *ibid*.). Subsequent purification of the polypeptide from the supernatant can be achieved using methods described herein.

Fungal cells, including yeast cells, can also be used within the present invention. Yeast species of particular interest in this regard include *Saccharomyces cerevisiae, Pichia pastoris*, and *Pichia methanolica.* Methods for transforming S. *cerevisiae* cells with exogenous DNA and producing recombinant polypeptides therefrom are disclosed by, for example, Kawasaki, U.S. Patent No. 4,599,311; Kawasaki et al., U.S. Patent No. 4,931,373; Brake, U.S. Patent No. 4,870,008; Welch et al., U.S. Patent No. 5,037,743; and Murray et al., U.S. Patent No. 4,845,075. Transformed cells are selected by phenotype determined by the selectable marker, commonly drug resistance or the ability to grow in the absence of a particular nutrient (*e*.*g*., leucine). A preferred vector system for use in *Saccharomyces cerevisiae* is the *POT1* vector system disclosed by Kawasaki et al. (U.S. Patent No. 4,931,373), which allows transformed cells to be selected by growth in glucose-containing media. Suitable promoters and terminators for use in yeast include those from glycolytic enzyme genes (see, *e*.*g*., Kawasaki, U.S. Patent No. 4,599,311; Kingsman et al., U.S. Patent No. 4,615,974; and Bitter, U.S. Patent No. 4,977,092) and alcohol dehydrogenase genes. See also U.S. Patents Nos. 4,990,446; 5,063,154; 5,139,936 and 4,661,454. Transformation systems for other yeasts, including *Hansenula polymorpha, Schizosaccharomyces pombe, Kluyveromyces lactis, Kluyveromyces fragilis, Ustilago maydis, Pichia pastoris, Pichia methanolica, Pichia guillermondii* and *Candida maltosa* are known in the art. See, for example, Gleeson *et al., J. Gen. Microbiol.* 132:3459 (1986) and Cregg, U.S. Patent No. 4,882,279. *Aspergillus* cells may be utilized according to the methods of McKnight et al., U.S. Patent No. 4,935,349. Methods for transforming *Acremonium chrysogenum* are disclosed by Sumino et al., U.S. Patent No. 5,162,228. Methods for transforming *Neurospora* are disclosed by Lambowitz, U.S. Patent No. 4,486,533.

The use of *Pichia methanolica* as host for the production of recombinant proteins is disclosed in WIPO Publications WO 97/17450, WO 97/17451, WO 98/02536, and WO 98/02565. DNA molecules for use in transforming *P. methanolica* will commonly be prepared as double-stranded, circular plasmids, which are preferably linearized prior to transformation. For polypeptide production in *P. methanolica,* it is preferred that the promoter and terminator in the plasmid be that of a *P. methanolica* gene, such as a *P. methanolica* alcohol utilization gene (*AUG1* or *AUG2*). Other useful promoters include those of the dihydroxyacetone synthase (DHAS), formate dehydrogenase (FMD), and catalase (CAT) genes. To facilitate integration of the DNA into the host chromosome, it is preferred to have the entire expression segment of the plasmid flanked at both ends by host DNA sequences. A preferred selectable marker for use in *Pichia methanolica* is a *P. methanolica ADE2* gene, which encodes phosphoribosyl-5-aminoimidazole carboxylase (AIRC; EC 4.1.1.21), which allows *ade2* host cells to grow in the absence of adenine. For large-scale, industrial processes where it is desirable to minimize the use of methanol, it is preferred to use host cells in which both methanol utilization genes (*AUG1* and *AUG2*) are deleted. For production of secreted proteins, host cells deficient in vacuolar protease genes (*PEP4* and *PRB1*) are preferred. Electroporation is used to facilitate the introduction of a plasmid containing DNA encoding a polypeptide of interest into *P. methanolica* cells. It is preferred to transform *P. methanolica* cells by electroporation using an exponentially decaying, pulsed electric field having a field strength of from 2.5 to 4.5 kV/cm, preferably about 3.75 kV/cm, and a time constant (t) of from 1 to 40 milliseconds, most preferably about 20 milliseconds.

Prokaryotic host cells, including strains of the bacteria *Escherichia coli, Bacillus* and other genera are also useful host cells within the present invention. Techniques for transforming these hosts and expressing foreign DNA sequences cloned therein are well known in the art; see, *e.g.,* Sambrook *et al., ibid*.). When expressing a polypeptide in bacteria such as *E. coli*, the polypeptide may be retained in the cytoplasm, typically as insoluble granules, or may be directed to the periplasmic space by a bacterial secretion sequence. In the former case, the cells are lysed, and the granules are recovered and denatured using, for example, guanidine isothiocyanate or urea. The denatured polypeptide can then be refolded and dimerized by diluting the denaturant, such as by dialysis against a solution of urea and a combination of reduced and oxidized glutathione, followed by dialysis against a buffered saline solution. In the latter case, the polypeptide can be recovered from the periplasmic space in a soluble and functional form by disrupting the cells (by, for example, sonication or osmotic shock) to release the contents of the periplasmic space and recovering the protein, thereby obviating the need for denaturation and refolding.

Transformed or transfected host cells are cultured according to conventional procedures in a culture medium containing nutrients and other components required for the growth of the chosen host cells. A variety of suitable media, including defined media and complex media, are known in the art and generally include a carbon source, a nitrogen source, essential amino acids, vitamins and minerals. Media may also contain such components as growth factors or serum, as required. The growth medium will generally select for cells containing the exogenously added DNA by, for example, drug selection or deficiency in an essential nutrient, which is complemented by the selectable marker carried on the expression vector or co-transfected into the host cell. *P. methanolica* cells are cultured in a medium comprising adequate sources of carbon, nitrogen and trace nutrients at a temperature of about 25°C to 35°C. Liquid cultures are provided with sufficient aeration by conventional means, such as shaking of small flasks or sparging of fermentors. A preferred culture medium for *P. methanolica* is YEPD (2% D-glucose, 2% Bacto^{™} Peptone (Difco Laboratories, Detroit, MI), 1% Bacto^{™} yeast extract (Difco Laboratories), 0.004% adenine and 0.006% L-leucine).

### Protein Isolation

It is preferred to purify the polypeptides of the present invention to ≥80% purity, more preferably to ≥90% purity, even more preferably ≥95% purity, and particularly preferred is a pharmaceutically pure state, that is greater than 99.9% pure with respect to contaminating macromolecules, particularly other proteins and nucleic acids, and free of infectious and pyrogenic agents. Preferably, a purified polypeptide is substantially free of other polypeptides, particularly other polypeptides of animal origin.

Expressed recombinant polypeptides (or chimeric polypeptides) can be purified using fractionation and/or conventional purification methods and media. Ammonium sulfate precipitation and acid or chaotrope extraction may be used for fractionation of samples. Exemplary purification steps may include hydroxyapatite, size exclusion, FPLC and reverse-phase high performance liquid chromatography. Suitable chromatographic media include derivatized dextrans, agarose, cellulose, polyacrylamide, specialty silicas, and the like. PEI, DEAE, QAE and Q derivatives are preferred. Exemplary chromatographic media include those media derivatized with phenyl, butyl, or octyl groups, such as Phenyl-Sepharose FF (Pharmacia), Toyopearl butyl 650 (Toso Haas, Montgomeryville, PA), Octyl-Sepharose (Pharmacia) and the like; or polyacrylic resins, such as Amberchrom CG 71 (Toso Haas) and the like. Suitable solid supports include glass beads, silica-based resins, cellulosic resins, agarose beads, cross-linked agarose beads, polystyrene beads, cross-linked polyacrylamide resins and the like that are insoluble under the conditions in which they are to be used. These supports may be modified with reactive groups that allow attachment of proteins by amino groups, carboxyl groups, sulfhydryl groups, hydroxyl groups and/or carbohydrate moieties. Examples of coupling chemistries include cyanogen bromide activation, N-hydroxysuccinimide activation, epoxide activation, sulfhydryl activation, hydrazide activation, and carboxyl and amino derivatives for carbodiimide coupling chemistries. These and other solid media are well known and widely used in the art, and are available from commercial suppliers. Methods for binding receptor polypeptides to support media are well known in the art. Selection of a particular method is a matter of routine design and is determined in part by the properties of the chosen support. See, for example, Affinity Chromatography: Principles & Methods (Pharmacia LKB Biotechnology, Uppsala, Sweden, 1988).

Polypeptides can be isolated by exploitation of their properties. For example, immobilized metal ion adsorption (IMAC) chromatography can be used to purify histidine-rich proteins, including those comprising polyhistidine tags. Briefly, a gel is first charged with divalent metal ions to form a chelate, Sulkowski, Trends in Biochem. 3:1 (1985). Histidine-rich proteins will be adsorbed to this matrix with differing affinities, depending upon the metal ion used, and will be eluted by competitive elution, lowering the pH, or use of strong chelating agents. Other methods of purification include purification of glycosylated proteins by lectin affinity chromatography and ion exchange chromatography. A protein fused to the Fc portion of an immunoglobulin can be purified using a 'Protein A column'. Methods in Enzymol., Vol. 182, "Guide to Protein Purification", M. Deutscher, (ed.),page 529-539 (Acad. Press, San Diego, 1990). Within additional embodiments of the invention, a fusion of the polypeptide of interest and an affinity tag (*e*.*g*., maltose-binding protein, an immunoglobulin domain) may be constructed to facilitate purification.

As used herein, the term "antibodies" includes polyclonal antibodies, affinity-purified polyclonal antibodies, monoclonal antibodies, and antigen-binding fragments, such as F(ab')₂ and Fab proteolytic fragments. Genetically engineered intact antibodies or fragments, such as chimeric antibodies, Fv fragments, single chain antibodies and the like, as well as synthetic antigen-binding peptides and polypeptides, are also included. Non-human antibodies may be humanized by grafting non-human CDRs onto human framework and constant regions, or by incorporating the entire non-human variable domains (optionally "cloaking" them with a human-like surface by replacement of exposed residues, wherein the result is a "veneered" antibody). In some instances, humanized antibodies may retain non-human residues within the human variable region framework domains to enhance proper binding characteristics. Through humanizing antibodies, biological half-life may be increased, and the potential for adverse immune reactions upon administration to humans is reduced.

A variety of assays known to those skilled in the art can be utilized to detect antibodies that bind to protein or peptide. Exemplary assays are described in detail in Antibodies: A Laboratory Manual, Harlow and Lane (Eds.) (Cold Spring Harbor Laboratory Press, 1988). Representative examples of such assays include: concurrent immunoelectrophoresis, radioimmunoassay, radioimmuno-precipitation, enzyme-linked immunosorbent assay (ELISA), dot blot or Western blot assay, inhibition or competition assay, and sandwich assay.

The soluble receptors of the present invention can be used to down-regulate IL-20, which has been shown to be involved in a number of inflammatory processes. Specifically, IL-20 has been shown to up-regulate IL-8. Inflammatory diseases in which IL-8 plays a significant role, and for which a decrease in IL-8 would be beneficial are, adult respiratory disease (ARD), septic shock, multiple organ failure, inflammatory lung injury such as asthma or bronchitis, bacterial pneumonia, psoriasis, eczema, atopic and contact dermatitis, and inflammatory bowel disease such as ulcerative colitis and Crohn's disease. Thus, the soluble receptor to IL-20 of the present invention as defined in the claims, can be for treating these diseases.

### Biology of IL-20, Its receptor and Its Role in Psoriasis

Two orphan class II cytokine receptors, both of which are expressed in skin, were identified as IL-20 receptor subunits. Both IL-20 receptor subunits are required for ligand binding, distinguishing their role from that of subunits in the four other known class II cytokine receptors. IL-22R and IL-20RB are also coexpressed in a number of human tissues besides skin, including ovary, adrenal gland, testis, salivary gland, muscle, lung, kidney, heart and to a lesser degree the small intestine suggesting additional target tissues for IL-20 action. We conclude that the IL-20 heterodimeric receptor is structurally similar to other class n cytokine receptors and is expressed in skin where we have demonstrated activity of the IL-20 ligand.

Two lines of evidence indicate that a role IL-20 and its receptor are involved in psoriasis. This multigenic skin disease is characterized by increased keratinocyte proliferation, altered keratinocyte differentiation, and infiltration of immune cells into the skin. The first line of evidence for a role of IL-20 in psoriasis is that the observed hyperkeratosis and thickened epidermis in the transgenic mice that resemble human psoriatic abnormalities. Decreased numbers of tonofilaments, thought to be related to defective keratinization, are a striking feature of human psoriasis. Intramitochondrial inclusions have been found in both chemically induced and naturally occurring hyperplastic skin conditions in mice. The cause of the inclusions and their effects on mitochondrial function, if any, are unknown. We conclude that IL-20 transgenic mice exhibit many of the characteristics observed in human psoriasis.

### Use of Antagonist to IL-20 to Treat Psoriasis

As indicated in the discussion above and the examples below, IL-20 is involved in the pathology of psoriasis. Thus, the soluble receptors of the present invention can down-regulate IL-20 and thus treat psoriasis.

Psoriasis is one of the most common dermatologic diseases, affecting up to 1 to 2 percent of the world's population. It is a chronic inflammatory skin disorder characterized by erythematous, sharply demarcated papules and rounded plaques, covered by silvery micaceous scale. The skin lesions of psoriasis are variably pruritic. Traumatized areas often develop lesions of psoriasis. Additionally, other external factors may exacerbate psoriasis including infections, stress, and medications, *e*.*g.* lithium, beta blockers, and anti-malarials.

The most common variety of psoriasis is called plaque type. Patients with plaque-type psoriasis will have stable, slowly growing plaques, which remain basically unchanged for long periods of time. The most common areas for plaque psoriasis to occur are the elbows knees, gluteal cleft, and the scalp. Involvement tends to be symmetrical. Inverse psoriasis affects the intertriginous regions including the axilla, groin, submammary region, and navel, and it also tends to affect the scalp, palms, and soles. The individual lesions are sharply demarcated plaques but may be moist due to their location. Plaque-type psoriasis generally develops slowly and runs an indolent course. It rarely spontaneously remits.

Eruptive psoriasis (guttate psoriasis) is most common in children and young adults. It develops acutely in individuals without psoriasis or in those with chronic plaque psoriasis. Patients present with many small erythematous, scaling papules, frequently after upper respiratory tract infection with beta-hemolytic streptococci. Patients with psoriasis may also develop pustular lesions. These may be localized to the palms and soles or may be generalized and associated with fever, malaise, diarrhea, and arthralgias..

About half of all patients with psoriasis have fingernail involvement, appearing as punctate pitting, nail thickening or subungual hyperkeratosis. About 5 to 10 percent of patients with psoriasis have associated joint complaints, and these are most often found in patients with fingernail involvement. Although some have the coincident occurrence of classic Although some have the coincident occurrence of classic rheumatoid arthritis, many have joint disease that falls into one of five type associated with psoriasis: (1) disease limited to a single or a few small joints (70 percent of cases); (2) a seronegative rheumatoid arthritis-like disease; (3) involvement of the distal interphalangeal joints; (4) severe destructive arthritis with the development of "arthritis mutilans"; and (5) disease limited to the spine.

Psoriasis can be treated with antagonists to IL-20. The preferred antagonists are either a soluble receptor to IL-20 as defined in the claims. The antagonists to IL-20 can be for administration alone or in combination with other established therapies such as lubricants, keratolytics, topical corticosteroids, topical vitamin D derivatives, anthralin, systemic antimetabolites such as methotrexate, psoralen-ultraviolet-light therapy (PUVA), etretinate, isotretinoin, cyclosporine, and the topical vitamin D3 derivative calcipotriol. The soluble receptor can be for administration to individual subcutaneously, intravenously, or transdermally using a cream or transdermal patch that contains the antagonist of IL-20. for administration subcutaneously, the antagonist can be for injection into one or more psoriatic plaques. For administered transdermally, the antagonists can be for administration directly on the plaques using a cream containing the antagonist to IL-20.

### Use of Antagonists to IL-20 to Treat Inflammatory Conditions of the Lung.

A soluble receptor of IL-20 of the present invention can be for administration to a person who has asthma, bronchitis or cystic fibrosis or other inflammatory lung disease to treat the disease. The antagonists can be for administration by any suitable method including intravenous, subcutaneous, bronchial lavage, and the use of inhalant containing an antagonist to IL-20.

### Administration of the IL-20 Soluble Receptor

The quantities of the IL-20 soluble necessary for effective therapy will depend upon many different factors, including means of administration, target site, physiological state of the patient, and other medications administered. Thus, treatment dosages should be titrated to optimize safety and efficacy. Typically, dosages used *in vitro* may provide useful guidance in the amounts useful for *in vivo* administration of these reagents. Animal testing of effective doses for treatment of particular disorders will provide further predictive indication of human dosage. Methods for administration include oral, intravenous, peritoneal, intramuscular, transdermal or administration into the lung or trachea in spray form by means or a nebulizer or atomizer. Pharmaceutically acceptable carriers will include water, saline, buffers to name just a few. Dosage ranges would ordinarily be expected from 1µg to 1000µg per kilogram of body weight per day. A dosage for an average adult of the IL-20 soluble receptor would be about 25 mg given twice weekly as a subcutaneous injection. Injections could be given at the site of psoriatic lesions for the treatment of psoriasis. For subcutaneous or intravenous administration of the antagonist to IL-20, the antibody or soluble receptor can be in phosphate buffered saline. Also in skin diseases such as psoriasis, the antagonist to IL-20 can be administered via an ointment or transdermal patch. The doses by may be higher or lower as can be determined by a medical doctor with ordinary skill in the art. For a complete discussion of drug formulations and dosage ranges *see* Remington's Pharmaceutical Sciences, 18th Ed., (Mack Publishing Co., Easton, Penn., 1996), and Goodman and Gilman's: The Pharmacological Bases of Therapeutics, 9th Ed. (Pergamon Press 1996).

The invention is further illustrated by the following non-limiting examples:

### Example 1

### Up-regulation of IL-8 by IL-20

### Methods:

Normal Human Epidermal neonatal keratinocytes (NHEK) (from Clonetics) at passage 2 were plated and grown to confluency in 12 well tissue culture plates. KGM (Keratinocyte growth media) was purchased from Clonetics. When cells reached confluency, they were washed with KGM media minus growth factors = KBM (keratinocyte basal media). Cells were serum starved in KBM for 72 hours prior to the addition of test compounds. Thrombin at 1 I.U./mL and trypsin at 25nM were used as positive controls. One mL of media/well was added. KBM only was used as the negative control.

IL-20 was made up in KBM media and added at varying concentrations, from 2.5µg/ml down to 618ng/mL in a first experiment and from 2.5µg/mL down to 3ng/mL in a second experiment.

Cells were incubated at 37° C, 5% CO₂ for 48 hours. Supernatants were removed and frozen at -80° C for several days prior to assaying for IL-8 and GM-CSF levels. Human IL-8 Immunoassay kit # D8050 (RandD Systems, Inc.) and human GM-CSF Immunoassay kit # HSGMO (RandD Systems, Inc.) were used to determine cytokine production following manufacturer's instructions.

### Results

The results indicated that the expression of IL-8 and GM-CSF were induced by IL-20.

### Example 2

### Cloning of IL-20RB

### Cloning of IL-20RB coding region

Two PCR primers were designed based on the sequence from International Patent Application No. PCT/US99/03735 (publication no. WO 99/46379) filed on March 8, 1999. SEQ ID NO: 38 contains the ATG (Met1) codon with an EcoRI restriction site, SEQ ID NO: 37 contains the stop codon (TAG) with an XhoI restriction site. The PCR amplification was carried out using a human keratinocyte (HaCaT) cDNA library DNA as a template and SEQ ID NO: 37 and SEQ ID NO: 38 as primers. The PCR reaction was performed as follows: incubation at 94°C for 1 min followed by 30 cycles of 94°C for 30 sec and 68°C for 2 min, after additional 68°C for 4 min, the reaction was stored at 4°C. The PCR products were run on 1% Agarose gel, and a 1 kb DNA band was observed. The PCR products were cut from the gel and the DNA was purified using a QIAquick Gel Extraction Kit (Qiagen). The purified DNA was digested with EcoRI and XhoI, and cloned into a pZP vector that was called pZP7N. A pZP plasmid is a mammalian expression vector containing an expression cassette having the mouse metallothionein-1 promoter, human tPA leader peptide, multiple restriction sites for insertion of coding sequences, a Glu-Glu tag, and a human growth hormone terminator. The plasmid also has an E. coli origin of replication, a mammalian selectable marker expression unit having an SV40 promoter, an enhancer and an origin of replication, as well as a DHFR gene, and the SV40 terminator. Several IL-20RB-pZP7N clones were sequenced. They all contain three non-conservative mutations compared with the sequence of IL-20RB in PCT/US99/03735: (sequence IL-20RB-pZP7N), 146 Pro (CCC) -- Thr (ACC), 148 His (CAT) -- Asp (GAT), and 171 Thr (ACG) -- Arg (AGG).

To verify the three substitutions in IL-20RB-pZP7N clone, PCR amplification was carried out using three difference cDNA sources -- fetal skin marathon cDNA, HaCaT cDNA library DNA, and prostate smooth muscle cDNA library DNA -- as templates. The PCR products were gel purified and sequenced. The sequence of each of the three PCR products was consistent with that of the IL-20RB-pZP7N clone. IL-20RB is SEQ ID NO: 22 and 23, and the mature extracellular domain is SEQ ID NO: 21.

### Example 3

### Binding of IL-20 to IL-20RB/ IL-22R Heterodimer

A cell-based binding assay was used to verify IL-20 binds to IL-22R- IL-20RB heterodimer.

Expression vectors containing known and orphan Class II cytokine receptors (including IL-22R and IL-20RB) were transiently transfected into Baf3 cells.

Plated cells out at 5000cells/well, treated cells with IL-20 (zcyto10), MDA-7, and Soluble Proteins.
- Inc. at 37 degrees for 3 days (72hrs.)
- Added 20ul/well of Alamar Blue, inc. at 37 degrees overnight (24 hrs.)
- Read on the f-Max (Molecular Devises) in the Robotics room on 544 excitation/ 590 emission setting.

### Results:

- Positive proliferative response with treatments of IL-20 (zcyto10) and MDA-7 from 0. 1ng/ml to 100ng/ml on Baf3/DIRS1/cytoR11 cell line.
- A neutralization of the positive proliferative response of IL-20 and MDA-7 (same conc.'s) when IL-20 and MDA-7 were treated in combination with the IL-22R Soluble Receptor (heterodimeric Sol. R.) at a 60 fold molar excess.
- A neutralization of the positive proliferative response of MDA-7 (from 0.1 to 10ng/ml) when MDA-7 was treated in combination with IL-20RB Soluble Protein (thrombin cleaved version) at a 60 fold molar excess.

### Example 4

### Up-regulation of Inflammatory Cytokines by IL-20

### Cell Treatment

The human keratinocyte cell line, HaCaT was grown at 37°C to several days post-confluence in T-75 tissue culture flasks. At this point, normal growth media (DMEM + 10% FBS) was removed and replaced with serum-free media. Cells were then incubated for two days at 37°C. DMEM was then removed and four flasks of cells per treatment were treated with one of each of the following conditions for four hours at 37°C: recombinant human (rh) IL-1 alpha at 5 ng/mL, rh IL-1 alpha at 20 ng/mL, rh IL-1 alpha at 5 ng/mL + IL-20 at 1µg/mL, IL-20 at 1µg/mL, or rh IL-10 at 10 ng/mL.

### RNA Isolation

Following cytokine treatment, media was removed and cells were lysed using a guanidium thiocyanate solution. Total RNA was isolated from the cell lysate by an overnight spin on a cesium chloride gradient. The following day, the RNA pellet was resuspended in a TE/SDS solution and ethanol precipitated. RNA was then quantitated using a spectrophotometer, followed by a DNase treatment as per Section V.B. of Clontech's Atlas^{™} cDNA Expression Arrays User Manual (version PT3140-1/PR9X390, published 11/5/99). Quality of RNA samples was verified by purity calculations based on spec readings, and by visualization on agarose gel. Genomic contamination of the RNA samples was ruled out by PCR analysis of the beta-actin gene.

Clontech's protocols for polyA+ enrichment, probe synthesis and hybridization to Atlas^{™} arrays were followed (see above, plus Atlas^{™} Pure Total RNA Labeling System User Manual, PT3231-1/PR96157, published 6/22/99). Briefly, polyA+ RNA was isolated from 50 mg of total RNA using streptavidin coated magnetic beads (by Clontech, Paolo Alto, CA) and a magnetic particle separator. PolyA+ RNA was then labeled with ^{alpha32}P-dATP via RT-PCR. Clontech CDS primers specific to the 268 genes on the Atlas^{™} human cytokine/receptor array (Cat. #7744-1) were used in the reaction. Labeled probe was isolated using column chromatography and counted in scintillation fluid.

### Array membrane Hybridization

Atlas^{™} arrays were pre-hybridized with Clontech ExpressHyb plus 100 mg/mL heat denatured salmon sperm DNA for at least thirty minutes at 68°C with continuous agitation. Membranes were then hybridized with 1.9 x 10⁶ CPM/mL (a total of 1.14 x 10⁷ CPM) overnight at 68°C with continuous agitation. The following day, membranes were washed for thirty minutes x 4 in 2X SSC, 1% SDS at 68° C, plus for thirty minutes x 1 in 0.1X SSC, 0.5% SDS at 68°C, followed by one final room temperature wash for five minutes in 2X SSC. Array membranes were then placed in Kodak plastic pouches sealed and exposed to a phosphor imager screen overnight at room temperature. The next day, phosphor screens were scanned on a phosphor imager and analyzed using Clontech's AtlasImage^{™} 1.0 software.

### Results

### Genes Up-regulated by IL-20

1. Tumor necrosis factor (TNF) was up-regulated 1.9-2.4 fold by IL-20.
2. Placental growth factors 1 & 2 (PLGF) were up-regulated 1.9-2.0 fold by IL-20.
3. Coagulating factor II receptor was up-regulated 2.0-2.5 fold by IL-20.
4. Calcitonin receptor was up-regulated 2.2-2.3 fold by IL-20.
5. TNF-inducible hyaluronate-binding protein TSG-6 was up-regulated 2.1-2.2 fold by IL-20.
6. Vascular endothelial growth factor (VEGF) receptor-1 precursor, tyrosine-protein kinase receptor (FLT-1) (SFLT) was up-regulated 2.1-2.7 fold by IL-20.
7. MRP-8 (calcium binding protein in macrophages MIF- related) was up-regulated 2.9-4.1 fold by IL-20.
8. MRP-14 (calcium binding protein in macrophages MIF-related) was up-regulated 3.0-3.8 fold by IL-20.
9. Relaxin H2 was up-regulated 3.14 fold by IL-20.
10. Transforming growth factor beta (TGFβ) receptor III 300 kDa was up-regulated 2.4-3.6 fold by IL-20.

### Genes Showing Synergy with IL-20 + IL-1 Treatment

1. Bone morphogenic protein 2a was up-regulated 1.8 fold with IL-20 treatment alone, 2.5 fold with IL-1 treatment alone, and 8.2 fold with both IL-20 and IL-1 treatment together.
2. MRP-8 was up-regulated 2.9 fold with IL-20 treatment alone, 10.7 fold with IL-1 treatment alone and 18.0 fold with both IL-20 and IL-1 treatment together.
3. Erythroid differentiation protein (EDF) was up-regulated 1.9 fold with IL-20 treatment alone, 9.7 fold with IL-1 treatment alone and 19.0 fold with both IL-20 and IL-1 treatment together.
4. MRP-14 (calcium binding protein in macrophages, MIF related) was up-regulated 3.0 fold with IL-20 treatment alone, 12.2 fold with IL-1 treatment alone and 20.3 fold with both IL-20 and IL-1 treatment together.
5. Heparin-binding EGF-like growth factor was up-regulated 2.0 fold with IL-20 treatment alone, 14 fold with IL-1 treatment alone and 25.0 fold with both IL-20 and IL-1 treatment together.
6. Beta-thromboglobulin-like protein was up-regulated 1.5 fold with IL-20 treatment alone, 15 fold with IL-1 treatment alone and 27 fold with both IL-20 and IL-1 treatment together.
7. Brain-derived neurotrophic factor (BDNF) was up-regulated 1.7 fold with IL-20 treatment alone, 25 fold with IL-1 treatment alone and 48 fold with both IL-20 and IL-1 treatment together.
8. Monocyte chemotactic and activating factor MCAF was up-regulated 1.3 fold with IL-20 treatment alone, 32 fold with IL-1 treatment alone and 56 fold with both IL-20 and IL-1 treatment together.

### Example 5

### IL-20 Transgenic Phenotype

Both human and mouse IL-20 were overexpressed in transgenic mice using a variety of promoters. The liver-specific mouse albumin promoter, directing expression of human IL-20, was used initially in an attempt to achieve circulating levels of protein. Subsequent studies were conducted using the keratin 14 (K14) promoter, which primarily targets expression to the epidermis and other stratified squamous epithelia; the mouse metallothionein-1 promoter, which gives a broad expression pattern; and the EµLCK promoter, which drives expression in cells of the lymphoid lineage. Similar results were obtained in all four cases, possibly because these promoters all give rise to circulating levels of IL-20.

In all cases, transgenic pups expressing the IL-20 transgene were smaller than non-transgenic littermates, had a shiny appearance with tight, wrinkled skin and died within the first few days after birth. Pups had milk in their stomachs indicating that they were able to suckle. These mice had swollen extremities, tail, nostril and mouth regions and had difficulty moving. In addition, the mice were frail, lacked visible adipose tissue and had delayed ear and toe development. Low expression levels in liver (less than 100 mRNA molecules/cell) were sufficient for both the neonatal lethality and skin abnormalities. Transgenic mice without a visible phenotype either did not express the transgene, did not express it at detectable levels, or were mosaic.

Histologic analysis of the skin of the IL-20 transgenic mice showed a thickened epidermis, hyperkeratosis and a compact stratum corneum compared to non-transgenic littermates. Serocellular crusts (scabs) were observed occasionally. Electron microscopic (EM) analysis of skin from transgenic mice showed intramitochondrial lipoid inclusions, mottled keratohyaline granules, and relatively few tonofilaments similar to that observed in human psoriatic skin and in mouse skin disease models. In addition, many of the transgenic mice had apoptotic thymic lymphocytes. No other abnormalities were detected by histopathological analysis. These histological and EM results support and extend the observed gross skin alterations.

### Example 6

### Experimental Procedures:

### Luciferase Assay

Luciferase reporter assays were performed using BHK cells stably transfected with IL-22R and IL-20RB and utilizing the STAT-driven luciferase reporter cassette. Cells were switched to serum-free medium overnight prior to treatment with serial dilutions of IL-19, IL-20, and MDA-7 in the presence or absence of IL-20RA/IL-20RB soluble receptor. Cells were lysed and read on the Berthold MicroLumat Plus for luciferase reporter activity.

### BaF3 Proliferation Assay

Proliferation assays used Alamar Blue, which was added to the cells 24 h prior to being read on a *f*max plate reader (Molecular Devices, Sunnyvale, CA) using the Softmax Pro program.

### RT-PCR Analysis on Human Tissues

RT-PCR was performed on a human Rapid-Scan gene expression panel (Origene Technologies, Inc.) using primers 5'-ccccagacacggtctacagcat-3' and 5'-gggtcaggccgaagaactcatat-3' to amplify a 440 bp fragment of human *IL22R*. PCR conditions are 94°C for 2 min., followed by 35 cycles of 94°C for 15 sec., 72°C for 90 sec, then a final extension step of 72°C for 2 min.

### Results:

Since IL-22R is a shared alpha subunit, we evaluated an Origene panel for the expression of *IL-22R* mRNA. The highest *IL-22R* expression was detected in the pancreas, with skin and lung also exhibiting strong expression.

To test the possibility that the IL-20 subfamily might activate other Class II receptor combinations, BaF3 cells were stably transfected with Class II receptor subunits alone or in combinations and treated with the ligands. The assay shows that both IL-20 and MDA-7 stimulate an additional receptor complex consisting of consisting of IL-22R/IL-20RB (Table 1). We next wanted to determine which soluble receptors could block ligand activity. As mentioned above, IL-20RA/IL-20RB heterodimeric soluble receptor blocked proliferation stimulated by IL-20, IL-19, and MDA-7. In addition, IL-20RB soluble receptor alone blocked the activity of IL-19 and MDA-7, but not IL-20.

Since IL-22R is a shared alpha subunit, we evaluated an Origene panel for the expression of *IL-22R* mRNA (Table 2). The highest expression was detected in the pancreas, with skin and lung also exhibiting strong expression. Thus, overall *IL-20R*α, *IL-20R*β, and *IL-22R* all have robust expression in skin and lung.

Since *IL-20R*α, *IL-20R*β, and *IL-22R* are all expressed in the lung, we used *in situ* hybridization to evaluate whether the same cell types expressed all three receptors. Figure 3 shows that both epithelial cells as well as immune infiltrates exhibit positive staining in lung sections tested for mRNA expression by *in situ* hybridization.

## Claims

1. An isolated soluble receptor comprising an IL-22R subunit and an IL-20RB subunit, wherein the IL-22R subunit comprises a polypeptide comprising an amino acid sequence selected from the group consisting of SEQ ID NOs: 12, 13, 25, 26, 31, and 32, and the IL-20RB subunit comprises a polypeptide comprising an amino acid sequence selected from the group consisting of SEQ ID NOs: 15, 16, 17, 18, 19, 20, 21, 23, 28, 29, 34, and 35.

2. The soluble receptor of claim 1, wherein the IL-22R subunit and the IL-20RB subunit are linked together by a polypeptide linker.

3. The soluble receptor of claim 2, wherein the polypeptide linker has about 100 to 240 amino acid residues.

4. The soluble receptor of claim 3, wherein the polypeptide linker has about 170 amino acid residues.

5. The soluble receptor of claim 1, wherein the IL-22R subunit and the IL-20RB subunit each comprise a polypeptide linker fused to the subunit, and each of the polypeptide linkers has at least one cysteine residue, wherein at least one disulfide bond forms with a cysteine from the polypeptide linker of the IL-22R subunit and with a cysteine from the polypeptide linker of the IL-20RB subunit.

6. The soluble receptor of claim 5, wherein the IL-22R subunit is fused to all or a portion of the constant region of a heavy chain of an immunoglobulin (Ig) molecule, and the IL-20RB subunit is fused to all or a portion of the constant region of a light chain of an immunoglobulin molecule, wherein the light chain and the heavy chain are disulfide bonded together.

7. The soluble receptor of claim 6, wherein the constant region of the heavy chain comprises a CH1 domain.

8. The soluble receptor of claim 6, wherein the constant region of the heavy chain comprises a CH2 domain.

9. The soluble receptor of claim 6, wherein the constant region of the heavy chain comprises a CH1 domain, a CH2 domain, and a hinge sequence that connects the CH1 domain with the CH2 domain.

10. The soluble receptor of claim 6, wherein the IL-22R subunit fused to the constant region of the heavy chain comprises an amino acid sequence selected from the group consisting of SEQ ID NOs: 25, 26, 31, and 32, and the IL-20RB subunit fused to the constant region of the light chain of the Ig molecule comprises an amino acid sequence selected from the group consisting of SEQ ID NOs: 28, 29, 34, and 35.

11. The soluble receptor of one of claims 6-10, wherein the soluble receptor is disulfide bonded to a soluble receptor of one of claims 6-10.

12. The soluble receptor of claim 5, wherein the IL-20RB subunit is fused to all or a portion of the constant region of a heavy chain of an Ig molecule, and the IL-22R subunit is fused to all or a portion of the constant region of a light chain of an immunoglobulin molecule, wherein the light chain and the heavy chain are disulfide bonded together.

13. The soluble receptor of one of claims 1-12, wherein the IL-22R subunit comprises a polypeptide comprising an amino acid sequence of SEQ ID NO:13.

14. The soluble receptor of one of claims 1-12, wherein the IL-20RB subunit comprises a polypeptide comprising an amino acid sequence of SEQ ID NO:18.

15. A method for producing a soluble receptor comprising extracellular domains of IL-22R and IL-20RB comprising (a) introducing into a host cell a first DNA sequence comprising a DNA sequence that encodes the extracellular portion of IL-22R and the DNA that encodes an immunoglobulin light chain constant region;(b) introducing into the host cell a second DNA construct comprising a DNA sequence that encodes the extracellular portion of IL-20RB and a DNA sequence that encodes an immunoglobulin heavy chain constant region domain; (c) growing the host cell in an appropriate growth medium under physiological conditions to allow production of a fusion protein comprising the extracellular domain of IL-22R and IL-20RB; and (d) isolating the polypeptide from the host cell, wherein the extracellular domain of IL-22R comprises a polypeptide selected from the group consisting of SEQ ID NOs: 12 and 13, and the extracellular domain of IL-20RB comprises a polypeptide selected from the group consisting of SEQ ID NOs: 16, 17, 18, 19, 20, and 21.

16. A method for producing a soluble receptor comprising the extracellular domains of IL-22R and IL-20RB comprising (a) introducing into a host cell a first DNA sequence comprising DNA that encodes the extracellular portion of IL-20RB and the DNA that encodes an immunoglobulin light chain constant region;(b) introducing into the host cell a second DNA construct comprising a DNA sequence that encodes the extracellular portion of IL-22R and a DNA sequence that encodes an immunoglobulin heavy chain constant region (c) growing the host cell in an appropriate growth medium under physiological conditions to allow the production of a dimerized heterodimeric fusion protein comprising the extracellular domain of IL-22R and IL-20RB; and (d) isolating the dimerized polypeptide from the host cell, wherein the extracellular domain of IL-22R comprises a polypeptide selected from the group consisting of SEQ ID NOs: 12 and 13, and the extracellular domain of IL-20RB comprises a polypeptide selected from the group consisting of SEQ ID NOs: 16, 17, 18, 19, 20, and 21.

17. A method for producing a soluble receptor comprising the extracellular domains of IL-22R and IL-20RB comprising (a) introducing into a host cell a DNA construct containing a DNA construct that encodes the extracellular portion of IL-20RB and a DNA construct of the extracellular portion of IL-22R, (b) growing the host cell in an appropriate medium under physiological conditions to allow the production of the extracellular domain of IL-22R and the extracellular domain of IL-20RB; and (d) isolating the polypeptides from the host cell, wherein the extracellular domain of IL-22R comprises a polypeptide selected from the group consisting of SEQ ID NOs: 12 and 13, and the extracellular domain of IL-20RB comprises a polypeptide selected from the group consisting of SEQ ID NOs: 16, 17, 18, 19, 20, and 21.

18. A host cell transformed or transfected with a DNA construct that encodes the extracellular domain of IL-22RB and a DNA construct that encodes the extracellular domain of IL-20RB, wherein the extracellular domain of IL-22R comprises a polypeptide selected from the group consisting of SEQ ID NOs: 12 and 13, and the extracellular domain of IL-20RB comprises a polypeptide selected from the group consisting of SEQ ID NOs: 16, 17, 18, 19, 20, and 21.

19. A soluble receptor produced by the methods of one of claims 15-17.

20. Use of an isolated soluble receptor of any of claims 1-14 for the preparation of a medicament for the treatment of a disease selected from the group consisting of psoriasis, asthma, bronchitis, cystic fibrosis, inflammatory bowel disease, ulcerative colitis, and Crohn's disease.

21. The use of claim 20, wherein said disease is psoriasis.

22. An isolated soluble receptor of any of claims 1-14 for the treatment of a disease selected from the group consisting of psoriasis, asthma, bronchitis, cystic fibrosis, inflammatory bowel disease, ulcerative colitis, and Crohn's disease.

## Patentansprüche

1. Isolierter löslicher Rezeptor, der eine IL-22R-Untereinheit und eine IL-20RB-Untereinheit aufweist, wobei die IL-22R-Untereinheit ein Polypeptid aufweist mit einer Aminosäuresequenz, die ausgewählt ist aus der Gruppe bestehend aus SEQ ID Nr. 12, 13, 25, 26, 31 und 32, und die IL-20RB-Untereinheit ein Polypeptid aufweist mit einer Aminosäuresequenz, die ausgewählt ist aus der Gruppe bestehend aus SEQ ID Nr. 15, 16, 17, 18, 19, 20, 21, 23, 28, 29, 34 und 35.

2. Löslicher Rezeptor nach Anspruch 1, wobei die IL-22R-Untereinheit und die IL-20RB-Untereinheit durch einen Polypeptid-Linker miteinander verbunden sind.

3. Löslicher Rezeptor nach Anspruch 2, wobei der Polypeptid-Linker etwa 100 bis 240 Aminosäurereste aufweist.

4. Löslicher Rezeptor nach Anspruch 3, wobei der Polypeptid-Linker etwa 170 Aminosäurereste aufweist.

5. Löslicher Rezeptor nach Anspruch 1, wobei die IL-22R-Untereinheit und die IL-20RB-Untereinheit jeweils einen Polypeptid-Linker aufweisen, der mit der Untereinheit kondensiert ist, und jeder der Polypeptid-Linker mindestens einen Cysteinrest aufweist, wobei mindestens eine Disulfidbindung mit einem Cystein vom Polypeptid-Linker der IL-22R-Untereinheit und mit einem Cystein vom Polypeptid-Linker der IL-20RB-Untereinheit gebildet ist.

6. Löslicher Rezeptor nach Anspruch 5, wobei die IL-22R-Untereinheit mit der gesamten konstanten Region oder einem Teil der konstanten Region einer schweren Kette eines Immunoglobulin (Ig)-Moleküls kondensiert ist und die IL-20RB-Untereinheit mit der gesamten konstanten Region oder einem Teil der konstanten Region einer leichten Kette eines Immunoglobulin-Moleküls kondensiert ist, wobei die leichte Kette und die schwere Kette über eine Disulfidbrücke miteinander verbunden sind.

7. Löslicher Rezeptor nach Anspruch 6, wobei die konstante Region der schweren Kette eine CH1-Domäne aufweist.

8. Löslicher Rezeptor nach Anspruch 6, wobei die konstante Region der schweren Kette eine CH2-Domäne aufweist.

9. Löslicher Rezeptor nach Anspruch 6, wobei die konstante Region der schweren Kette eine CH1-Domäne, eine CH2-Domäne und eine flexible Linkersequenz aufweist, die die CH1-Domäne mit der CH2-Domäne verbindet.

10. Löslicher Rezeptor nach Anspruch 6, wobei die IL-22R-Untereinheit, die mit der konstanten Region der schweren Kette kondensiert ist, eine Aminosäuresequenz aufweist, die ausgewählt ist aus der Gruppe bestehend aus SEQ ID Nr. 25, 26, 31 und 32, und die IL-20RB-Untereinheit, die mit der konstanten Region der leichten Kette des Ig-Moleküls kondensiert ist, eine Aminosäuresequenz aufweist, die ausgewählt ist aus der Gruppe bestehend aus SEQ ID Nr. 28, 29, 34 und 35.

11. Löslicher Rezeptor nach einem der Ansprüche 6 - 10, wobei der lösliche Rezeptor über eine Disulfidbrücke mit einem löslichen Rezeptor nach einem der Ansprüche 6 - 10 verbunden ist.

12. Löslicher Rezeptor nach Anspruch 5, wobei die IL-20RB-Untereinheit mit der gesamten konstanten Region oder einem Teil der konstanten Region einer schweren Kette eines Ig-Moleküls kondensiert ist und die IL-22R-Untereinheit mit der gesamten konstanten Region oder einem Teil der konstanten Region einer leichten Kette eines Immunoglobulin-Moleküls kondensiert ist, wobei die leichte Kette und die schwere Kette über eine Disulfidbrücke miteinander verbunden sind.

13. Löslicher Rezeptor nach einem der Ansprüche 1 - 12, wobei die IL-22R-Untereinheit ein Polypeptid aufweist, das eine Aminosäuresequenz SEQ ID Nr. 13 aufweist.

14. Löslicher Rezeptor nach einem der Ansprüche 1 - 12, wobei die IL-20RB-Untereinheit ein Polypeptid aufweist, das eine Aminosäuresequenz SEQ ID Nr. 18 aufweist.

15. Verfahren zur Erzeugung eines löslichen Rezeptors, der die extrazellulären Domänen von IL-22R und IL-20RB aufweist, das umfasst: (a) Einführen einer ersten DNA-Sequenz, die eine den extrazellulären Abschnitt von IL-22R kodierende DNA-Sequenz und die eine konstante Region einer leichten Immunoglobulinkette kodierende DNA aufweist, in eine Wirtszelle; (b) Einführen eines zweiten DNA-Konstrukts, das eine den extrazellulären Abschnitt von IL-20RB kodierende DNA-Sequenz und eine eine Domäne einer konstanten Region einer schweren Immunoglobulin-Kette kodierende DNA-Sequenz aufweist, in die Wirtszelle; (c) Züchten der Wirtszelle in einem geeigneten Zuchtmedium unter physiologischen Bedingungen, um die Erzeugung eines Fusionsproteins zu ermöglichen, das die extrazelluläre Domäne von IL-22R und IL-20RB umfasst; und (d) Isolieren des Polypeptids aus der Wirtszelle, wobei die extrazelluläre Domäne von IL-22R ein Polypeptid aufweist, das ausgewählt ist aus der Gruppe bestehend aus SEQ ID Nr. 12 und 13, und die extrazelluläre Domäne von IL-20RB ein Polypeptid umfasst, das ausgewählt ist aus der Gruppe bestehend aus SEQ ID Nr. 16, 17, 18, 19, 20 und 21.

16. Verfahren zur Erzeugung eines löslichen Rezeptors, der extrazelluläre Domänen von IL-22R und IL-20RB aufweist, das umfasst: (a) Einführen einer ersten DNA-Sequenz, die den extrazellulären Abschnitt von IL-20RB kodierende DNA und die eine konstante Region einer leichten Immunoglobulinkette kodierende DNA aufweist, in eine Wirtszelle; (b) Einführen eines zweiten DNA-Konstrukts, das eine den extrazellulären Abschnitt von IL-22R kodierende DNA-Sequenz und eine eine konstante Region einer schweren Immunoglobulin-Kette kodierende DNA-Sequenz aufweist, in die Wirtszelle; (c) Züchten der Wirtszelle in einem geeigneten Zuchtmedium unter physiologischen Bedingungen, um die Erzeugung eines dimerisierten, heterodimeren Fusionsproteins zu ermöglichen, das die extrazelluläre Domäne von IL-22R und IL-20RB aufweist; und (d) Isolieren des dimerisierten Polypeptids aus der Wirtszelle, wobei die extrazelluläre Domäne von IL-22R ein Polypeptid umfasst, das ausgewählt ist aus der Gruppe bestehend aus SEQ ID Nr. 12 und 13, und die extrazelluläre Domäne von IL-20RB ein Polypeptid umfasst, das ausgewählt ist aus der Gruppe bestehend aus SEQ ID Nr. 16, 17, 18, 19, 20 und 21.

17. Verfahren zur Erzeugung eines löslichen Rezeptors, der die extrazellulären Domänen von IL-22R und IL-20RB aufweist, das umfasst: (a) Einführen eines DNA-Konstrukts, das ein den extrazellulären Abschnitt von IL-20RB kodierendes DNA-Konstrukt und ein DNA-Konstrukt des extrazellulären Abschnitts von IL-22R enthält, in eine Wirtszelle, (b) Züchten der Wirtszelle in einem geeigneten Medium unter physiologischen Bedingungen, um die Erzeugung der extrazellulären Domäne von IL-22R und der extrazellulären Domäne von IL-20RB zu ermöglichen; und (d) Isolieren der Polypeptide aus der Wirtszelle, wobei die extrazelluläre Domäne von IL-22R ein Polypeptid aufweist, das ausgewählt ist aus der Gruppe bestehend aus SEQ ID Nr. 12 und 13, und die extrazelluläre Domäne von IL-20RB ein Polypeptid aufweist, das ausgewählt ist aus der Gruppe bestehend aus SEQ ID Nr. 16, 17, 18, 19, 20 und 21.

18. Wirtszelle, die mit einem DNA-Konstrukt, das die extrazelluläre Domäne von IL-22RB kodiert, und einem DNA-Konstrukt, das die extrazelluläre Domäne von IL-20RB kodiert, transformiert oder transfiziert ist, wobei die extrazelluläre Domäne von IL-22R ein Polypeptid aufweist, das ausgewählt ist aus der Gruppe bestehend aus SEQ ID Nr. 12 und 13, und die extrazelluläre Domäne von IL-20RB ein Polypeptid aufweist, das ausgewählt ist aus der Gruppe bestehend aus SEQ ID Nr. 16, 17, 18, 19, 20 und 21.

19. Löslicher Rezeptor, der anhand der Verfahren nach einem der Ansprüche 15 - 17 erzeugt wurde.

20. Verwendung eines isolierten löslichen Rezeptors nach einem der Ansprüche 1 - 14 für die Erzeugung eines Medikaments für die Behandlung einer Krankheit, die ausgewählt ist aus der Gruppe bestehend aus Psoriasis, Asthma, Bronchitis, zystischer Fibrose, entzündlicher Darmerkrankung, Colitis ulcerosa und Morbus Crohn.

21. Verwendung nach Anspruch 20, wobei die Krankheit Psoriasis ist.

22. Isolierter löslicher Rezeptor nach einem der Ansprüche 1 - 14 für die Behandlung einer Krankheit, die ausgewählt ist aus der Gruppe bestehend aus Psoriasis, Asthma, Bronchitis, zystischer Fibrose, entzündlicher Darmerkrankung, Colitis ulcerosa und Morbus Crohn.

## Revendications

1. Récepteur soluble isolé comprenant une sous-unité IL-22R et une sous-unité IL-20RB, où la sous-unité IL-22R comprend un polypeptide comprenant une séquence d'acides aminés choisie dans le groupe constitué des SEQ ID NO : 12, 13, 25, 26, 31 et 32, et la sous-unité IL-20RB comprend un polypeptide comprenant une séquence d'acides aminés choisie dans le groupe constitué des SEQ ID NO : 15, 16, 17, 18, 19, 20, 21, 23, 28, 29, 34 et 35.

2. Récepteur soluble selon la revendication 1, dans lequel la sous-unité IL-22R et la sous-unité IL-20RB sont liées ensemble par un lieur polypeptidique.

3. Récepteur soluble selon la revendication 2, dans lequel le lieur polypeptidique a environ 100 à 240 résidus d'acides aminés.

4. Récepteur soluble selon la revendication 3, dans lequel le lieur polypeptidique a environ 170 résidus d'acides aminés.

5. Récepteur soluble selon la revendication 1, dans lequel la sous-unité IL-22R et la sous-unité IL-20RB comprennent chacune un lieur polypeptidique fusionné à la sous-unité, et chacun des lieurs polypeptidiques a au moins un résidu de cystéine, où au moins une liaison disulfure se forme avec une cystéine provenant du lieur polypeptidique de la sous-unité IL-22R et avec une cystéine provenant du lieur polypeptidique de la sous-unité IL-20RB.

6. Récepteur soluble selon la revendication 5, dans lequel la sous-unité IL-22R est fusionnée à la totalité ou à une partie de la région constante d'une chaîne lourde d'une molécule d'immunoglobuline (Ig), et la sous-unité IL-20RB est fusionnée à la totalité ou à une partie de la région constante d'une chaîne légère d'une molécule d'immunoglobuline, où la chaîne légère et la chaîne lourde sont liées ensemble par liaison disulfure.

7. Récepteur soluble selon la revendication 6, dans lequel la région constante de la chaîne lourde comprend un domaine CH1.

8. Récepteur soluble selon la revendication 6, dans lequel la région constante de la chaîne lourde comprend un domaine CH2.

9. Récepteur soluble selon la revendication 6, dans lequel la région constante de la chaîne lourde comprend un domaine CH1, un domaine CH2, et une séquence charnière qui relie le domaine CH1 au domaine CH2.

10. Récepteur soluble selon la revendication 6, dans lequel la sous-unité IL-22R fusionnée à la région constante de la chaîne lourde comprend une séquence d'acides aminés choisie dans le groupe constitué des SEQ ID NO : 25, 26, 31 et 32, et la sous-unité IL-20RB fusionnée à la région constante de la chaîne légère de la molécule d'Ig comprend une séquence d'acides aminés choisie dans le groupe constitué des SEQ ID NO : 28, 29, 34 et 35.

11. Récepteur soluble selon l'une quelconque des revendications 6 à 10, où le récepteur soluble est lié par liaison disulfure à un récepteur soluble selon l'une quelconque des revendications 6 à 10.

12. Récepteur soluble selon la revendication 5, dans lequel la sous-unité IL-20RB est fusionnée à la totalité ou à une partie de la région constante d'une chaîne lourde d'une molécule d'Ig, et la sous-unité IL-22R est fusionnée à la totalité ou à une partie de la région constante d'une chaîne légère d'une molécule d'immunoglobuline, où la chaîne légère et la chaîne lourde sont liées ensemble par liaison disulfure.

13. Récepteur soluble selon l'une quelconque des revendications 1 à 12, dans lequel la sous-unité IL-22R comprend un polypeptide comprenant une séquence d'acides aminés de SEQ ID NO : 13.

14. Récepteur soluble selon l'une quelconque des revendications 1 à 12, dans lequel la sous-unité IL-20RB comprend un polypeptide comprenant une séquence d'acides aminés de SEQ ID NO : 18.

15. Procédé de production d'un récepteur soluble comprenant des domaines extracellulaires d'IL-22R et d'IL-20RB comprenant (a) l'introduction dans une cellule hôte d'une première séquence d'ADN comprenant une séquence d'ADN qui code pour la partie extracellulaire d'IL-22R et l'ADN qui code pour une région constante de chaîne légère d'immunoglobuline ; (b) l'introduction dans la cellule hôte d'un second produit de construction d'ADN comprenant une séquence d'ADN qui code pour la partie extracellulaire d'IL-20RB et une séquence d'ADN qui code pour un domaine de région constante de chaîne lourde d'immunoglobuline ; (c) la culture de la cellule hôte dans un milieu de croissance approprié dans des conditions physiologiques pour permettre la production d'une protéine de fusion comprenant le domaine d'extracellulaire d'IL-22R et d'IL-20RB ; et (d) l'isolement du polypeptide à partir de la cellule hôte, où le domaine extracellulaire d'IL-22R comprend un polypeptide choisi dans le groupe constitué des SEQ ID NO : 12 et 13, et le domaine extracellulaire d'IL-20RB comprend un polypeptide choisi dans le groupe constitué des SEQ ID NO : 16, 17, 18, 19, 20 et 21.

16. Procédé de production d'un récepteur soluble comprenant les domaines extracellulaires d'IL-22R et d'IL-20RB comprenant (a) l'introduction dans une cellule hôte d'une première séquence d'ADN comprenant l'ADN qui code pour la partie extracellulaire d'IL-20RB et l'ADN qui code pour une région constante de chaîne légère d'immunoglobuline ; (b) l'introduction dans la cellule hôte d'un second produit de construction d'ADN comprenant une séquence d'ADN qui code pour la partie extracellulaire d'IL-22R et une séquence d'ADN qui code pour une région constante de chaîne lourde d'immunoglobuline ; (c) la culture de la cellule hôte dans un milieu de croissance approprié dans des conditions physiologiques pour permettre la production d'une protéine de fusion dimérisée hétérodimère comprenant le domaine extracellulaire d'IL-22R et d'IL-20RB ; et (d) l'isolement du polypeptide dimérisé à partir de la cellule hôte, où le domaine extracellulaire d'IL-22R comprend un polypeptide choisi dans le groupe constitué des SEQ ID NO : 12 et 13, et le domaine extracellulaire d'IL-20RB comprend un polypeptide choisi dans le groupe constitué des SEQ ID NO : 16, 17, 18, 19, 20 et 21.

17. Procédé de production d'un récepteur soluble comprenant les domaines extracellulaires d'IL-22R et d'IL-20RB comprenant (a) l'introduction dans une cellule hôte d'un produit de construction d'ADN qui code pour la partie extracellulaire d'IL-20RB et d'un produit de construction d'ADN de la partie extracellulaire d'IL-22R ; (b) la culture de la cellule hôte dans un milieu de croissance approprié dans des conditions physiologiques pour permettre la production du domaine extracellulaire d'IL-22R et du domaine extracellulaire d'IL-20B ; et (d) l'isolement des polypeptides à partir de la cellule hôte, où le domaine extracellulaire d'IL-22R comprend un polypeptide choisi dans le groupe constitué des SEQ ID NO : 12 et 13, et le domaine extracellulaire d'IL-20RB comprend un polypeptide choisi dans le groupe constitué des SEQ ID NO : 16, 17, 18, 19, 20 et 21.

18. Cellule hôte transformée ou transfectée avec un produit de construction d'ADN qui code pour le domaine extracellulaire d'IL-22RB et un produit de construction d'ADN qui code pour le domaine extracellulaire d'IL-20RB, où le domaine extracellulaire d'IL-22R comprend un polypeptide choisi dans le groupe constitué des SEQ ID NO : 12 et 13, et le domaine extracellulaire d'IL-20RB comprend un polypeptide choisi dans le groupe constitué des SEQ ID NO : 16, 17, 18, 19, 20 et 21.

19. Récepteur soluble produit par les procédés selon l'une quelconque des revendications 15 à 17.

20. Utilisation d'un récepteur soluble isolé selon l'une quelconque des revendications 1 à 14 pour la préparation d'un médicament destiné au traitement d'une maladie choisie dans le groupe constitué du psoriasis, de l'asthme, d'une bronchite, d'une fibrose kystique, d'une maladie intestinale inflammatoire, d'une rectocolite hémorragique et de la maladie de Crohn.

21. Utilisation selon la revendication 20, où ladite maladie est le psoriasis.

22. Récepteur soluble isolé selon l'une quelconque des revendications 1 à 14 destiné au traitement d'une maladie choisie dans le groupe constitué du psoriasis, de l'asthme, d'une bronchite, d'une fibrose kystique, d'une maladie intestinale inflammatoire, d'une rectocolite hémorragique et de la maladie de Crohn.
